# EUROPEAN PATENT SPECIFICATION

(11) **EP 4 150 630 B1**
(45) Date of publication and mention of the grant of the patent: **12.11.2025**
(21) Application number: 22729812.2
(22) Date of filing: 25.05.2022
(51) Int. Cl.: G16H 20/40, G16H 40/63, G16H 40/67

(54) **CONTROL ACCESS VERIFICATION OF A HEALTH CARE PROFESSIONAL**
KONTROLLZUGANGSÜBERPRÜFUNG EINES GESUNDHEITSPFLICHTIGEN PROFESSIONELLEN
VÉRIFICATION D'ACCÈS À LA COMMANDE D'UN PROFESSIONNEL DE SANTÉ

(30) Priority: 28.05.2021 US 202163194675 P; 01.06.2021 US 202117335362
(43) Date of publication of application: 22.03.2023
(73) Proprietor: Cilag GmbH International, 6300 Zug (CH)
(72) Inventor: SHELTON, IV, Frederick E., Cincinnati, Ohio 45242 (US); FIEBIG, Kevin M., Cincinnati, Ohio 45242 (US); HARRIS, Jason L., Cincinnati, Ohio 45242 (US)
(74) Representative: Carpmaels & Ransford LLP
(86) International application number: PCT/IB2022/054909
(87) International publication number: WO 2022/249102

(56) References cited:
- US-A1- 2017 086 926
- US-A1- 2021 015 554

## Description

### Background

Successful surgeries depend on the expertise of several types of operating room (OR) team members. The roles of operating room personnel vary. In addition, surgeons make the critical decisions involved in directing the course of a procedure. Surgeons may perform the incisions involved in an operation. Anesthesiologists or nurse anesthetists may be in charge of safely administering anesthesia to patients prior to surgery, monitoring them during surgery and making sure that they safely come out of anesthesia after the operation. A circulating technician may bring the patient to the operating room, tie the surgical gowns of surgeons and other personnel, deliver needed additional supplies such as instruments and medicine, and document the surgery. Scrub technicians may sterilize instruments before and after the surgery, keep the surgical field organized during surgery, and provide the surgeon with needed instruments. Registered nurses may perform the duties typically associated with surgical technicians, including acting as circulating nurses and scrub nurses. In addition, a nurse may act as the first assistant to the surgeon. OR human traffic has been implicated as a cause of surgical site infection.

US2017/086926A1 describes a medical system that allows for facial recognition of an individual associated with a particular surgical procedure, and more particularly, to a medical surgical system that can identify an individual where only a portion of the individual's face is viewable by a camera such that the medical surgical system will allow access to and be automatically configured based upon the identification.

US2021/015554A1 describes methods and systems for controlling (or facilitating control of) surgical tools during surgical procedures. More specifically, the disclosure relates to methods and systems that use computer-vision to facilitate controlling the operation of surgical tools during surgical procedures, thereby improving the safety and reliability of surgeries.

### Summary

A computer-implemented method may comprise: identifying a surgical instrument associated with a surgical procedure in an operating room (OR); detecting a control input by a health care professional (HCP) to control the surgical instrument; determining the HCP's access control level associated with the surgical instrument; and based on the detected control input by the HCP, determine whether to effectuate the control input based on the HCP's access control level associated with the surgical instrument, wherein based on the determination that the HCP's access control level associated with the surgical instrument does not authorize the HCP to effectuate the control input to control the surgical instrument, blocking the control input by the HCP to control the surgical instrument, and wherein based on the determination that the HCP's access control level associated with the surgical instrument authorizes the HCP to effectuate the control input to control the surgical instrument, effectuating the control input by the HCP to control the surgical instrument.

The computer-implemented method may provide the technical effect of preventing inadvertent control of the surgical instrument by authorizing or not authorizing the HCP to effectuate the control input to control the surgical instrument. Such inadvertent activating of the surgical instrument may occur while the HCP is cleaning up and/or disposing of the instrument, for example.

The access control level associated with the surgical instrument may comprise a first access control level to control the surgical instrument and a second access control level to control the surgical instrument, and the first access control level may comprise at least one of turning on the surgical instrument or turning off the surgical instrument and the second access control level may comprise at least one of increasing an energy level associated with the surgical instrument or decreasing the energy level associated with the surgical instrument.

The computer-implemented method may provide the technical effect of permitting tiered access to surgical instruments by permitting some, but not all, control to specified HCPs.

The control input may be a first control input, the HCP may be a first HCP, and the method may comprise: detecting a second control input by a second HCP to control the surgical instrument, wherein the second control input is configured to turn on or turn off the surgical instrument; determining the second HCP's access control level associated with the surgical instrument; and based on the determination that the second HCP's access control level associated with the surgical instrument authorizes the second HCP to turn on or turn off the surgical instrument, effectuating the second control input to turn on or turn off the surgical instrument.

The control input may be a first control input, the HCP may be a first HCP, and the method may comprise: detecting a second control input by a second HCP to control the surgical instrument, wherein the second control input is configured change an energy level associated with the surgical instrument; determining the second HCP's access control level associated with the surgical instrument; and based on the determination that the second HCP's access control level associated with the surgical instrument does not authorize the second HCP to change the energy level associated with the surgical instrument, blocking the control input to control the surgical instrument.

The computer-implemented method may comprise sending an alert to at least one of a device associated with the HCP or a display in the OR, the alert may comprise at least one of a notification that the control input by the HCP has been blocked or access control level information assigned to the HCP.

The computer-implemented method may provide the technical effect of keeping an HCP better informed of the surgical instrument in the OR, and may permit the HCP to alter an access control level of another HCP.

The computer-implemented method may comprise: monitoring a movement associated with the first HCP; determining that the first HCP is in a proximity to an operating table; based on the determination that the first HCP is in the proximity to the operating table, sending an access control level adjustment inquiry message to a second HCP, the access control level adjustment inquiry message being configured to prompt the HCP to indicate whether the HCP's access control level associated with the surgical instrument needs an adjustment based on the proximity of the first HCP to the operating table; receiving an access control level adjustment request from the second HCP in response to the access control level adjustment inquiry message; and based on the access control level adjustment request, adjusting the access control level associated with the first HCP.

The computer-implemented method may provide the technical effect of permitting a second HCP to oversee and adjust an access control level of a first HCP to permit a more efficient surgical procedure.

The computer-implemented method may comprise sending an access control level adjustment notification to the HCP, the access control level adjustment notification notifying that the HCP's access control level associated with the surgical instrument has been adjusted by the second HCP.

The computer-implemented method may provide the technical effect of ensuring that HCPs in an OR are better informed of other HCPs actions, which may result in a more efficient surgical procedure.

The method may comprise: identifying a current surgical step in the surgical procedure; determining whether to adjust the access control level associated with the HCP based on the identified current surgical step; and based on the determination to adjust the HCP's access control level associated with the surgical instrument, adjusting the HCP's access control level associated with the surgical instrument to allow the HCP to control the surgical instrument during the current surgical step in the surgical procedure.

The computer-implemented method may provide the technical effect of preventing or permitting an HCP from performing an adjustment of the surgical instrument dependent on the current surgical step, which may improve the safety of a patient and/or the outcome of the surgery.

The method may comprise: identifying an energized state of the surgical instrument; and determining whether to adjust the HCP's access control level associated with the surgical instrument based on the identified energized state of the surgical instrument, wherein based on the identified energized state of the surgical instrument being at a high energized state, the method may comprise adjusting the HCP's access control level associated with the surgical instrument to block the control input by the HCP to control the surgical instrument, and wherein based the identified energized state of the surgical instrument being at a low energized state, the method may comprise adjusting the HCP's access control level associated with the surgical instrument to effectuate the control input by the HCP to control the surgical instrument.

The computer-implemented method may provide the technical effect of preventing an inadvertent control of the surgical instrument, for example, by blocking a control input by a scrub nurse when it is determined that the surgical instrument is in a high energized state.

The method may comprise: monitoring a biomarker associated with the HCP, the biomarker may comprise at least one of a fatigue level or a stress level; and determining whether to adjust the HCP's access control level associated with the surgical instrument based on the monitored biomarker associated with the HCP, wherein based on the monitored biomarker associated with the HCP indicating that the HCP has an increased level of at least one of the fatigue level or the stress level, the method may comprise adjusting the HCP's access control level associated with the surgical instrument to block the control input by the HCP to control the surgical instrument, and wherein based the monitored biomarker associated with the HCP indicating that the HCP has an acceptable level of at least one of the fatigue level or the stress level, the method may comprise adjusting the HCP's access control level associated with the surgical instrument to effectuate the control input by the HCP to control the surgical instrument.

The computer-implemented method may provide the technical effect of improving the safety of the patient and/or the outcome of the surgery based on a biomarker of the HCP.

A computing system may comprise a processor configured to perform any of the methods described above.

A data processing device may comprise means for carrying out any of the methods described above.

A system may comprise the computing system described above, and a surgical instrument communicatively coupled with the computer system.

A system may comprise the data processing device described above, and a surgical instrument a communicatively coupled with the data processing device.

A computer readable medium may have instructions which, when executed by a computer, cause implementation of any of the methods described above.

A computing system may identify a surgical instrument associated with a surgical procedure in an operating room (OR). During a surgical operation, the computing system may detect a control input by a health care professional (HCP) to control the surgical instrument. The control input by the HCP may be turning on/off the surgical instrument, increase/decrease an energized state of the surgical instrument, and/or the like. If the computing system detects a control input by the HCP, the computing system may determine whether the HCP is authorized to provide the control input to control the surgical instrument. For example, the computing system may determine the HCP's access control level associated with the surgical instrument. The control level may provide information about whether the HCP is authorized to control the surgical instrument. In examples, the control level may indicate that the HCP is unauthorized to control the surgical instrument. In examples, the control level may indicate that the HCP is authorized to control the surgical instrument. The access control level may provide information about whether the HCP has a full control to control a surgical instrument. The access control level may provide information about whether the HCP has a partial control to control a surgical instrument (e.g., limited to turning on/off the surgical instrument). For example, the access control level for the HCP may be tiered. A lower-tiered access control level may allow/authenticate the HCP to turn on/off the surgical instrument. A higher-tiered access control may allow/authenticate the HCP to adjust an energized state of the surgical instrument.

The computing system may effectuate the control input by the HCP if the computing system determines that the HCP is authorized to control the surgical instrument. For example, if the computing system determines that the HCP is unauthorized to control the surgical instrument, the computing system may block the control input by the HCP. If the computing system determines that the HCP is authorized to control the surgical instrument, the computing system may effectuate the control input by the HCP.

The computing system may send an alert to the HCP. The alert may notify the HCP whether the HCP is authorized or unauthorized to control the surgical instrument. The alert may also notify the HCP whether the control input has been effectuated or blocked, e.g., based on the control access level associated with the HCP. The alert may be or may include one or more of a virtual alert, an audible alert, a haptic alert, or an augmented reality alert.

The computing system may adjust the HCP's control access level. The computing system may adjust the control level of the HCP based on a proximity to and/or within a virtual boundary of restricted access area. For example, if the HCP is unauthorized to control a surgical instrument and the computing system determines that the HCP is within a virtual boundary area of an operating table and/or a virtual boundary area of a surgeon, the computing system may adjust the control access level of the HCP and provide a partial authorization to control the surgical instrument (e.g., turning on/off the surgical instrument).

The computing system may adjust a control access level of an HCP based on a request from other HCP(s) in the OR. For example, if the computing system blocks a control input by the HCP, such as a scrub nurse, the computing system may send a message to other HCP in the OR, such as a surgeon. The message may be or may include an access control level adjustment message. If the other HCP, such as the surgeon, determines that the scrub nurse should be able to control the surgical instrument, e.g., turning on the surgical instrument before handing the instrument to the surgeon, the surgeon may send an access control level adjustment request to the computing system. The computing system may, based on receiving the access control level adjustment request from the surgeon, adjust the access control of the scrub nurse. The computing system may send a notification, such as an access control level adjustment notification, to the HCP. The notification may notify the HCP that the access control level associated with the HCP has been adjusted by other HCP, such as the surgeon.

The computing system may adjust a control access level of an HCP based on a surgical step in a surgical procedure. The computing system may identify a current surgical step in the surgical procedure. Based on the current surgical step, the computing system may adjust the control access level of the HCP. For example, to prevent an inadvertent control of a surgical instrument, the computing system may adjust the control access level of the HCP when the computing system determines the current surgical step matches with operating the surgical instrument. The computing system may block the control input by the HCP in other surgical steps.

The computing system may adjust a control access level of an HCP based on an energized state of a surgical instrument. The computing system may determine whether the surgical instrument is in a low energized state or a high energized state. The computing system may block a control input by the HCP if the surgical instrument is in a high energized state. The computing system may effectuate the control input by the HCP if the surgical instrument is in a low energized state.

The computing system may adjust a control access level of an HCP based on biomarker measurements associated with the HCP. For example, the computing system may monitor one or more biomarker of the HCP. If the computing system detects an elevated and/or an increased stress level or a fatigue level of the HCP, the computing system may adjust the control access level of the HCP by blocking a control input by the HCP. The computing system may adjust the control access level of the HCP by effectuating the control input by the HCP if the computing system detects an acceptable stress level and/or an acceptable fatigue level of the HCP.

### Brief Description of the Drawings

FIG. 1 is a block diagram of a computer-implemented healthcare personnel (HCP) monitoring system.
FIG. 2 shows an example of an HCP monitoring system in a surgical operating room.
FIG. 3 illustrates an example surgical hub paired with various systems.
FIG. 4 illustrates a surgical data network having a set of communication surgical hubs configured to connect with a set of sensing systems, an environmental sensing system, a set of devices, etc.
FIG. 5 illustrates an example computer-implemented interactive surgical system that may be part of an HCP monitoring system.
FIG. 6 illustrates a logic diagram of a control system of a surgical instrument.
FIG. 7 shows an exemplary sensing system with a sensor unit and a data processing and communication unit.
FIG. 8 illustrates an exemplary timeline of an illustrative surgical procedure indicating adjusting operational parameters of a surgical device based on a surgeon biomarker level.
FIG. 9 is a block diagram of the computer-implemented interactive HCP monitoring system.
FIG. 10 shows an example surgical system having a surgical instrument in communication with a console or a portable device.
FIG. 11 is a diagram of an example situationally aware surgical system.
FIG. 12 illustrates examples of one or more virtual boundaries associated with restricted accesses in an operating room (OR).
FIG. 13 illustrates examples of one or more virtual boundaries associated with restricted accesses in an OR.
FIG. 14 illustrates examples of a constant virtual boundary, a selective virtual boundary, or an adaptive virtual boundary in an OR
FIG. 15 illustrates example virtual boundary associated with a surgical instrument.
FIG. 16 illustrates an example notification of a computing system for an unauthorized energy level change of a surgical instrument.
FIG. 17 illustrates an example flow of monitoring an HCP movement relative to a virtual boundary of restricted access area in an OR.
FIG. 18 illustrates an example flow of control access verification of an HCP in an OR in accordance with an embodiment of the invention.

### Detailed Description

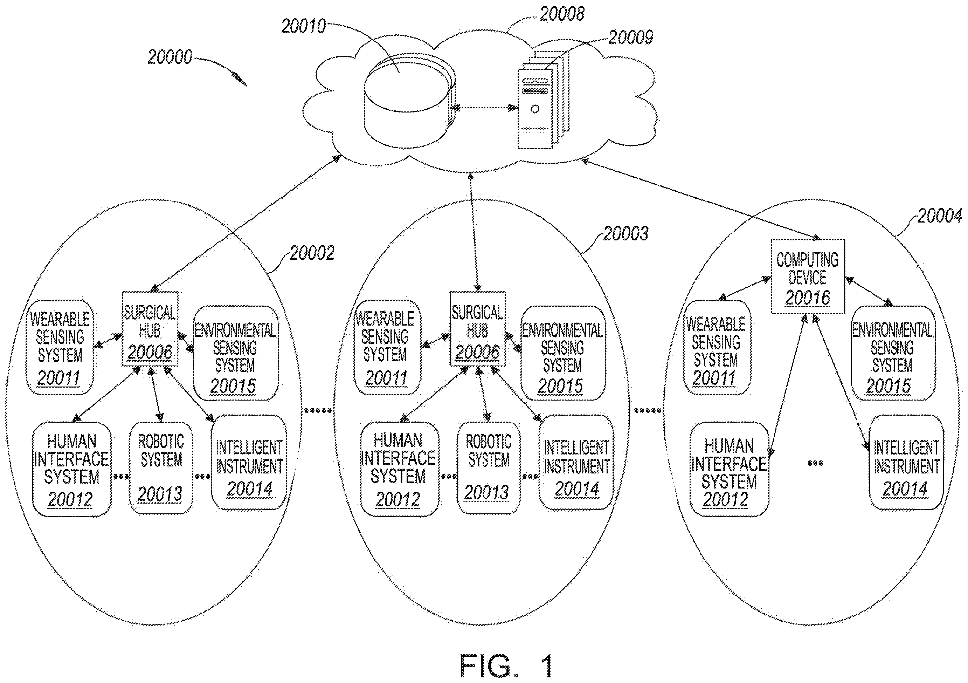

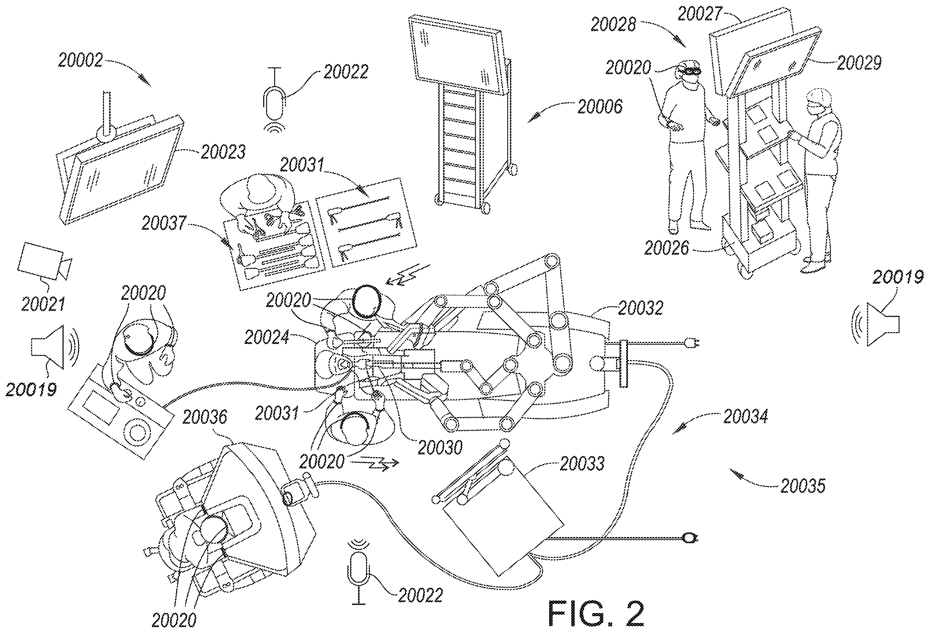

FIG. 1 is a block diagram of a computer-implemented HCP monitoring system 20000. An example HCP monitoring system such as the HCP monitoring system 20000 may include one or more HCP monitoring systems (e.g., HCP monitoring sub-systems) 20002, 20003 and 20004. For example, HCP monitoring system 20002 may include a computer-implemented interactive surgical system. For example, HCP monitoring system 20002 may include at least one of the following: a surgical hub 20006 in communication with a cloud computing system 20008, for example, as described in FIG. 2. An HCP monitoring system may include at least one of the following: a surgical hub 20006 or a computing device 20016 in communication with a could computing system 20008. The cloud computing system 20008 may include at least one remote cloud server 20009 and at least one remote cloud storage unit 20010. Example HCP monitoring systems 20002, 20003, or 20004 may include a wearable sensing system 20011, an environmental sensing system 20015, a robotic system 20013, one or more intelligent instruments 20014, human interface system 20012, etc. The human interface system is also referred herein as the human interface device. The wearable sensing system 20011 may include one or more HCP sensing systems, and/or one or more patient sensing systems. The environmental sensing system 20015 may include one or more devices, for example, used for measuring one or more environmental attributes, for example, as further described in FIG. 2. The robotic system 20013 may include a plurality of devices used for performing a surgical procedure, for example, as further described in FIG. 2.

The HCP monitoring system 20002 may be in communication with a remote server 20009 that may be part of a cloud computing system 20008. In an example, HCP monitoring system 20002 may be in communication with a remote server 20009 via an internet service provider's cable/FIOS networking node. In an example, a patient sensing system may be in direct communication with a remote server 20009. The HCP monitoring system 20002 and/or a component therein may communicate with the remote servers 20009 via a cellular transmission/reception point (TRP) or a base station using one or more of the following cellular protocols: GSM/GPRS/EDGE (2G), UMTS/HSPA (3G), long term evolution (LTE) or 4G, LTE-Advanced (LTE-A), new radio (NR) or 5G.

A surgical hub 20006 may have cooperative interactions with one of more means of displaying the image from the laparoscopic scope and information from one or more other smart devices and one or more sensing systems 20011. The surgical hub 20006 may interact with one or more sensing systems 20011, one or more smart devices, and multiple displays. The surgical hub 20006 may be configured to gather measurement data from the one or more sensing systems 20011 and send notifications or control messages to the one or more sensing systems 20011. The surgical hub 20006 may send and/or receive information including notification information to and/or from the human interface system 20012. The human interface system 20012 may include one or more human interface devices (HIDs). The surgical hub 20006 may send and/or receive notification information or control information to audio, display and/or control information to various devices that are in communication with the surgical hub.

For example, the sensing systems 20001 may include the wearable sensing system 20011 (which may include one or more HCP sensing systems and one or more patient sensing systems) and the environmental sensing system 20015 as discussed in FIG. 1. The one or more sensing systems 20001 may measure data relating to various biomarkers. The one or more sensing systems 20001 may measure the biomarkers using one or more sensors, for example, photosensors (e.g., photodiodes, photoresistors), mechanical sensors (e.g., motion sensors), acoustic sensors, electrical sensors, electrochemical sensors, thermoelectric sensors, infrared sensors, etc. The one or more sensors may measure the biomarkers as described herein using one of more of the following sensing technologies: photoplethysmography, electrocardiography, electroencephalography, colorimetry, impedimentary, potentiometry, amperometry, etc.

The biomarkers measured by the one or more sensing systems 20001 may include, but are not limited to, sleep, core body temperature, maximal oxygen consumption, physical activity, alcohol consumption, respiration rate, oxygen saturation, blood pressure, blood sugar, heart rate variability, blood potential of hydrogen, hydration state, heart rate, skin conductance, peripheral temperature, tissue perfusion pressure, coughing and sneezing, gastrointestinal motility, gastrointestinal tract imaging, respiratory tract bacteria, edema, mental aspects, sweat, circulating tumor cells, autonomic tone, circadian rhythm, and/or menstrual cycle.

The biomarkers may relate to physiologic systems, which may include, but are not limited to, behavior and psychology, cardiovascular system, renal system, skin system, nervous system, gastrointestinal system, respiratory system, endocrine system, immune system, tumor, musculoskeletal system, and/or reproductive system. Information from the biomarkers may be determined and/or used by the computer-implemented patient and HCP monitoring system 20000, for example. The information from the biomarkers may be determined and/or used by the computer-implemented patient and HCP monitoring system 20000 to improve said systems and/or to improve patient outcomes, for example. The one or more sensing systems 20001, biomarkers 20005, and physiological systems are described in more detail in U.S. App No. 17/156,287 (attorney docket number END9290USNP1), titled METHOD OF ADJUSTING A SURGICAL PARAMETER BASED ON BIOMARKER MEASUREMENTS, filed January 22, 2021.

FIG. 2 shows an example of an HCP monitoring system 20002 in a surgical operating room. As illustrated in FIG. 2, a patient is being operated on by one or more health care professionals (HCPs). The HCPs are being monitored by one or more HCP sensing systems 20020 worn by the HCPs. The HCPs and the environment surrounding the HCPs may also be monitored by one or more environmental sensing systems including, for example, a set of cameras 20021, a set of microphones 20022, and other sensors, etc. that may be deployed in the operating room. The HCP sensing systems 20020 and the environmental sensing systems may be in communication with a surgical hub 20006, which in turn may be in communication with one or more cloud servers 20009 of the cloud computing system 20008, as shown in FIG. 1. The environmental sensing systems may be used for measuring one or more environmental attributes, for example, HCP position in the surgical theater, HCP movements, ambient noise in the surgical theater, temperature/humidity in the surgical theater, etc.

As illustrated in FIG. 2, a primary display 20023 and one or more audio output devices (e.g., speakers 20019) are positioned in the sterile field to be visible to an operator at the operating table 20024. In addition, a visualization/notification tower 20026 is positioned outside the sterile field. The visualization/notification tower 20026 may include a first non-sterile human interactive device (HID) 20027 and a second non-sterile HID 20029, which may face away from each other. The HID may be a display or a display with a touchscreen allowing a human to interface directly with the HID. A human interface system, guided by the surgical hub 20006, may be configured to utilize the HIDs 20027, 20029, and 20023 to coordinate information flow to operators inside and outside the sterile field. In an example, the surgical hub 20006 may cause an HID (e.g., the primary HID 20023) to display a notification and/or information about the patient and/or a surgical procedure step. In an example, the surgical hub 20006 may prompt for and/or receive input from personnel in the sterile field or in the non-sterile area. In an example, the surgical hub 20006 may cause an HID to display a snapshot of a surgical site, as recorded by an imaging device 20030, on a non-sterile HID 20027 or 20029, while maintaining a live feed of the surgical site on the primary HID 20023. The snapshot on the non-sterile display 20027 or 20029 can permit a non-sterile operator to perform a diagnostic step relevant to the surgical procedure, for example.

In one aspect, the surgical hub 20006 may be configured to route a diagnostic input or feedback entered by a non-sterile operator at the visualization tower 20026 to the primary display 20023 within the sterile field, where it can be viewed by a sterile operator at the operating table. In one example, the input can be in the form of a modification to the snapshot displayed on the non-sterile display 20027 or 20029, which can be routed to the primary display 20023 by the surgical hub 20006.

Referring to FIG. 2, a surgical instrument 20031 is being used in the surgical procedure as part of the HCP monitoring system 20002. The hub 20006 may be configured to coordinate information flow to a display of the surgical instrument 20031. For example, in U.S. Patent Application Publication No. US 2019-0200844 A1 (U.S. Patent Application No. 16/209,385), titled METHOD OF HUB COMMUNICATION, PROCESSING, STORAGE AND DISPLAY, filed December 4, 2018. A diagnostic input or feedback entered by a non-sterile operator at the visualization tower 20026 can be routed by the hub 20006 to the surgical instrument display within the sterile field, where it can be viewed by the operator of the surgical instrument 20031. Example surgical instruments that are suitable for use with the HCP monitoring system 20002 are described under the heading "Surgical Instrument Hardware" and in U.S. Patent Application Publication No. US 2019-0200844 A1 (U.S. Patent Application No. 16/209,385), titled METHOD OF HUB COMMUNICATION, PROCESSING, STORAGE AND DISPLAY, filed December 4, 2018.

FIG. 2 illustrates an example of an HCP monitoring system 20002 being used to perform a surgical procedure on a patient who is lying down on an operating table 20024 in a surgical operating room 20035. A robotic system 20034 may be used in the surgical procedure as a part of the HCP monitoring system 20002. The robotic system 20034 may include a surgeon's console 20036, a patient side cart 20032 (surgical robot), and a surgical robotic hub 20033. The patient side cart 20032 can manipulate at least one removably coupled surgical tool 20037 through a minimally invasive incision in the body of the patient while the surgeon views the surgical site through the surgeon's console 20036. An image of the surgical site can be obtained by a medical imaging device 20030, which can be manipulated by the patient side cart 20032 to orient the imaging device 20030. The robotic hub 20033 can be used to process the images of the surgical site for subsequent display to the surgeon through the surgeon's console 20036.

Other types of robotic systems can be readily adapted for use with the HCP monitoring system 20002. Various examples of robotic systems and surgical tools that are suitable for use with the present disclosure are described in U.S. Patent Application Publication No. US 2019-0201137 A1 (U.S. Patent Application No. 16/209,407), titled METHOD OF ROBOTIC HUB COMMUNICATION, DETECTION, AND CONTROL, filed December 4, 2018.

Various examples of cloud-based analytics that are performed by the cloud computing system 20008, and are suitable for use with the present disclosure, are described in U.S. Patent Application Publication No. US 2019-0206569 A1 (U.S. Patent Application No. 16/209,403), titled METHOD OF CLOUD BASED DATA ANALYTICS FOR USE WITH THE HUB, filed December 4, 2018.

In various aspects, the imaging device 20030 may include at least one image sensor and one or more optical components. Suitable image sensors may include, but are not limited to, Charge-Coupled Device (CCD) sensors and Complementary Metal-Oxide Semiconductor (CMOS) sensors.

The optical components of the imaging device 20030 may include one or more illumination sources and/or one or more lenses. The one or more illumination sources may be directed to illuminate portions of the surgical field. The one or more image sensors may receive light reflected or refracted from the surgical field, including light reflected or refracted from tissue and/or surgical instruments.

The one or more illumination sources may be configured to radiate electromagnetic energy in the visible spectrum as well as the invisible spectrum. The visible spectrum, sometimes referred to as the optical spectrum or luminous spectrum, is that portion of the electromagnetic spectrum that is visible to (i.e., can be detected by) the human eye and may be referred to as visible light or simply light. A typical human eye will respond to wavelengths in air that range from about 380 nm to about 750 nm.

The invisible spectrum (e.g., the non-luminous spectrum) is that portion of the electromagnetic spectrum that lies below and above the visible spectrum (i.e., wavelengths below about 380 nm and above about 750 nm). The invisible spectrum is not detectable by the human eye. Wavelengths greater than about 750 nm are longer than the red visible spectrum, and they become invisible infrared (IR), microwave, and radio electromagnetic radiation. Wavelengths less than about 380 nm are shorter than the violet spectrum, and they become invisible ultraviolet, x-ray, and gamma ray electromagnetic radiation.

In various aspects, the imaging device 20030 is configured for use in a minimally invasive procedure. Examples of imaging devices suitable for use with the present disclosure include, but are not limited to, an arthroscope, angioscope, bronchoscope, choledochoscope, colonoscope, cytoscope, duodenoscope, enteroscope, esophagogastro-duodenoscope (gastroscope), endoscope, laryngoscope, nasopharyngo-neproscope, sigmoidoscope, thoracoscope, and ureteroscope.

The imaging device may employ multi-spectrum monitoring to discriminate topography and underlying structures. A multi-spectral image is one that captures image data within specific wavelength ranges across the electromagnetic spectrum. The wavelengths may be separated by filters or by the use of instruments that are sensitive to particular wavelengths, including light from frequencies beyond the visible light range, e.g., IR and ultraviolet. Spectral imaging can allow extraction of additional information that the human eye fails to capture with its receptors for red, green, and blue. The use of multi-spectral imaging is described in greater detail under the heading "Advanced Imaging Acquisition Module" in U.S. Patent Application Publication No. US 2019-0200844 A1 (U.S. Patent Application No. 16/209,385), titled METHOD OF HUB COMMUNICATION, PROCESSING, STORAGE AND DISPLAY, filed December 4, 2018. Multi-spectrum monitoring can be a useful tool in relocating a surgical field after a surgical task is completed to perform one or more of the previously described tests on the treated tissue. It is axiomatic that strict sterilization of the operating room and surgical equipment is required during any surgery. The strict hygiene and sterilization conditions required in a "surgical theater," i.e., an operating or treatment room, necessitate the highest possible sterility of all medical devices and equipment. Part of that sterilization process is the need to sterilize anything that comes in contact with the patient or penetrates the sterile field, including the imaging device 20030 and its attachments and components. It will be appreciated that the sterile field may be considered a specified area, such as within a tray or on a sterile towel, that is considered free of microorganisms, or the sterile field may be considered an area, immediately around a patient, who has been prepared for a surgical procedure. The sterile field may include the scrubbed team members, who are properly attired, and all furniture and fixtures in the area.

Wearable sensing system 20011 illustrated in FIG. 1 may include one or more sensing systems, for example, HCP sensing systems 20020 as shown in FIG. 2. The HCP sensing systems 20020 may include sensing systems to monitor and detect a set of physical states and/or a set of physiological states of a healthcare personnel (HCP). An HCP may be a surgeon or one or more healthcare personnel assisting the surgeon or other healthcare service providers in general. In an example, a sensing system 20020 may measure a set of biomarkers to monitor the heart rate of an HCP. In an example, a sensing system 20020 worn on a surgeon's wrist (e.g., a watch or a wristband) may use an accelerometer to detect hand motion and/or shakes and determine the magnitude and frequency of tremors. The sensing system 20020 may send the measurement data associated with the set of biomarkers and the data associated with a physical state of the surgeon to the surgical hub 20006 for further processing. One or more environmental sensing devices may send environmental information to the surgical hub 20006. For example, the environmental sensing devices may include a camera 20021 for detecting hand/body position of an HCP. The environmental sensing devices may include microphones 20022 for measuring the ambient noise in the surgical theater. Other environmental sensing devices may include devices, for example, a thermometer to measure temperature and a hygrometer to measure humidity of the surroundings in the surgical theater, etc. The surgical hub 20006, alone or in communication with the cloud computing system, may use the surgeon biomarker measurement data and/or environmental sensing information to modify the control algorithms of hand-held instruments or the averaging delay of a robotic interface, for example, to minimize tremors. In an example, the HCP sensing systems 20020 may measure one or more surgeon biomarkers associated with an HCP and send the measurement data associated with the surgeon biomarkers to the surgical hub 20006. The HCP sensing systems 20020 may use one or more of the following RF protocols for communicating with the surgical hub 20006: Bluetooth, Bluetooth Low-Energy (BLE), Bluetooth Smart, Zigbee, Z-wave, IPv6 Low-power wireless Personal Area Network (6LoWPAN), Wi-Fi. The surgeon biomarkers may include one or more of the following: stress, heart rate, etc. The environmental measurements from the surgical theater may include ambient noise level associated with the surgeon or the patient, surgeon and/or staff movements, surgeon and/or staff attention level, etc.

The surgical hub 20006 may use the surgeon biomarker measurement data associated with an HCP to adaptively control one or more surgical instruments 20031. For example, the surgical hub 20006 may send a control program to a surgical instrument 20031 to control its actuators to limit or compensate for fatigue and use of fine motor skills. The surgical hub 20006 may send the control program based on situational awareness and/or the context on importance or criticality of a task. The control program may instruct the instrument to alter operation to provide more control when control is needed.

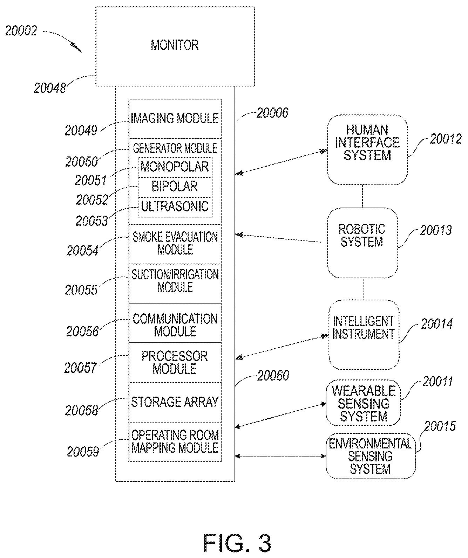

FIG. 3 shows an example HCP monitoring system 20002 with a surgical hub 20006 paired with a wearable sensing system 20011, an environmental sensing system 20015, a human interface system 20012, a robotic system 20013, and an intelligent instrument 20014. The hub 20006 includes a display 20048, an imaging module 20049, a generator module 20050, a communication module 20056, a processor module 20057, a storage array 20058, and an operating-room mapping module 20059. In certain aspects, as illustrated in FIG. 3, the hub 20006 further includes a smoke evacuation module 20054 and/or a suction/irrigation module 20055. During a surgical procedure, energy application to tissue, for sealing and/or cutting, is generally associated with smoke evacuation, suction of excess fluid, and/or irrigation of the tissue. Fluid, power, and/or data lines from different sources are often entangled during the surgical procedure. Valuable time can be lost addressing this issue during a surgical procedure. Detangling the lines may necessitate disconnecting the lines from their respective modules, which may require resetting the modules. The hub modular enclosure 20060 offers a unified environment for managing the power, data, and fluid lines, which reduces the frequency of entanglement between such lines. Aspects of the present disclosure present a surgical hub 20006 for use in a surgical procedure that involves energy application to tissue at a surgical site. The surgical hub 20006 includes a hub enclosure 20060 and a combo generator module slidably receivable in a docking station of the hub enclosure 20060. The docking station includes data and power contacts. The combo generator module includes two or more of an ultrasonic energy generator component, a bipolar RF energy generator component, and a monopolar RF energy generator component that are housed in a single unit. In one aspect, the combo generator module also includes a smoke evacuation component, at least one energy delivery cable for connecting the combo generator module to a surgical instrument, at least one smoke evacuation component configured to evacuate smoke, fluid, and/or particulates generated by the application of therapeutic energy to the tissue, and a fluid line extending from the remote surgical site to the smoke evacuation component. In one aspect, the fluid line may be a first fluid line, and a second fluid line may extend from the remote surgical site to a suction and irrigation module 20055 slidably received in the hub enclosure 20060. In one aspect, the hub enclosure 20060 may include a fluid interface. Certain surgical procedures may require the application of more than one energy type to the tissue. One energy type may be more beneficial for cutting the tissue, while another different energy type may be more beneficial for sealing the tissue. For example, a bipolar generator can be used to seal the tissue while an ultrasonic generator can be used to cut the sealed tissue. Aspects of the present disclosure present a solution where a hub modular enclosure 20060 is configured to accommodate different generators and facilitate an interactive communication therebetween. One of the advantages of the hub modular enclosure 20060 is enabling the quick removal and/or replacement of various modules. Aspects of the present disclosure present a modular surgical enclosure for use in a surgical procedure that involves energy application to tissue. The modular surgical enclosure includes a first energy-generator module, configured to generate a first energy for application to the tissue, and a first docking station comprising a first docking port that includes first data and power contacts, wherein the first energy-generator module is slidably movable into an electrical engagement with the power and data contacts and wherein the first energy-generator module is slidably movable out of the electrical engagement with the first power and data contacts. Further to the above, the modular surgical enclosure also includes a second energy-generator module configured to generate a second energy, different than the first energy, for application to the tissue, and a second docking station comprising a second docking port that includes second data and power contacts, wherein the second energy generator module is slidably movable into an electrical engagement with the power and data contacts, and wherein the second energy-generator module is slidably movable out of the electrical engagement with the second power and data contacts. In addition, the modular surgical enclosure also includes a communication bus between the first docking port and the second docking port, configured to facilitate communication between the first energy-generator module and the second energy-generator module. Referring to FIG. 3, aspects of the present disclosure are presented for a hub modular enclosure 20060 that allows the modular integration of a generator module 20050, a smoke evacuation module 20054, and a suction/irrigation module 20055. The hub modular enclosure 20060 further facilitates interactive communication between the modules 20059, 20054, and 20055. The generator module 20050 can be a generator module 20050 with integrated monopolar, bipolar, and ultrasonic components supported in a single housing unit slidably insertable into the hub modular enclosure 20060. The generator module 20050 can be configured to connect to a monopolar device 20051, a bipolar device 20052, and an ultrasonic device 20053. Alternatively, the generator module 20050 may comprise a series of monopolar, bipolar, and/or ultrasonic generator modules that interact through the hub modular enclosure 20060. The hub modular enclosure 20060 can be configured to facilitate the insertion of multiple generators and interactive communication between the generators docked into the hub modular enclosure 20060 so that the generators would act as a single generator.

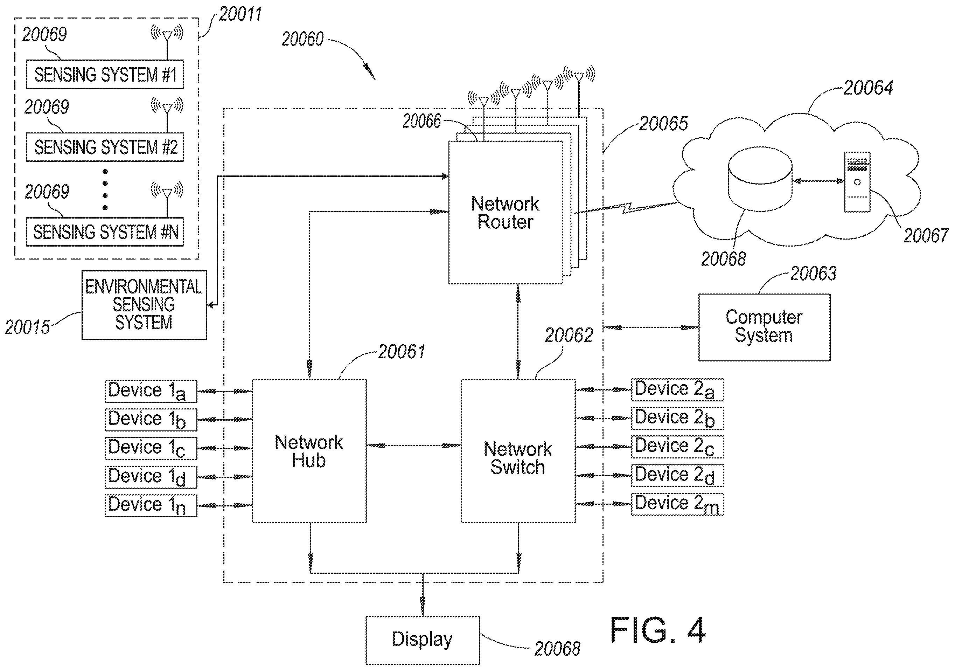

FIG. 4 illustrates a surgical data network having a set of communication hubs configured to connect a set of sensing systems, environment sensing system(s), and a set of other modular devices located in one or more operating theaters of a healthcare facility, a patient recovery room, or a room in a healthcare facility specially equipped for surgical operations, to the cloud, in accordance with at least one aspect of the present disclosure.

As illustrated in FIG. 4, a surgical hub system 20060 may include a modular communication hub 20065 that is configured to connect modular devices located in a healthcare facility to a cloud-based system (e.g., a cloud computing system 20064 that may include a remote server 20067 coupled to a remote storage 20068). The modular communication hub 20065 and the devices may be connected in a room in a healthcare facility specially equipped for surgical operations. In one aspect, the modular communication hub 20065 may include a network hub 20061 and/or a network switch 20062 in communication with a network router 20066. The modular communication hub 20065 may be coupled to a local computer system 20063 to provide local computer processing and data manipulation.

The computer system 20063 may comprise a processor and a network interface 20100. The processor may be coupled to a communication module, storage, memory, non-volatile memory, and input/output (I/O) interface via a system bus. The system bus can be any of several types of bus structure(s) including the memory bus or memory controller, a peripheral bus or external bus, and or a local bus using any variety of available bus architectures including, but not limited to, 9-bit bus, Industrial Standard Architecture (ISA), Micro-Charmel Architecture (MSA), Extended ISA (EISA), Intelligent Drive Electronics (IDE), VESA Local Bus (VLB), Peripheral Component Interconnect (PCI), USB, Advanced Graphics Port (AGP), Personal Computer Memory Card International Association bus (PCMCIA), Small Computer Systems Interface (SCSI), or any other proprietary bus.

The processor may be any single-core or multicore processor such as those known under the trade name ARM Cortex by Texas Instruments. In one aspect, the processor may be an LM4F230H5QR ARM Cortex-M4F Processor Core, available from Texas Instruments, for example, comprising an on-chip memory of 256 KB single-cycle flash memory, or other non-volatile memory, up to 40 MHz, a prefetch buffer to improve performance above 40 MHz, a 32 KB single-cycle serial random access memory (SRAM), an internal read-only memory (ROM) loaded with StellarisWare^{®} software, a 2 KB electrically erasable programmable read-only memory (EEPROM), and/or one or more pulse width modulation (PWM) modules, one or more quadrature encoder inputs (QEI) analogs, one or more 12-bit analog-to-digital converters (ADCs) with 12 analog input channels, details of which are available for the product datasheet.

In an example, the processor may comprise a safety controller comprising two controller-based families such as TMS570 and RM4x, known under the trade name Hercules ARM Cortex R4, also by Texas Instruments. The safety controller may be configured specifically for IEC 61508 and ISO 26262 safety critical applications, among others, to provide advanced integrated safety features while delivering scalable performance, connectivity, and memory options.

It is to be appreciated that the computer system 20063 may include software that acts as an intermediary between users and the basic computer resources described in a suitable operating environment. Such software may include an operating system. The operating system, which can be stored on the disk storage, may act to control and allocate resources of the computer system. System applications may take advantage of the management of resources by the operating system through program modules and program data stored either in the system memory or on the disk storage. It is to be appreciated that various components described herein can be implemented with various operating systems or combinations of operating systems.

A user may enter commands or information into the computer system 20063 through input device(s) coupled to the I/O interface. The input devices may include, but are not limited to, a pointing device such as a mouse, trackball, stylus, touch pad, keyboard, microphone, joystick, game pad, satellite dish, scanner, TV tuner card, digital camera, digital video camera, web camera, and the like. These and other input devices connect to the processor 20102 through the system bus via interface port(s). The interface port(s) include, for example, a serial port, a parallel port, a game port, and a USB. The output device(s) use some of the same types of ports as input device(s). Thus, for example, a USB port may be used to provide input to the computer system 20063 and to output information from the computer system 20063 to an output device. An output adapter may be provided to illustrate that there can be some output devices like monitors, displays, speakers, and printers, among other output devices that may require special adapters. The output adapters may include, by way of illustration and not limitation, video and sound cards that provide a means of connection between the output device and the system bus. It should be noted that other devices and/or systems of devices, such as remote computer(s), may provide both input and output capabilities.

The computer system 20063 can operate in a networked environment using logical connections to one or more remote computers, such as cloud computer(s), or local computers. The remote cloud computer(s) can be a personal computer, server, router, network PC, workstation, microprocessor-based appliance, peer device, or other common network node, and the like, and typically includes many or all of the elements described relative to the computer system. For purposes of brevity, only a memory storage device is illustrated with the remote computer(s). The remote computer(s) may be logically connected to the computer system through a network interface and then physically connected via a communication connection. The network interface may encompass communication networks such as local area networks (LANs) and wide area networks (WANs). LAN technologies may include Fiber Distributed Data Interface (FDDI), Copper Distributed Data Interface (CDDI), Ethernet/IEEE 802.3, Token Ring/IEEE 802.5, and the like. WAN technologies may include, but are not limited to, point-to-point links, circuit-switching networks like Integrated Services Digital Networks (ISDN) and variations thereon, packet-switching networks, and Digital Subscriber Lines (DSL).

In various examples, the computer system 20063 may comprise an image processor, image-processing engine, media processor, or any specialized digital signal processor (DSP) used for the processing of digital images. The image processor may employ parallel computing with single instruction, multiple data (SIMD) or multiple instruction, multiple data (MIMD) technologies to increase speed and efficiency. The digital image-processing engine can perform a range of tasks. The image processor may be a system on a chip with multicore processor architecture.

The communication connection(s) may refer to the hardware/software employed to connect the network interface to the bus. While the communication connection is shown for illustrative clarity inside the computer system 20063, it can also be external to the computer system 20063. The hardware/software necessary for connection to the network interface may include, for illustrative purposes only, internal and external technologies such as modems, including regular telephone-grade modems, cable modems, optical fiber modems, and DSL modems, ISDN adapters, and Ethernet cards. In some examples, the network interface may also be provided using an RF interface.

Surgical data network associated with the surgical hub system 20060 may be configured as passive, intelligent, or switching. A passive surgical data network serves as a conduit for the data, enabling it to go from one device (or segment) to another and to the cloud computing resources. An intelligent surgical data network includes additional features to enable the traffic passing through the surgical data network to be monitored and to configure each port in the network hub 20061 or network switch 20062. An intelligent surgical data network may be referred to as a manageable hub or switch. A switching hub reads the destination address of each packet and then forwards the packet to the correct port.

Modular devices 1a-1n located in the operating theater may be coupled to the modular communication hub 20065. The network hub 20061 and/or the network switch 20062 may be coupled to a network router 20066 to connect the devices 1a-1n to the cloud computing system 20064 or the local computer system 20063. Data associated with the devices 1a-1n may be transferred to cloud-based computers via the router for remote data processing and manipulation. Data associated with the devices 1a-1n may also be transferred to the local computer system 20063 for local data processing and manipulation. Modular devices 2a-2m located in the same operating theater also may be coupled to a network switch 20062. The network switch 20062 may be coupled to the network hub 20061 and/or the network router 20066 to connect the devices 2a-2m to the cloud 20064. Data associated with the devices 2a-2m may be transferred to the cloud computing system 20064 via the network router 20066 for data processing and manipulation. Data associated with the devices 2a-2m may also be transferred to the local computer system 20063 for local data processing and manipulation.

The wearable sensing system 20011 may include one or more sensing systems 20069. The sensing systems 20069 may include an HCP sensing system and/or a patient sensing system. The one or more sensing systems 20069 may be in communication with the computer system 20063 of a surgical hub system 20060 or the cloud server 20067 directly via one of the network routers 20066 or via a network hub 20061 or network switching 20062 that is in communication with the network routers 20066.

The sensing systems 20069 may be coupled to the network router 20066 to connect to the sensing systems 20069 to the local computer system 20063 and/or the cloud computing system 20064. Data associated with the sensing systems 20069 may be transferred to the cloud computing system 20064 via the network router 20066 for data processing and manipulation. Data associated with the sensing systems 20069 may also be transferred to the local computer system 20063 for local data processing and manipulation.

As illustrated in FIG. 4, the surgical hub system 20060 may be expanded by interconnecting multiple network hubs 20061 and/or multiple network switches 20062 with multiple network routers 20066. The modular communication hub 20065 may be contained in a modular control tower configured to receive multiple devices 1a-1n/2a-2m. The local computer system 20063 also may be contained in a modular control tower. The modular communication hub 20065 may be connected to a display 20068 to display images obtained by some of the devices 1a-1n/2a-2m, for example during surgical procedures. In various aspects, the devices 1a-1n/2a-2m may include, for example, various modules such as an imaging module coupled to an endoscope, a generator module coupled to an energy-based surgical device, a smoke evacuation module, a suction/irrigation module, a communication module, a processor module, a storage array, a surgical device coupled to a display, and/or a non-contact sensor module, among other modular devices that may be connected to the modular communication hub 20065 of the surgical data network.

In one aspect, the surgical hub system 20060 illustrated in FIG. 4 may comprise a combination of network hub(s), network switch(es), and network router(s) connecting the devices 1a-1n/2a-2m or the sensing systems 20069 to the cloud-base system 20064. One or more of the devices 1a-1n/2a-2m or the sensing systems 20069 coupled to the network hub 20061 or network switch 20062 may collect data or measurement data in real-time and transfer the data to cloud computers for data processing and manipulation. It will be appreciated that cloud computing relies on sharing computing resources rather than having local servers or personal devices to handle software applications. The word "cloud" may be used as a metaphor for "the Internet," although the term is not limited as such. Accordingly, the term "cloud computing" may be used herein to refer to "a type of Internet-based computing," where different services-such as servers, storage, and applications-are delivered to the modular communication hub 20065 and/or computer system 20063 located in the surgical theater (e.g., a fixed, mobile, temporary, or field operating room or space) and to devices connected to the modular communication hub 20065 and/or computer system 20063 through the Internet. The cloud infrastructure may be maintained by a cloud service provider. In this context, the cloud service provider may be the entity that coordinates the usage and control of the devices 1a-1n/2a-2m located in one or more operating theaters. The cloud computing services can perform a large number of calculations based on the data gathered by smart surgical instruments, robots, sensing systems, and other computerized devices located in the operating theater. The hub hardware enables multiple devices, sensing systems, and/or connections to be connected to a computer that communicates with the cloud computing resources and storage.

Applying cloud computer data processing techniques on the data collected by the devices 1a-1n/2a-2m, the surgical data network can provide improved surgical outcomes, reduced costs, and improved patient satisfaction. At least some of the devices 1a-1n/2a-2m may be employed to view tissue states to assess leaks or perfusion of sealed tissue after a tissue sealing and cutting procedure. At least some of the devices 1a-1n/2a-2m may be employed to identify pathology, such as the effects of diseases, using the cloud-based computing to examine data including images of samples of body tissue for diagnostic purposes. This may include localization and margin confirmation of tissue and phenotypes. At least some of the devices 1a-1n/2a-2m may be employed to identify anatomical structures of the body using a variety of sensors integrated with imaging devices and techniques such as overlaying images captured by multiple imaging devices. The data gathered by the devices 1a-1n/2a-2m, including image data, may be transferred to the cloud computing system 20064 or the local computer system 20063 or both for data processing and manipulation including image processing and manipulation. The data may be analyzed to improve surgical procedure outcomes by determining if further treatment, such as the application of endoscopic intervention, emerging technologies, a targeted radiation, targeted intervention, and precise robotics to tissue-specific sites and conditions, may be pursued. Such data analysis may further employ outcome analytics processing and using standardized approaches may provide beneficial feedback to either confirm surgical treatments and the behavior of the surgeon or suggest modifications to surgical treatments and the behavior of the surgeon.

Applying cloud computer data processing techniques on the measurement data collected by the sensing systems 20069, the surgical data network can provide improved surgical outcomes, improved recovery outcomes, reduced costs, and improved patient satisfaction. At least some of the sensing systems 20069 may be employed to assess physiological conditions of a surgeon operating on a patient or a patient being prepared for a surgical procedure or a patient recovering after a surgical procedure. The cloud-based computing system 20064 may be used to monitor biomarkers associated with a surgeon or a patient in real-time and to generate surgical plans based at least on measurement data gathered prior to a surgical procedure, provide control signals to the surgical instruments during a surgical procedure, notify a patient of a complication during post-surgical period.

The operating theater devices 1a-1n may be connected to the modular communication hub 20065 over a wired channel or a wireless channel depending on the configuration of the devices 1a-1n to a network hub 20061. The network hub 20061 may be implemented, in one aspect, as a local network broadcast device that works on the physical layer of the Open System Interconnection (OSI) model. The network hub may provide connectivity to the devices 1a-1n located in the same operating theater network. The network hub 20061 may collect data in the form of packets and sends them to the router in half duplex mode. The network hub 20061 may not store any media access control/Internet Protocol (MAC/IP) to transfer the device data. Only one of the devices 1a-1n can send data at a time through the network hub 20061. The network hub 20061 may not have routing tables or intelligence regarding where to send information and broadcasts all network data across each connection and to a remote server 20067 of the cloud computing system 20064. The network hub 20061 can detect basic network errors such as collisions but having all information broadcast to multiple ports can be a security risk and cause bottlenecks.

The operating theater devices 2a-2m may be connected to a network switch 20062 over a wired channel or a wireless channel. The network switch 20062 works in the data link layer of the OSI model. The network switch 20062 may be a multicast device for connecting the devices 2a-2m located in the same operating theater to the network. The network switch 20062 may send data in the form of frames to the network router 20066 and may work in full duplex mode. Multiple devices 2a-2m can send data at the same time through the network switch 20062. The network switch 20062 stores and uses MAC addresses of the devices 2a-2m to transfer data.

The network hub 20061 and/or the network switch 20062 may be coupled to the network router 20066 for connection to the cloud computing system 20064. The network router 20066 works in the network layer of the OSI model. The network router 20066 creates a route for transmitting data packets received from the network hub 20061 and/or network switch 20062 to cloud-based computer resources for further processing and manipulation of the data collected by any one of or all the devices 1a-1n/2a-2m and wearable sensing system 20011. The network router 20066 may be employed to connect two or more different networks located in different locations, such as, for example, different operating theaters of the same healthcare facility or different networks located in different operating theaters of different healthcare facilities. The network router 20066 may send data in the form of packets to the cloud computing system 20064 and works in full duplex mode. Multiple devices can send data at the same time. The network router 20066 may use IP addresses to transfer data.

In an example, the network hub 20061 may be implemented as a USB hub, which allows multiple USB devices to be connected to a host computer. The USB hub may expand a single USB port into several tiers so that there are more ports available to connect devices to the host system computer. The network hub 20061 may include wired or wireless capabilities to receive information over a wired channel or a wireless channel. In one aspect, a wireless USB short-range, high-bandwidth wireless radio communication protocol may be employed for communication between the devices 1a-1n and devices 2a-2m located in the operating theater.

In examples, the operating theater devices 1a-1n/2a-2m and/or the sensing systems 20069 may communicate to the modular communication hub 20065 via Bluetooth wireless technology standard for exchanging data over short distances (using short-wavelength UHF radio waves in the ISM band from 2.4 to 2.485 GHz) from fixed and mobile devices and building personal area networks (PANs). The operating theater devices 1a-1n/2a-2m and/or the sensing systems 20069 may communicate to the modular communication hub 20065 via a number of wireless or wired communication standards or protocols, including but not limited to Bluetooth, Low-Energy Bluetooth, near-field communication (NFC), Wi-Fi (IEEE 802.11 family), WiMAX (IEEE 802.16 family), IEEE 802.20, new radio (NR), long-term evolution (LTE), and Ev-DO, HSPA+, HSDPA+, HSUPA+, EDGE, GSM, GPRS, CDMA, TDMA, DECT, and Ethernet derivatives thereof, as well as any other wireless and wired protocols that are designated as 3G, 4G, 5G, and beyond. The computing module may include a plurality of communication modules. For instance, a first communication module may be dedicated to shorter-range wireless communications such as Wi-Fi and Bluetooth Low-Energy Bluetooth, Bluetooth Smart, and a second communication module may be dedicated to longer-range wireless communications such as GPS, EDGE, GPRS, CDMA, WiMAX, LTE, Ev-DO, HSPA+, HSDPA+, HSUPA+, EDGE, GSM, GPRS, CDMA, TDMA, and others.

The modular communication hub 20065 may serve as a central connection for one or more of the operating theater devices 1a-1n/2a-2m and/or the sensing systems 20069 and may handle a data type known as frames. Frames may carry the data generated by the devices 1a-1n/2a-2m and/or the sensing systems 20069. When a frame is received by the modular communication hub 20065, it may be amplified and/or sent to the network router 20066, which may transfer the data to the cloud computing system 20064 or the local computer system 20063 by using a number of wireless or wired communication standards or protocols, as described herein.

The modular communication hub 20065 can be used as a standalone device or be connected to compatible network hubs 20061 and network switches 20062 to form a larger network. The modular communication hub 20065 can be generally easy to install, configure, and maintain, making it a good option for networking the operating theater devices 1a-1n/2a-2m.

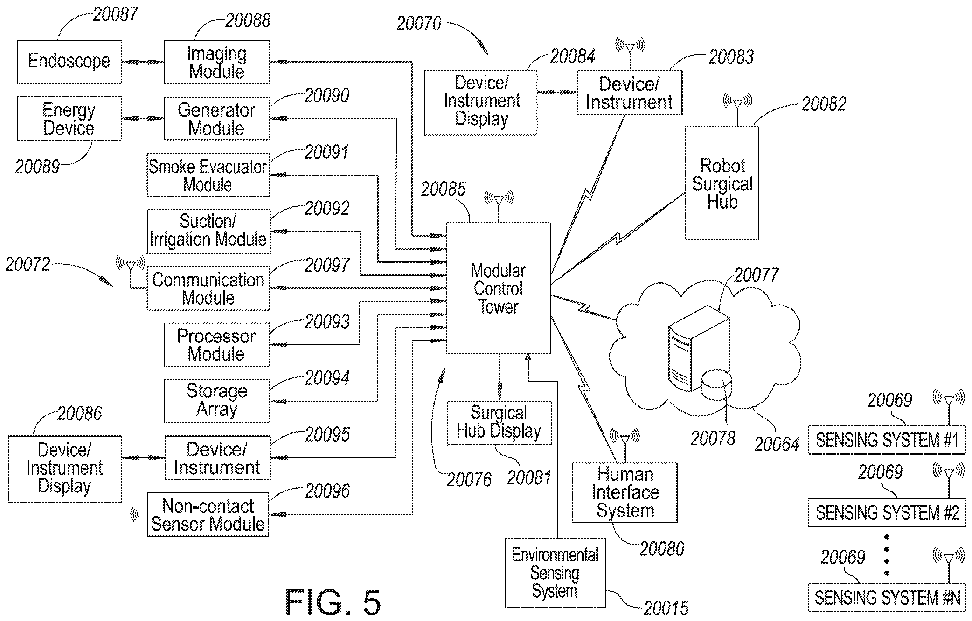

FIG. 5 illustrates a computer-implemented interactive surgical system 20070 that may be a part of the HCP monitoring system 20002. The computer-implemented interactive surgical system 20070 is similar in many respects to the HCP sensing system 20002. For example, the computer-implemented interactive surgical system 20070 may include one or more surgical sub-systems 20072, which are similar in many respects to the HCP monitoring systems 20002. Each sub-surgical system 20072 may include at least one surgical hub 20076 in communication with a cloud computing system 20064 that may include a remote server 20077 and a remote storage 20078. In one aspect, the computer-implemented interactive surgical system 20070 may include a modular control tower 20085 connected to multiple operating theater devices such as sensing systems 20001, intelligent surgical instruments, robots, and other computerized devices located in the operating theater.

As illustrated in the example of FIG. 5, the modular control tower 20085 may be coupled to an imaging module 20088 that may be coupled to an endoscope 20087, a generator module 20090 that may be coupled to an energy device 20089, a smoke evacuator module 20091, a suction/irrigation module 20092, a communication module 20097, a processor module 20093, a storage array 20094, a smart device/instrument 20095 optionally coupled to a display 20086 and 20084 respectively, and a non-contact sensor module 20096. The non-contact sensor module 20096 may measure the dimensions of the operating theater and generate a map of the surgical theater using, ultrasonic, laser-type, and/or the like, non-contact measurement devices. Other distance sensors can be employed to determine the bounds of an operating room. An ultrasound-based non-contact sensor module may scan the operating theater by transmitting a burst of ultrasound and receiving the echo when it bounces off the perimeter walls of an operating theater as described under the heading "Surgical Hub Spatial Awareness Within an Operating Room" in U.S. Provisional Patent Application Serial No. 62/611,341, titled INTERACTIVE SURGICAL PLATFORM, filed December 2017. The sensor module may be configured to determine the size of the operating theater and to adjust Bluetooth-pairing distance limits. A laser-based non-contact sensor module may scan the operating theater by transmitting laser light pulses, receiving laser light pulses that bounce off the perimeter walls of the operating theater, and comparing the phase of the transmitted pulse to the received pulse to determine the size of the operating theater and to adjust Bluetooth pairing distance limits, for example.

The modular control tower 20085 may also be in communication with one or more sensing systems 20069 and an environmental sensing system 20015. The sensing systems 20069 may be connected to the modular control tower 20085 either directly via a router or via the communication module 20097. The operating theater devices may be coupled to cloud computing resources and data storage via the modular control tower 20085. A robot surgical hub 20082 also may be connected to the modular control tower 20085 and to the cloud computing resources. The devices/instruments 20095 or 20084, human interface system 20080, among others, may be coupled to the modular control tower 20085 via wired or wireless communication standards or protocols, as described herein. The human interface system 20080 may include a display sub-system and a notification sub-system. The modular control tower 20085 may be coupled to a hub display 20081 (e.g., monitor, screen) to display and overlay images received from the imaging module 20088, device/instrument display 20086, and/or other human interface systems 20080. The hub display 20081 also may display data received from devices connected to the modular control tower 20085 in conjunction with images and overlaid images.

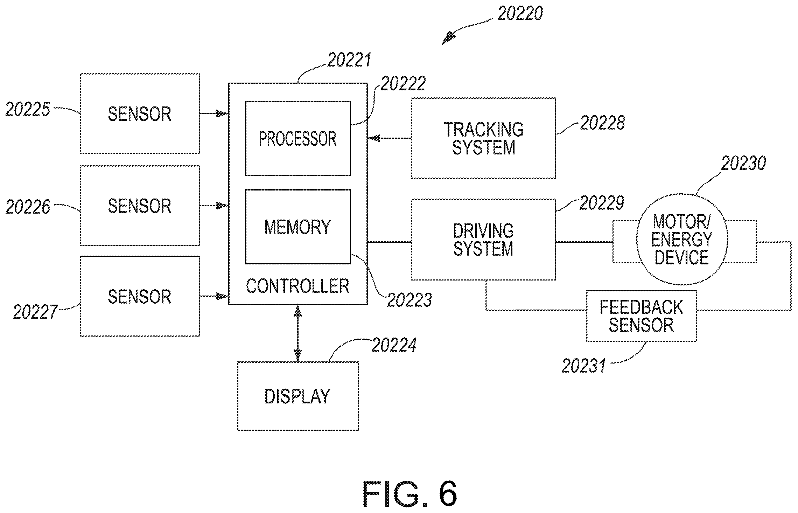

FIG. 6 illustrates a logical diagram of a control system 20220 of a surgical instrument or a surgical tool in accordance with one or more aspects of the present disclosure. The surgical instrument or the surgical tool may be configurable. The surgical instrument may include surgical fixtures specific to the procedure at-hand, such as imaging devices, surgical staplers, energy devices, endocutter devices, or the like. For example, the surgical instrument may include any of a powered stapler, a powered stapler generator, an energy device, an advanced energy device, an advanced energy jaw device, an endocutter clamp, an energy device generator, an in-operating-room imaging system, a smoke evacuator, a suction-irrigation device, an insufflation system, or the like. The system 20220 may comprise a control circuit. The control circuit may include a microcontroller 20221 comprising a processor 20222 and a memory 20223. One or more of sensors 20225, 20226, 20227, for example, provide real-time feedback to the processor 20222. A motor 20230, driven by a motor driver 20229, operably couples a longitudinally movable displacement member to drive the I-beam knife element. A tracking system 20228 may be configured to determine the position of the longitudinally movable displacement member. The position information may be provided to the processor 20222, which can be programmed or configured to determine the position of the longitudinally movable drive member as well as the position of a firing member, firing bar, and I-beam knife element. Additional motors may be provided at the tool driver interface to control I-beam firing, closure tube travel, shaft rotation, and articulation. A display 20224 may display a variety of operating conditions of the instruments and may include touch screen functionality for data input. Information displayed on the display 20224 may be overlaid with images acquired via endoscopic imaging modules.

The microcontroller 20221 may be any single-core or multicore processor such as those known under the trade name ARM Cortex by Texas Instruments. In one aspect, the main microcontroller 20221 may be an LM4F230H5QR ARM Cortex-M4F Processor Core, available from Texas Instruments, for example, comprising an on-chip memory of 256 KB single-cycle flash memory, or other non-volatile memory, up to 40 MHz, a prefetch buffer to improve performance above 40 MHz, a 32 KB single-cycle SRAM, and internal ROM loaded with StellarisWare^{®} software, a 2 KB EEPROM, one or more PWM modules, one or more QEI analogs, and/or one or more 12-bit ADCs with 12 analog input channels, details of which are available for the product datasheet.

The microcontroller 20221 may comprise a safety controller comprising two controller-based families such as TMS570 and RM4x, known under the trade name Hercules ARM Cortex R4, also by Texas Instruments. The safety controller may be configured specifically for IEC 61508 and ISO 26262 safety critical applications, among others, to provide advanced integrated safety features while delivering scalable performance, connectivity, and memory options.

The microcontroller 20221 may be programmed to perform various functions such as precise control over the speed and position of the knife and articulation systems. In one aspect, the microcontroller 20221 may include a processor 20222 and a memory 20223. The electric motor 20230 may be a brushed direct current (DC) motor with a gearbox and mechanical links to an articulation or knife system. In one aspect, a motor driver 20229 may be an A3941 available from Allegro Microsystems, Inc. Other motor drivers may be readily substituted for use in the tracking system 20228 comprising an absolute positioning system. A detailed description of an absolute positioning system is described in U.S. Patent Application Publication No. 2017/0296213, titled SYSTEMS AND METHODS FOR CONTROLLING A SURGICAL STAPLING AND CUTTING INSTRUMENT, which published on October 19, 2017.

The microcontroller 20221 may be programmed to provide precise control over the speed and position of displacement members and articulation systems. The microcontroller 20221 may be configured to compute a response in the software of the microcontroller 20221. The computed response may be compared to a measured response of the actual system to obtain an "observed" response, which is used for actual feedback decisions. The observed response may be a favorable, tuned value that balances the smooth, continuous nature of the simulated response with the measured response, which can detect outside influences on the system.

The motor 20230 may be controlled by the motor driver 20229 and can be employed by the firing system of the surgical instrument or tool. In various forms, the motor 20230 may be a brushed DC driving motor having a maximum rotational speed of approximately 25,000 RPM. In some examples, the motor 20230 may include a brushless motor, a cordless motor, a synchronous motor, a stepper motor, or any other suitable electric motor. The motor driver 20229 may comprise an H-bridge driver comprising field-effect transistors (FETs), for example. The motor 20230 can be powered by a power assembly releasably mounted to the handle assembly or tool housing for supplying control power to the surgical instrument or tool. The power assembly may comprise a battery which may include a number of battery cells connected in series that can be used as the power source to power the surgical instrument or tool. In certain circumstances, the battery cells of the power assembly may be replaceable and/or rechargeable. In at least one example, the battery cells can be lithium-ion batteries which can be couplable to and separable from the power assembly.

The motor driver 20229 may be an A3941 available from Allegro Microsystems, Inc. A3941 may be a full-bridge controller for use with external N-channel power metal-oxide semiconductor field-effect transistors (MOSFETs) specifically designed for inductive loads, such as brush DC motors. The driver 20229 may comprise a unique charge pump regulator that can provide full (>10 V) gate drive for battery voltages down to 7 V and can allow the A3941 to operate with a reduced gate drive, down to 5.5 V. A bootstrap capacitor may be employed to provide the above battery supply voltage required for N-channel MOSFETs. An internal charge pump for the high-side drive may allow DC (100% duty cycle) operation. The full bridge can be driven in fast or slow decay modes using diode or synchronous rectification. In the slow decay mode, current recirculation can be through the high-side or the low-side FETs. The power FETs may be protected from shoot-through by resistor-adjustable dead time. Integrated diagnostics provide indications of undervoltage, overtemperature, and power bridge faults and can be configured to protect the power MOSFETs under most short circuit conditions. Other motor drivers may be readily substituted for use in the tracking system 20228 comprising an absolute positioning system.

The tracking system 20228 may comprise a controlled motor drive circuit arrangement comprising a position sensor 20225 according to one aspect of this disclosure. The position sensor 20225 for an absolute positioning system may provide a unique position signal corresponding to the location of a displacement member. In some examples, the displacement member may represent a longitudinally movable drive member comprising a rack of drive teeth for meshing engagement with a corresponding drive gear of a gear reducer assembly. In some examples, the displacement member may represent the firing member, which could be adapted and configured to include a rack of drive teeth. In some examples, the displacement member may represent a firing bar or the I-beam, each of which can be adapted and configured to include a rack of drive teeth. Accordingly, as used herein, the term displacement member can be used generically to refer to any movable member of the surgical instrument or tool such as the drive member, the firing member, the firing bar, the I-beam, or any element that can be displaced. In one aspect, the longitudinally movable drive member can be coupled to the firing member, the firing bar, and the I-beam. Accordingly, the absolute positioning system can, in effect, track the linear displacement of the I-beam by tracking the linear displacement of the longitudinally movable drive member. In various aspects, the displacement member may be coupled to any position sensor 20225 suitable for measuring linear displacement. Thus, the longitudinally movable drive member, the firing member, the firing bar, or the I-beam, or combinations thereof, may be coupled to any suitable linear displacement sensor. Linear displacement sensors may include contact or non-contact displacement sensors. Linear displacement sensors may comprise linear variable differential transformers (LVDT), differential variable reluctance transducers (DVRT), a slide potentiometer, a magnetic sensing system comprising a movable magnet and a series of linearly arranged Hall effect sensors, a magnetic sensing system comprising a fixed magnet and a series of movable, linearly arranged Hall effect sensors, an optical sensing system comprising a movable light source and a series of linearly arranged photo diodes or photo detectors, an optical sensing system comprising a fixed light source and a series of movable linearly, arranged photodiodes or photodetectors, or any combination thereof.

The electric motor 20230 can include a rotatable shaft that operably interfaces with a gear assembly that is mounted in meshing engagement with a set, or rack, of drive teeth on the displacement member. A sensor element may be operably coupled to a gear assembly such that a single revolution of the position sensor 20225 element corresponds to some linear longitudinal translation of the displacement member. An arrangement of gearing and sensors can be connected to the linear actuator, via a rack and pinion arrangement, or a rotary actuator, via a spur gear or other connection. A power source may supply power to the absolute positioning system and an output indicator may display the output of the absolute positioning system. The displacement member may represent the longitudinally movable drive member comprising a rack of drive teeth formed thereon for meshing engagement with a corresponding drive gear of the gear reducer assembly. The displacement member may represent the longitudinally movable firing member, firing bar, I-beam, or combinations thereof.

A single revolution of the sensor element associated with the position sensor 20225 may be equivalent to a longitudinal linear displacement d1 of the of the displacement member, where d1 is the longitudinal linear distance that the displacement member moves from point "a" to point "b" after a single revolution of the sensor element coupled to the displacement member. The sensor arrangement may be connected via a gear reduction that results in the position sensor 20225 completing one or more revolutions for the full stroke of the displacement member. The position sensor 20225 may complete multiple revolutions for the full stroke of the displacement member.

A series of switches, where n is an integer greater than one, may be employed alone or in combination with a gear reduction to provide a unique position signal for more than one revolution of the position sensor 20225. The state of the switches may be fed back to the microcontroller 20221 that applies logic to determine a unique position signal corresponding to the longitudinal linear displacement d1 + d2 + ... dn of the displacement member. The output of the position sensor 20225 is provided to the microcontroller 20221. The position sensor 20225 of the sensor arrangement may comprise a magnetic sensor, an analog rotary sensor like a potentiometer, or an array of analog Hall-effect elements, which output a unique combination of position signals or values.

The position sensor 20225 may comprise any number of magnetic sensing elements, such as, for example, magnetic sensors classified according to whether they measure the total magnetic field or the vector components of the magnetic field. The techniques used to produce both types of magnetic sensors may encompass many aspects of physics and electronics. The technologies used for magnetic field sensing may include search coil, fluxgate, optically pumped, nuclear precession, SQUID, Hall-effect, anisotropic magnetoresistance, giant magnetoresistance, magnetic tunnel junctions, giant magnetoimpedance, magnetostrictive/piezoelectric composites, magnetodiode, magnetotransistor, fiber-optic, magneto-optic, and microelectromechanical systems-based magnetic sensors, among others.

The position sensor 20225 for the tracking system 20228 comprising an absolute positioning system may comprise a magnetic rotary absolute positioning system. The position sensor 20225 may be implemented as an AS5055EQFT single-chip magnetic rotary position sensor available from Austria Microsystems, AG. The position sensor 20225 is interfaced with the microcontroller 20221 to provide an absolute positioning system. The position sensor 20225 may be a low-voltage and low-power component and may include four Hall-effect elements in an area of the position sensor 20225 that may be located above a magnet. A high-resolution ADC and a smart power management controller may also be provided on the chip. A coordinate rotation digital computer (CORDIC) processor, also known as the digit-by-digit method and Volder's algorithm, may be provided to implement a simple and efficient algorithm to calculate hyperbolic and trigonometric functions that require only addition, subtraction, bit-shift, and table lookup operations. The angle position, alarm bits, and magnetic field information may be transmitted over a standard serial communication interface, such as a serial peripheral interface (SPI) interface, to the microcontroller 20221. The position sensor 20225 may provide 12 or 14 bits of resolution. The position sensor 20225 may be an AS5055 chip provided in a small QFN 16-pin 4x4x0.85mm package.

The tracking system 20228 comprising an absolute positioning system may comprise and/or be programmed to implement a feedback controller, such as a PID, state feedback, and adaptive controller. A power source converts the signal from the feedback controller into a physical input to the system: in this case the voltage. Other examples include a PWM of the voltage, current, and force. Other sensor(s) may be provided to measure physical parameters of the physical system in addition to the position measured by the position sensor 20225. In some aspects, the other sensor(s) can include sensor arrangements such as those described in U.S. Patent No. 9,345,481, titled STAPLE CARTRIDGE TISSUE THICKNESS SENSOR SYSTEM, which issued on May 24, 2016; U.S. Patent Application Publication No. 2014/0263552, titled STAPLE CARTRIDGE TISSUE THICKNESS SENSOR SYSTEM, which published on September 18, 2014; and U.S. Patent Application Serial No. 15/628,175, titled TECHNIQUES FOR ADAPTIVE CONTROL OF MOTOR VELOCITY OF A SURGICAL STAPLING AND CUTTING INSTRUMENT, filed June 20, 2017. In a digital signal processing system, an absolute positioning system is coupled to a digital data acquisition system where the output of the absolute positioning system will have a finite resolution and sampling frequency. The absolute positioning system may comprise a compare-and-combine circuit to combine a computed response with a measured response using algorithms, such as a weighted average and a theoretical control loop, that drive the computed response towards the measured response. The computed response of the physical system may take into account properties like mass, inertia, viscous friction, inductance resistance, etc., to predict what the states and outputs of the physical system will be by knowing the input.

The absolute positioning system may provide an absolute position of the displacement member upon power-up of the instrument, without retracting or advancing the displacement member to a reset (zero or home) position as may be required with conventional rotary encoders that merely count the number of steps forwards or backwards that the motor 20230 has taken to infer the position of a device actuator, drive bar, knife, or the like.

A sensor 20226, such as, for example, a strain gauge or a micro-strain gauge, may be configured to measure one or more parameters of the end effector, such as, for example, the amplitude of the strain exerted on the anvil during a clamping operation, which can be indicative of the closure forces applied to the anvil. The measured strain may be converted to a digital signal and provided to the processor 20222. Alternatively, or in addition to the sensor 20226, a sensor 20227, such as, for example, a load sensor, can measure the closure force applied by the closure drive system to the anvil. The sensor 20227, such as, for example, a load sensor, can measure the firing force applied to an I-beam in a firing stroke of the surgical instrument or tool. The I-beam is configured to engage a wedge sled, which is configured to upwardly cam staple drivers to force out staples into deforming contact with an anvil. The I-beam also may include a sharpened cutting edge that can be used to sever tissue as the I-beam is advanced distally by the firing bar. Alternatively, a current sensor 20231 can be employed to measure the current drawn by the motor 20230. The force required to advance the firing member can correspond to the current drawn by the motor 20230, for example. The measured force may be converted to a digital signal and provided to the processor 20222.

For example, the strain gauge sensor 20226 can be used to measure the force applied to the tissue by the end effector. A strain gauge can be coupled to the end effector to measure the force on the tissue being treated by the end effector. A system for measuring forces applied to the tissue grasped by the end effector may comprise a strain gauge sensor 20226, such as, for example, a micro-strain gauge, that can be configured to measure one or more parameters of the end effector, for example. In one aspect, the strain gauge sensor 20226 can measure the amplitude or magnitude of the strain exerted on a jaw member of an end effector during a clamping operation, which can be indicative of the tissue compression. The measured strain can be converted to a digital signal and provided to a processor 20222 of the microcontroller 20221. A load sensor 20227 can measure the force used to operate the knife element, for example, to cut the tissue captured between the anvil and the staple cartridge. A magnetic field sensor can be employed to measure the thickness of the captured tissue. The measurement of the magnetic field sensor also may be converted to a digital signal and provided to the processor 20222.

The measurements of the tissue compression, the tissue thickness, and/or the force required to close the end effector on the tissue, as respectively measured by the sensors 20226, 20227, can be used by the microcontroller 20221 to characterize the selected position of the firing member and/or the corresponding value of the speed of the firing member. In one instance, a memory 20223 may store a technique, an equation, and/or a lookup table which can be employed by the microcontroller 20221 in the assessment.

The control system 20220 of the surgical instrument or tool also may comprise wired or wireless communication circuits to communicate with the modular communication hub 20065 as shown in FIG. 5.

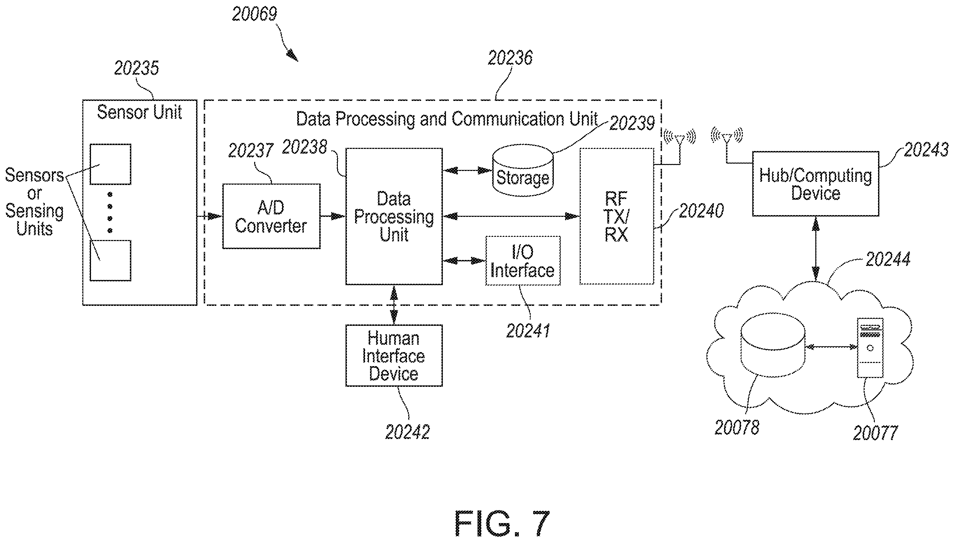

FIG. 7 shows an example sensing system 20069. The sensing system may be an HCP sensing system or a patient sensing system. The sensing system 20069 may include a sensor unit 20235 and a human interface system 20242 that are in communication with a data processing and communication unit 20236. The data processing and communication unit 20236 may include an analog-to-digital converted 20237, a data processing unit 20238, a storage unit 20239, and an input/output interface 20241, a transceiver 20240. The sensing system 20069 may be in communication with a surgical hub or a computing device 20243, which in turn is in communication with a cloud computing system 20244. The cloud computing system 20244 may include a cloud storage system 20078 and one or more cloud servers 20077.

The sensor unit 20235 may include one or more ex vivo or in vivo sensors for measuring one or more biomarkers. The biomarkers may include, for example, blood pH, hydration state, oxygen saturation, core body temperature, heart rate, heart rate variability, sweat rate, skin conductance, blood pressure, light exposure, environmental temperature, respiratory rate, coughing and sneezing, gastrointestinal motility, gastrointestinal tract imaging, tissue perfusion pressure, bacteria in respiratory tract, alcohol consumption, lactate (sweat), peripheral temperature, positivity and optimism, adrenaline (sweat), cortisol (sweat), edema, mycotoxins, VO2 max, pre-operative pain, chemicals in the air, circulating tumor cells, stress and anxiety, confusion and delirium, physical activity, autonomic tone, circadian rhythm, menstrual cycle, sleep, etc. These biomarkers may be measured using one or more sensors, for example, photosensors (e.g., photodiodes, photoresistors), mechanical sensors (e.g., motion sensors), acoustic sensors, electrical sensors, electrochemical sensors, thermoelectric sensors, infrared sensors, etc. The sensors may measure the biomarkers as described herein using one of more of the following sensing technologies: photoplethysmography, electrocardiography, electroencephalography, colorimetry, impedimentary, potentiometry, amperometry, etc.

As illustrated in FIG. 7, a sensor in the sensor unit 20235 may measure a physiological signal (e.g., a voltage, a current, a PPG signal, etc.) associated with a biomarker to be measured. The physiological signal to be measured may depend on the sensing technology used, as described herein. The sensor unit 20235 of the sensing system 20069 may be in communication with the data processing and communication unit 20236. In an example, the sensor unit 20235 may communicate with the data processing and communication unit 20236 using a wireless interface. The data processing and communication unit 20236 may include an analog-to-digital converter (ADC) 20237, a data processing unit 20238, a storage 20239, an I/O interface 20241, and an RF transceiver 20240. The data processing unit 20238 may include a processor and a memory unit.

The sensor unit 20235 may transmit the measured physiological signal to the ADC 20237 of the data processing and communication unit 20236. In an example, the measured physiological signal may be passed through one or more filters (e.g., an RC low-pass filter) before being sent to the ADC. The ADC may convert the measured physiological signal into measurement data associated with the biomarker. The ADC may pass measurement data to the data processing unit 20238 for processing. In an example, the data processing unit 20238 may send the measurement data associated with the biomarker to a surgical hub or a computing device 20243, which in turn may send the measurement data to a cloud computing system 20244 for further processing. The data processing unit may send the measurement data to the surgical hub or the computing device 20243 using one of the wireless protocols, as described herein. In an example, the data processing unit 20238 may first process the raw measurement data received from the sensor unit and send the processed measurement data to the surgical hub or a computing device 20243.

In an example, the data processing and communication unit 20236 of the sensing system 20069 may receive a threshold value associated with a biomarker for monitoring from a surgical hub, a computing device 20243, or directly from a cloud server 20077 of the cloud computing system 20244. The data processing unit 20236 may compare the measurement data associated with the biomarker to be monitored with the corresponding threshold value received from the surgical hub, the computing device 20243, or the cloud server 20077. The data processing and communication unit 20236 may send a notification message to the HID 20242 indicating that a measurement data value has crossed the threshold value. The notification message may include the measurement data associated with the monitored biomarker. The data processing and computing unit 20236 may send a notification via a transmission to a surgical hub or a computing device 20243 using one of the following RF protocols: Bluetooth, Bluetooth Low-Energy (BLE), Bluetooth Smart, Zigbee, Z-wave, IPv6 Low-power wireless Personal Area Network (6LoWPAN), Wi-Fi. The data processing unit 20238 may send a notification (e.g., a notification for an HCP) directly to a cloud server via a transmission to a cellular transmission/reception point (TRP) or a base station using one or more of the following cellular protocols: GSM/GPRS/EDGE (2G), UMTS/HSPA (3G), long term evolution (LTE) or 4G, LTE-Advanced (LTE-A), new radio (NR) or 5G. In an example, the sensing unit may be in communication with the hub/computing device via a router.

In an example, the sensor unit may include a sensor and an analog-to-digital converted (ADC). The ADC in the sensor unit may convert a physiological signal measured by the sensor into measurement data associated with a biomarker. The sensor unit may send the measurement data to the data processing and communication unit for further processing. In an example, the sensor unit may send the measurement data to the data processing and communication unit using an inter-integrated circuit (I2C) interface.

The data processing and communication unit includes a data processing unit, a storage unit, and an RF transceiver. The sensing system may be in communication with a surgical hub or a computing device, which in turn may be in communication with a cloud computing system 20244. The cloud computing system 20244 may include a remote server 20077 and an associated remote storage 20078. The sensor unit may include one or more ex vivo or in vivo sensors for measuring one or more biomarkers, as described herein.

The data processing and communication unit after processing the measurement data received from the sensor unit may further process the measurement data and/or send the measurement data to the smart hub or the computing device 20243. In an example, the data processing and communication unit may send the measurement data received from the sensor unit to the remote server 20077 of the cloud computing system 20244 for further processing and/or monitoring.

In an example, the sensor unit may include multiple sensors to measure one or more physiological signals associated with a patient or surgeon's biomarkers and/or one or more physical state signals associated with physical state of a person. A list of biomarkers may include biomarkers such as those biomarkers disclosed herein. The ADC(s) in the sensor unit may convert each of the physiological signals and/or physical state signals measured by the multiple sensors into respective measurement data. The sensor unit may send the measurement data associated with one or more biomarkers as well as the physical state of the person being monitored to the data processing and communication unit for further processing. The sensor unit may send the measurement data to the data processing and communication unit individually for each of the sensors or combined for all the sensors. In an example, the sensor unit may send the measurement data to the data processing and communication unit via an I2C interface.

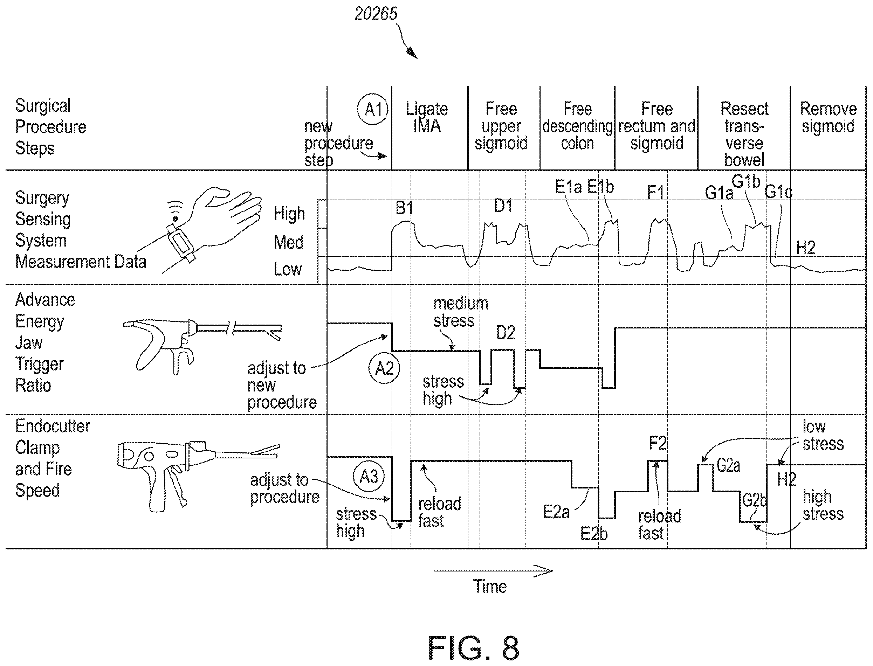

FIG. 8 is an example of using a surgical task situational awareness and measurement data from one or more HCP sensing systems to adjust surgical instrument controls. FIG. 8 illustrates a timeline 20265 of an illustrative surgical procedure and the contextual information that a surgical hub can derive from data received from one or more surgical devices, one or more HCP sensing systems, and/or one or more environmental sensing systems at each step in the surgical procedure. The devices that could be controlled by a surgical hub may include advanced energy devices, endocutter clamps, etc. The HCP sensing systems may include sensing systems for measuring one or more biomarkers associated with the surgeon, for example, heart rate, sweat composition, respiratory rate, etc. The environmental sensing system may include systems for measuring one or more of the environmental attributes, for example, cameras for detecting a surgeon's position/movements/breathing pattern, spatial microphones, for example to measure ambient noise in the surgical theater and/or the tone of voice of a healthcare provider, temperature/humidity of the surroundings, etc.

In the following description of the timeline 20265 illustrated in FIG. 8, reference should also be made to FIG. 5. FIG. 5 provides various components used in a surgical procedure. The timeline 20265 depicts the steps that may be taken individually and/or collectively by the nurses, surgeons, and other medical personnel during the course of an exemplary colorectal surgical procedure. In a colorectal surgical procedure, a situationally aware surgical hub 20076 may receive data from various data sources throughout the course of the surgical procedure, including data generated each time an HCP utilizes a modular device /instrument 20095 that is paired with the surgical hub 20076. The surgical hub 20076 may receive this data from the paired modular devices 20095. The surgical hub may receive measurement data from sensing systems 20069. The surgical hub may use the data from the modular device/instruments 20095 and/or measurement data from the sensing systems 20069 to continually derive inferences (i.e., contextual information) about an HCP's stress level and the ongoing procedure as new data is received, such that the stress level of the surgeon relative to the step of the procedure that is being performed is obtained. The situational awareness system of the surgical hub 20076 may perform one or more of the following: record data pertaining to the procedure for generating reports, verify the steps being taken by the medical personnel, provide data or prompts (e.g., via a display screen) that may be pertinent for the particular procedural step, adjust modular devices based on the context (e.g., activate monitors, adjust the FOV of the medical imaging device, change the energy level of an ultrasonic surgical instrument or RF electrosurgical instrument), or take any other such action described herein. In an example, these steps may be performed by a remote server 20077 of a cloud system 20064 and communicated with the surgical hub 20076.

As a first step (not shown in FIG. 8 for brevity), the hospital staff members may retrieve the patient's EMR from the hospital's EMR database. Based on select patient data in the EMR, the surgical hub 20076 may determine that the procedure to be performed is a colorectal procedure. The staff members may scan the incoming medical supplies for the procedure. The surgical hub 20076 may cross-reference the scanned supplies with a list of supplies that can be utilized in various types of procedures and confirms that the mix of supplies corresponds to a colorectal procedure. The surgical hub 20076 may pair each of the sensing systems 20069 worn by different HCPs.

Once each of the devices is ready and pre-surgical preparation is complete, the surgical team may begin by making incisions and place trocars. The surgical team may perform access and prep by dissecting adhesions, if any, and identifying inferior mesenteric artery (IMA) branches. The surgical hub 20076 can infer that the surgeon is in the process of dissecting adhesions, at least based on the data it may receive from the RF or ultrasonic generator indicating that an energy instrument is being fired. The surgical hub 20076 may cross-reference the received data with the retrieved steps of the surgical procedure to determine that an energy instrument being fired at this point in the process (e.g., after the completion of the previously discussed steps of the procedure) corresponds to the dissection step.

After dissection, the HCP may proceed to the ligation step (e.g., indicated by A1) of the procedure. As illustrated in FIG. 8, the HCP may begin by ligating the IMA. The surgical hub 20076 may infer that the surgeon is ligating arteries and veins because it may receive data from the advanced energy jaw device and/or the endocutter indicating that the instrument is being fired. The surgical hub may also receive measurement data from one of the HCP's sensing systems indicating higher stress level of the HCP (e.g., indicated by B1 mark on the time axis). For example, higher stress level may be indicated by change in the HCP's heart rate from a base value. The surgical hub 20076, like the prior step, may derive this inference by cross-referencing the receipt of data from the surgical stapling and cutting instrument with the retrieved steps in the process (e.g., as indicated by A2 and A3). The surgical hub 20076 may monitor the advance energy jaw trigger ratio and/or the endocutter clamp and firing speed during the high stress time periods. In an example, the surgical hub 20076 may send an assistance control signal to the advanced energy jaw device and/or the endocutter device to control the device in operation. The surgical hub may send the assistance signal based on the stress level of the HCP that is operating the surgical device and/or situational awareness known to the surgical hub. For example, the surgical hub 20076 may send control assistance signals to an advanced energy device or an endocutter clamp, as indicated in FIG. 8 by A2 and A3.

The HCP may proceed to the next step of freeing the upper sigmoid followed by freeing descending colon, rectum, and sigmoid. The surgical hub 20076 may continue to monitor the high stress markers of the HCP (e.g., as indicated by D1, E1a, E1b, F1). The surgical hub 20076 may send assistance signals to the advanced energy jaw device and/or the endocutter device during the high stress time periods, as illustrated in FIG. 8.

After mobilizing the colon, the HCP may proceed with the segmentectomy portion of the procedure. For example, the surgical hub 20076 may infer that the HCP is transecting the bowel and sigmoid removal based on data from the surgical stapling and cutting instrument, including data from its cartridge. The cartridge data can correspond to the size or type of staple being fired by the instrument, for example. As different types of staples are utilized for different types of tissues, the cartridge data can thus indicate the type of tissue being stapled and/or transected. It should be noted that surgeons regularly switch back and forth between surgical stapling/cutting instruments and surgical energy (e.g., RF or ultrasonic) instruments depending upon the step in the procedure because different instruments are better adapted for particular tasks. Therefore, the sequence in which the stapling/cutting instruments and surgical energy instruments are used can indicate what step of the procedure the surgeon is performing.

The surgical hub may determine and send a control signal to surgical device based on the stress level of the HCP. For example, during time period G1b, a control signal G2b may be sent to an endocutter clamp. Upon removal of the sigmoid, the incisions are closed, and the post-operative portion of the procedure may begin. The patient's anesthesia can be reversed. The surgical hub 20076 may infer that the patient is emerging from the anesthesia based on one or more sensing systems attached to the patient.

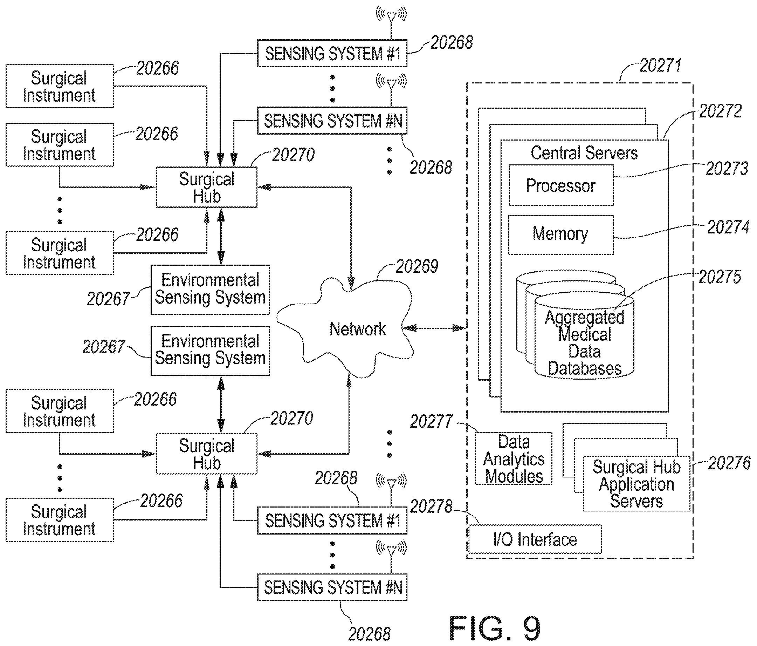

FIG. 9 shows an example computer-implemented interactive surgical system may be configured to monitor HCP biomarkers using one or more sensing systems 20001. The computer-implemented interactive surgical system may be configured to monitor HCP biomarkers using one or more sensing systems 20069. The HCP biomarkers and/or the patient biomarkers may be measured before, after, and/or during a surgical procedure. In one aspect, the computer-implemented interactive surgical system may be configured to monitor and analyze data related to the operation of various surgical systems 20069 that include surgical hubs, surgical instruments, robotic devices and operating theaters or healthcare facilities. The computer-implemented interactive surgical system may include a cloud-based analytics system. The computer-implemented interactive surgical system may include a local analytics system. The cloud-based analytics system may include one or more analytics servers.

A monitoring and analytics system may include a plurality of sensing systems 20268 (may be the same or similar to the sensing systems 20069), surgical instruments 20266 (may be the same or similar to instruments 20031), and/or a plurality of surgical hubs 20270 (may be the same or similar to hubs 20006). A surgical data network 20269 (may be the same or similar to the surgical data network described in FIG. 4) may couple the surgical hubs 20270 to the computing system 20271 (may be a local computing system, an edge computing system, or may be a cloud computing system such as cloud computing system 20064). The surgical hubs 20270 may be communicatively coupled to one or more surgical instruments 20266. The surgical hubs 20270 may also be communicatively coupled to the one or more sensing systems 20268, and the cloud 20271 of the computer-implemented interactive surgical system via the network 20269. The surgical hubs 20270 and the sensing systems 20268 may be communicatively coupled using wireless protocols as described herein. The computing system 20271 may be a local or remote centralized source of hardware and software for storing, processing, manipulating, and communicating measurement data from the sensing systems 20268 and data generated based on the operation of various surgical instruments 20266.

As shown in FIG. 9, access to the computing system 20271 may be achieved via the network 20269. The network 20269 may be the Internet or some other suitable computer network. Surgical hubs 20270 that may be coupled to the computing system 20271 can be considered the client side of the computing system (e.g., cloud-based analytics system). Surgical instruments 20266 may be paired with the surgical hubs 20270 for control and implementation of various surgical procedures and/or operations, as described herein. Sensing systems 20268 may be paired with surgical hubs 20270 for in-surgical HCP monitoring of surgeon related biomarkers, pre-surgical HCP monitoring, monitoring, or post-surgical HCP monitoring. Environmental sensing systems 20267 may be paired with surgical hubs 20270 measuring environmental attributes associated with an HCP.

Surgical instruments 20266, environmental sensing systems 20267, and sensing systems 20268 may comprise wired or wireless transceivers for data transmission to and from their corresponding surgical hubs 20270 (which may also comprise transceivers). Combinations of one or more of surgical instruments 20266, sensing systems 20268, or surgical hubs 20270 may indicate particular locations, such as operating theaters, intensive care unit (ICU) rooms, or recovery rooms in healthcare facilities (e.g., hospitals), for providing medical operations, pre-surgical preparation, and/or post-surgical recovery. For example, the memory of a surgical hub 20270 may store location data.

As shown in FIG. 9, the computing system 20271 may include one or more central servers 20272 (may be same or similar to remote server 20067), surgical hub application servers 20276, data analytics modules 20277, and an input/output ("I/O") interface 20278. The central servers 20272 of the computing system 20271 may collectively administer the cloud computing system, which includes monitoring requests by client surgical hubs 20270 and managing the processing capacity of the computing system 20271 for executing the requests. Each of the central servers 20272 may comprise one or more processors 20273 coupled to suitable memory devices 20274 which can include volatile memory such as random-access memory (RAM) and non-volatile memory such as magnetic storage devices. The memory devices 20274 may comprise machine executable instructions that when executed cause the processors 20273 to execute the data analytics modules 20277 for the cloud-based data analysis, real-time monitoring of measurement data received from the sensing systems 20268, operations, recommendations, and other operations as described herein. The processors 20273 can execute the data analytics modules 20277 independently or in conjunction with hub applications independently executed by the hubs 20270. The central servers 20272 also may include aggregated medical data databases 20275, which can reside in the memory 20274.

Based on connections to various surgical hubs 20270 via the network 20269, the computing system 20271 can aggregate data from specific data generated by various surgical instruments 20266, real-time data from sensing systems 20268, and/or the surgical hubs 20270. Such aggregated data may be stored within the aggregated medical databases 20275 associated with the computing system 20271. The computing system 20271 may track real-time measurement data from the sensing systems 20268 and/or perform data analysis and operations on the measurement data and/or the aggregated data to yield insights and/or perform functions that individual hubs 20270 could not achieve on their own.

As shown in FIG. 9, the computing system 20271 and the surgical hubs 20270 may be communicatively coupled to send and receive information. The I/O interface 20278 is connected to the plurality of surgical hubs 20270 via the network 20269. The I/O interface 20278 can be configured to transfer information between the surgical hubs 20270 and the aggregated medical data databases 20275. The I/O interface 20278 may facilitate read/write operations of the cloud-based analytics system. Such read/write operations may be executed in response to requests from hubs 20270. These requests could be transmitted to the surgical hubs 20270 through the hub applications. The I/O interface 20278 may include one or more high speed data ports, which may include universal serial bus (USB) ports, IEEE 1394 ports, as well as Wi-Fi and Bluetooth I/O interfaces for connecting the computing system 20271 to surgical hubs 20270. The hub application servers 20276 of the computing system 20271 may be configured to host and supply shared capabilities to software applications (e.g., hub applications) executed by surgical hubs 20270. For example, the hub application servers 20276 may manage requests made by the hub applications through the hubs 20270, control access to the aggregated medical data databases 20275, and perform load balancing.

The computing system may address various issues arising in the context of medical operations (e.g., pre-surgical monitoring, in-surgical monitoring, and post-surgical monitoring) and procedures performed using medical devices, such as the surgical instruments 20266, 20031. The surgical instruments 20266 may be digital surgical devices configured to interact with the computing system 20271 for implementing techniques to improve the performance of surgical operations. The computing system may address various issues arising in the context of monitoring one or more biomarkers associated with HCP(s) or a patient in pre-surgical, in-surgical, and post-surgical procedures using sensing systems 20268. Sensing systems 20268 may interact with the surgical hub 20270 and/or with the computing system 20271 for implementing techniques to monitor surgeon biomarkers and/or patient biomarkers. The sensing systems 20268 may include systems with one or more sensors that are configured to measure one or more biomarkers associated with an HCP participating in a medical operation and/or a patient on whom a medical operation is planned to be performed, is being performed or has been performed. Various surgical instruments 20266, sensing systems 20268, and/or surgical hubs 20270 may include human interface systems (e.g., having a touch-controlled user interfaces) such that HCPS and/or patients may control aspects of interaction between the surgical instruments 20266 or the sensing system 20268 and the computing system 20271. Other suitable user interfaces for control such as auditory controlled user interfaces may also be used.

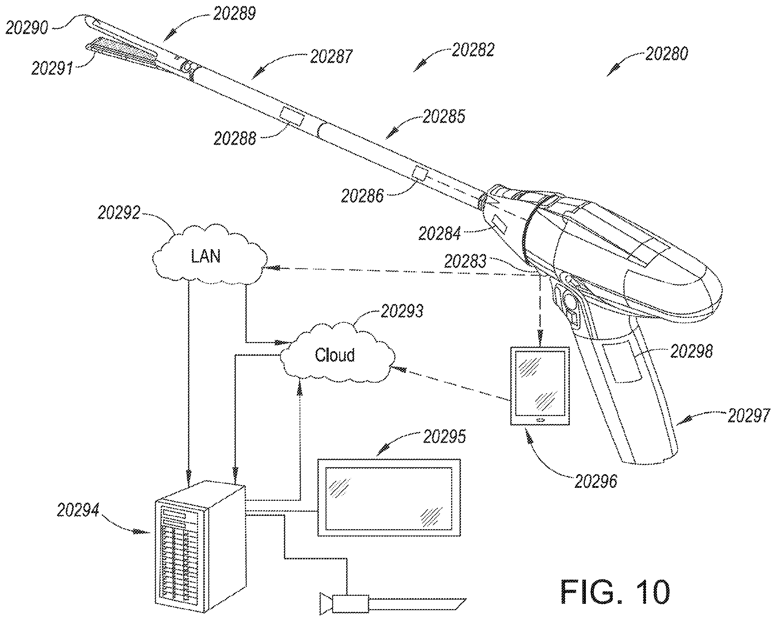

FIG. 10 illustrates an example surgical system 20280 in accordance with the present disclosure and may include a surgical instrument 20282 that can be in communication with a console 20294 or a portable device 20296 through a local area network 20292 and/or a cloud network 20293 via a wired and/or wireless connection. The console 20294 and the portable device 20296 may be any suitable computing device. The surgical instrument 20282 may include a handle 20297, an adapter 20285, and a loading unit 20287. The adapter 20285 releasably couples to the handle 20297 and the loading unit 20287 releasably couples to the adapter 20285 such that the adapter 20285 transmits a force from a drive shaft to the loading unit 20287. The adapter 20285 or the loading unit 20287 may include a force gauge (not explicitly shown) disposed therein to measure a force exerted on the loading unit 20287. The loading unit 20287 may include an end effector 20289 having a first jaw 20291 and a second jaw 20290. The loading unit 20287 may be an in-situ loaded or multi-firing loading unit (MFLU) that allows a clinician to fire a plurality of fasteners multiple times without requiring the loading unit 20287 to be removed from a surgical site to reload the loading unit 20287.

The first and second jaws 20291, 20290 may be configured to clamp tissue therebetween, fire fasteners through the clamped tissue, and sever the clamped tissue. The first jaw 20291 may be configured to fire at least one fastener a plurality of times or may be configured to include a replaceable multi-fire fastener cartridge including a plurality of fasteners (e.g., staples, clips, etc.) that may be fired more than one time prior to being replaced. The second jaw 20290 may include an anvil that deforms or otherwise secures the fasteners, as the fasteners are ejected from the multi-fire fastener cartridge.

The handle 20297 may include a motor that is coupled to the drive shaft to affect rotation of the drive shaft. The handle 20297 may include a control interface to selectively activate the motor. The control interface may include buttons, switches, levers, sliders, touchscreen, and any other suitable input mechanisms or user interfaces, which can be engaged by a clinician to activate the motor.

The control interface of the handle 20297 may be in communication with a controller 20298 of the handle 20297 to selectively activate the motor to affect rotation of the drive shafts. The controller 20298 may be disposed within the handle 20297 and may be configured to receive input from the control interface and adapter data from the adapter 20285 or loading unit data from the loading unit 20287. The controller 20298 may analyze the input from the control interface and the data received from the adapter 20285 and/or loading unit 20287 to selectively activate the motor. The handle 20297 may also include a display that is viewable by a clinician during use of the handle 20297. The display may be configured to display portions of the adapter or loading unit data before, during, or after firing of the instrument 20282.

The adapter 20285 may include an adapter identification device 20284 disposed therein and the loading unit 20287 may include a loading unit identification device 20288 disposed therein. The adapter identification device 20284 may be in communication with the controller 20298, and the loading unit identification device 20288 may be in communication with the controller 20298. It will be appreciated that the loading unit identification device 20288 may be in communication with the adapter identification device 20284, which relays or passes communication from the loading unit identification device 20288 to the controller 20298.

The adapter 20285 may also include a plurality of sensors 20286 (one shown) disposed thereabout to detect various conditions of the adapter 20285 or of the environment (e.g., if the adapter 20285 is connected to a loading unit, if the adapter 20285 is connected to a handle, if the drive shafts are rotating, the torque of the drive shafts, the strain of the drive shafts, the temperature within the adapter 20285, a number of firings of the adapter 20285, a peak force of the adapter 20285 during firing, a total amount of force applied to the adapter 20285, a peak retraction force of the adapter 20285, a number of pauses of the adapter 20285 during firing, etc.). The plurality of sensors 20286 may provide an input to the adapter identification device 20284 in the form of data signals. The data signals of the plurality of sensors 20286 may be stored within or be used to update the adapter data stored within the adapter identification device 20284. The data signals of the plurality of sensors 20286 may be analog or digital. The plurality of sensors 20286 may include a force gauge to measure a force exerted on the loading unit 20287 during firing.

The handle 20297 and the adapter 20285 can be configured to interconnect the adapter identification device 20284 and the loading unit identification device 20288 with the controller 20298 via an electrical interface. The electrical interface may be a direct electrical interface (i.e., include electrical contacts that engage one another to transmit energy and signals therebetween). Additionally, or alternatively, the electrical interface may be a non-contact electrical interface to wirelessly transmit energy and signals therebetween (e.g., inductively transfer). It is also contemplated that the adapter identification device 20284 and the controller 20298 may be in wireless communication with one another via a wireless connection separate from the electrical interface.

The handle 20297 may include a transceiver 20283 that is configured to transmit instrument data from the controller 20298 to other components of the system 20280 (e.g., the LAN 20292, the cloud 20293, the console 20294, or the portable device 20296). The controller 20298 may also transmit instrument data and/or measurement data associated with one or more sensors 20286 to a surgical hub 20270, as illustrated in FIG. 9. The transceiver 20283 may receive data (e.g., cartridge data, loading unit data, adapter data, or other notifications) from the surgical hub 20270. The transceiver 20283 may receive data (e.g., cartridge data, loading unit data, or adapter data) from the other components of the system 20280. For example, the controller 20298 may transmit instrument data including a serial number of an attached adapter (e.g., adapter 20285) attached to the handle 20297, a serial number of a loading unit (e.g., loading unit 20287) attached to the adapter 20285, and a serial number of a multi-fire fastener cartridge loaded into the loading unit to the console 20294. Thereafter, the console 20294 may transmit data (e.g., cartridge data, loading unit data, or adapter data) associated with the attached cartridge, loading unit, and adapter, respectively, back to the controller 20298. The controller 20298 can display messages on the local instrument display or transmit the message, via transceiver 20283, to the console 20294 or the portable device 20296 to display the message on the display 20295 or portable device screen, respectively.

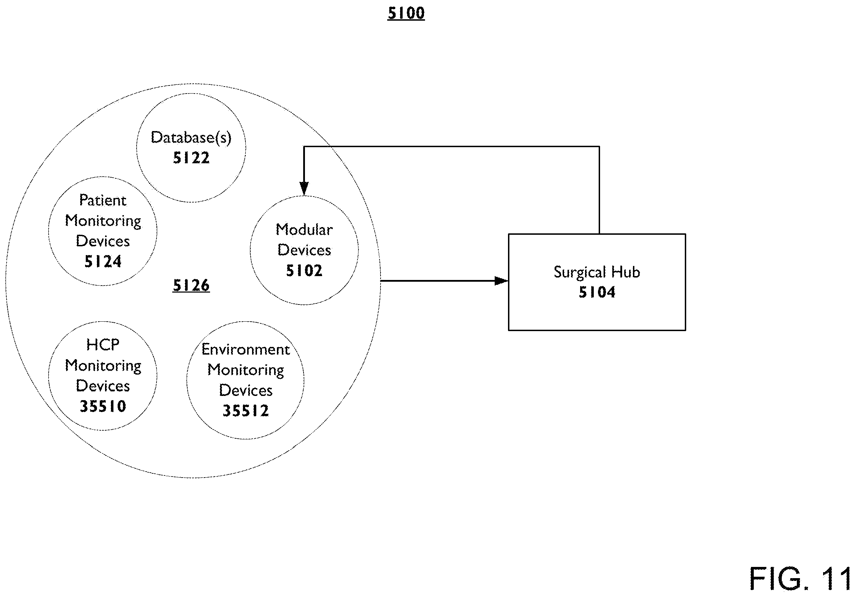

FIG. 11 illustrates a diagram of a situationally aware surgical system 5100, in accordance with at least one aspect of the present disclosure. The data sources 5126 may include, for example, the modular devices 5102 (which can include sensors configured to detect parameters associated with the patient, HCPs and environment and/or the modular device itself), databases 5122 (e.g., an EMR database containing patient records), and patient monitoring devices 5124 (e.g., a blood pressure (BP) monitor and an electrocardiography (EKG) monitor), HCP monitoring devices 35510, and/or environment monitoring devices 35512. The surgical hub 5104 can be configured to derive the contextual information pertaining to the surgical procedure from the data based upon, for example, the particular combination(s) of received data or the particular order in which the data is received from the data sources 5126. The contextual information inferred from the received data can include, for example, the type of surgical procedure being performed, the particular step of the surgical procedure that the surgeon is performing, the type of tissue being operated on, or the body cavity that is the subject of the procedure. This ability by some aspects of the surgical hub 5104 to derive or infer information related to the surgical procedure from received data can be referred to as "situational awareness." For example, the surgical hub 5104 can incorporate a situational awareness system, which is the hardware and/or programming associated with the surgical hub 5104 that derives contextual information pertaining to the surgical procedure from the received data.

The situational awareness system of the surgical hub 5104 can be configured to derive the contextual information from the data received from the data sources 5126 in a variety of different ways. For example, the situational awareness system can include a pattern recognition system, or machine learning system (e.g., an artificial neural network), that has been trained on training data to correlate various inputs (e.g., data from database(s) 5122, patient monitoring devices 5124, modular devices 5102, HCP monitoring devices 35510, and/or environment monitoring devices 35512) to corresponding contextual information regarding a surgical procedure. A machine learning system can be trained to accurately derive contextual information regarding a surgical procedure from the provided inputs. In examples, the situational awareness system can include a lookup table storing pre-characterized contextual information regarding a surgical procedure in association with one or more inputs (or ranges of inputs) corresponding to the contextual information. In response to a query with one or more inputs, the lookup table can return the corresponding contextual information for the situational awareness system for controlling the modular devices 5102. In examples, the contextual information received by the situational awareness system of the surgical hub 5104 can be associated with a particular control adjustment or set of control adjustments for one or more modular devices 5102. In examples, the situational awareness system can include a further machine learning system, lookup table, or other such system, which generates or retrieves one or more control adjustments for one or more modular devices 5102 when provided the contextual information as input.

A surgical hub 5104 incorporating a situational awareness system can provide a number of benefits for the surgical system 5100. One benefit may include improving the interpretation of sensed and collected data, which would in turn improve the processing accuracy and/or the usage of the data during the course of a surgical procedure. To return to a previous example, a situationally aware surgical hub 5104 could determine what type of tissue was being operated on; therefore, when an unexpectedly high force to close the surgical instrument's end effector is detected, the situationally aware surgical hub 5104 could correctly ramp up or ramp down the motor of the surgical instrument for the type of tissue.

The type of tissue being operated can affect the adjustments that are made to the compression rate and load thresholds of a surgical stapling and cutting instrument for a particular tissue gap measurement. A situationally aware surgical hub 5104 could infer whether a surgical procedure being performed is a thoracic or an abdominal procedure, allowing the surgical hub 5104 to determine whether the tissue clamped by an end effector of the surgical stapling and cutting instrument is lung (for a thoracic procedure) or stomach (for an abdominal procedure) tissue. The surgical hub 5104 could then adjust the compression rate and load thresholds of the surgical stapling and cutting instrument appropriately for the type of tissue.

The type of body cavity being operated in during an insufflation procedure can affect the function of a smoke evacuator. A situationally aware surgical hub 5104 could determine whether the surgical site is under pressure (by determining that the surgical procedure is utilizing insufflation) and determine the procedure type. As a procedure type can be generally performed in a specific body cavity, the surgical hub 5104 could then control the motor rate of the smoke evacuator appropriately for the body cavity being operated in. Thus, a situationally aware surgical hub 5104 could provide a consistent amount of smoke evacuation for both thoracic and abdominal procedures.

The type of procedure being performed can affect the optimal energy level for an ultrasonic surgical instrument or radio frequency (RF) electrosurgical instrument to operate at. Arthroscopic procedures, for example, may require higher energy levels because the end effector of the ultrasonic surgical instrument or RF electrosurgical instrument is immersed in fluid. A situationally aware surgical hub 5104 could determine whether the surgical procedure is an arthroscopic procedure. The surgical hub 5104 could then adjust the RF power level or the ultrasonic amplitude of the generator (e.g., "energy level") to compensate for the fluid filled environment. Relatedly, the type of tissue being operated on can affect the optimal energy level for an ultrasonic surgical instrument or RF electrosurgical instrument to operate at. A situationally aware surgical hub 5104 could determine what type of surgical procedure is being performed and then customize the energy level for the ultrasonic surgical instrument or RF electrosurgical instrument, respectively, according to the expected tissue profile for the surgical procedure. Furthermore, a situationally aware surgical hub 5104 can be configured to adjust the energy level for the ultrasonic surgical instrument or RF electrosurgical instrument throughout the course of a surgical procedure, rather than just on a procedure-by-procedure basis. A situationally aware surgical hub 5104 could determine what step of the surgical procedure is being performed or will subsequently be performed and then update the control algorithms for the generator and/or ultrasonic surgical instrument or RF electrosurgical instrument to set the energy level at a value appropriate for the expected tissue type according to the surgical procedure step.

In examples, data can be drawn from additional data sources 5126 to improve the conclusions that the surgical hub 5104 draws from one data source 5126. A situationally aware surgical hub 5104 could augment data that it receives from the modular devices 5102 with contextual information that it has built up regarding the surgical procedure from other data sources 5126. For example, a situationally aware surgical hub 5104 can be configured to determine whether hemostasis has occurred (e.g., whether bleeding at a surgical site has stopped) according to video or image data received from a medical imaging device. The surgical hub 5104 can be further configured to compare a physiologic measurement (e.g., blood pressure sensed by a BP monitor communicably connected to the surgical hub 5104) with the visual or image data of hemostasis (e.g., from a medical imaging device communicably coupled to the surgical hub 5104) to make a determination on the integrity of the staple line or tissue weld. The situational awareness system of the surgical hub 5104 can consider the physiological measurement data to provide additional context in analyzing the visualization data. The additional context can be useful when the visualization data may be inconclusive or incomplete on its own.

For example, a situationally aware surgical hub 5104 could proactively activate the generator to which an RF electrosurgical instrument is connected if it determines that a subsequent step of the procedure requires the use of the instrument. Proactively activating the energy source can allow the instrument to be ready for use a soon as the preceding step of the procedure is completed.

The situationally aware surgical hub 5104 could determine whether the current or subsequent step of the surgical procedure requires a different view or degree of magnification on the display according to the feature(s) at the surgical site that the surgeon is expected to need to view. The surgical hub 5104 could proactively change the displayed view (supplied by, e.g., a medical imaging device for the visualization system 108) accordingly so that the display automatically adjusts throughout the surgical procedure.

The situationally aware surgical hub 5104 could determine which step of the surgical procedure is being performed or will subsequently be performed and whether particular data or comparisons between data will be required for that step of the surgical procedure. The surgical hub 5104 can be configured to automatically call up data screens based upon the step of the surgical procedure being performed, without waiting for the surgeon to ask for the particular information.

Errors may be checked during the setup of the surgical procedure or during the course of the surgical procedure. For example, the situationally aware surgical hub 5104 could determine whether the operating theater is setup properly or optimally for the surgical procedure to be performed. The surgical hub 5104 can be configured to determine the type of surgical procedure being performed, retrieve the corresponding checklists, product location, or setup needs (e.g., from a memory), and then compare the current operating theater layout to the standard layout for the type of surgical procedure that the surgical hub 5104 determines is being performed. In some exemplifications, the surgical hub 5104 can compare the list of items for the procedure and/or a list of devices paired with the surgical hub 5104 to a recommended or anticipated manifest of items and/or devices for the given surgical procedure. If there are any discontinuities between the lists, the surgical hub 5104 can provide an alert indicating that a particular modular device 5102, patient monitoring device 5124, HCP monitoring devices 35510, environment monitoring devices 35512, and/or other surgical item is missing. In some examples, the surgical hub 5104 can determine the relative distance or position of the modular devices 5102 and patient monitoring devices 5124 via proximity sensors, for example. The surgical hub 5104 can compare the relative positions of the devices to a recommended or anticipated layout for the particular surgical procedure. If there are any discontinuities between the layouts, the surgical hub 5104 can be configured to provide an alert indicating that the current layout for the surgical procedure deviates from the recommended layout.

The situationally aware surgical hub 5104 could determine whether the surgeon (or other HCP(s)) was making an error or otherwise deviating from the expected course of action during the course of a surgical procedure. For example, the surgical hub 5104 can be configured to determine the type of surgical procedure being performed, retrieve the corresponding list of steps or order of equipment usage (e.g., from a memory), and then compare the steps being performed or the equipment being used during the course of the surgical procedure to the expected steps or equipment for the type of surgical procedure that the surgical hub 5104 determined is being performed. The surgical hub 5104 can provide an alert indicating that an unexpected action is being performed or an unexpected device is being utilized at the particular step in the surgical procedure.

The surgical instruments (and other modular devices 5102) may be adjusted for the particular context of each surgical procedure (such as adjusting to different tissue types) and validating actions during a surgical procedure. Next steps, data, and display adjustments may be provided to surgical instruments (and other modular devices 5102) in the surgical theater according to the specific context of the procedure.

Object detection can be performed via computer vision and image processing that deals with detecting instances of semantic objects of a certain class (such as humans, equipment, or objects) in digital images and videos. Object detection is used in computer vision tasks such as image annotation, activity recognition, face detection, face recognition, video object co-segmentation. Object detection is used in tracking objects, for example tracking a ball during a football match, tracking movement of a cricket bat, or tracking a person in a video. Object detection may be performed via neural network-based approach(es), and/or non-neural approaches. For example, features may be defined and classification may be performed based on the defined features, for example, via support vector machine (SVM). Neural techniques may perform object detection without specifically defining features, and may be based on convolutional neural networks (CNN).

Moving object detection may be performed to recognize the physical movement of a person or an object in a given place or region. By acting segmentation among moving objects and stationary area or region, the moving objects motion could be tracked and thus could be analyzed later. Moving object detection may be performed via background subtraction, frame differencing, temporal differencing, and or optical flow analysis.

Smart and self-identifying RF systems may be used to locate HCPs and equipment in the OR. HCPs, instruments, equipment and/or boundaries may be located using spatial identifying sensors. The locations of HCPs, equipment and/or boundaries may be tracked with respect to the room and/or with respect to the patient, via wireless sensors and beacons.

A wearable monitoring system may be coupled to a person, such as an HCP or a patient. Ultra-wide band monitoring may be performed to identify the location of the person within the OR, and/or the person's relative position to an equipment, a surgical instrument, another person, a boundary, and/or the like.

RF element identification and boundary monitoring may be performed. RF beacons may be placed on equipment or instruments. BLE beacons may be placed in physical OR's. For example, beacons may be placed in corners of the OR, outlining the boundaries of the room. BLE beacons may be place on structures, such as large structures within the OR. BLE beacons may be placed on device for monitoring the device location relative to one or more boundaries.

For example, RFID tags may be affixed onto instruments and/or HCPs. Electromagnetic gates may be placed in the OR to prevent or to minimize violation of boundaries.

Boundary violations may be detected by tracking beacon movements. For example, the surgical hub may detect a boundary violation upon determining that the beacon has moved across a boundary. If the boundary violation is associated with a device crossing a boundary, the surgical hub may deactivate the device and alert an HCP of change of status. If the boundary violation is a larger structure, an urgent message can be displayed to indicate the equipment is of concern.

RF beacons may be used to locate HCPS in the OR theater. The location of an HCP may be tracked via their identification badge. The identification badge may have an imbedded RF beacon. The RF beacon may be high frequency (HF) or ultra-high frequency (UHF). The read range may not be affected by fluids or the need to penetrate structures. Since the size of the OR's can vary, the HF or UHF can provide sufficient coverage to accommodate potential distance from the surgical hub.

A surgical instrument may be associated with a unique RFID identification. The location of the surgical instrument may be tracked. The current use and/or the previous history of the surgical instrument may be determined based on location tracking. An external reader from the surgical hub may be positioned around the patient. The external reader may be used to track surgical instruments that enter the OR field. A low frequency or an HF chip may be used on instruments. The low or high frequencies may enable the reader to penetrate body fluids.

Instrument use can be determined and tracked based on its location. For example, the number of times a surgical instrument has been used, the length of time a device was in the patient, and/or a level of security that the device had been through a sterilization cycle prior to being introduced into the OR may be determined via location tracking. Whether an instrument has been left in the patient may be detected via location tracking of the instrument.

OR room imaging may be performed by multiple sources via various technologies to determine HCP, instrument, and/or equipment locations and their aspects. More than one imaging device can be used through different energy types to locate objects and people within the OR, the walls of the OR, and/or their proximity. The location and/or proximity information may be used to identify and/or control their interactions. Instruments and/or equipment may be configured based on the identified interactions.

For example, ultrasonic echo location may be used to determine object motion and measurements of location. OR visual or multi-spectral imaging may be used to monitor user location, interaction, and communication. Audio monitoring may be used to determine and record noise generating monitoring and tracking.

Infrared (IR) thermographic monitoring may be used to monitor the core temperate and the changes of temperature of HCPs and patient to adjust systems, monitor for infection, and/or understand situational awareness. As an HCP enters into the OR, an IR signature may be created for the HCP. An initial IR association response may be logged, for example, via the surgical hub. During a procedure, if the stress levels of the HCP increases, the IR signature may display or register the increased stress levels.

The various imaging systems may be used in concert. For example, the surgical hub may be positioned in the OR. Once powered up, the surgical hub may use an utlrasonic echo to determine the OR boundaries (e.g., walls) and large equipment locations to map room. A grid map may be established with all required boundaries.

When an HCP enters into OR theater, the HCP may be identified as entering via a BLE beacon associated with HCP. The surgical hub may check the HCP into theater. The surgical hub may begin tracking the HCP via an optical camera. The IR camera may perform the initial scan of the identified HCP for base line stress level(s). Once procedure begins, the audio monitoring system may monitor and record sounds. The surgical hub may monitor for voice inflections and/or raised nervous voices. The audio monitoring system can be synchronized with the IR camera, for example, for verifying an increase in stress levels. This process may be performed for multiple HCPs.

The HCPs in the OR may be recognized and tracked via an ID badge having an RFID, NFC, and/or a wearable device. For example, as an HCP move in and out of a room, as they come close to another wearable device, as they come close to a surgical hub, the identity of the HCP may be determined. The surgical hub may request HCP identifying information from an RFID reader, NFC reader, or a wearable device, upon detecting an HCP entering the OR. The surgical hub may confirm the identity of an HCP based on other source(s) of information. The surgical hub may assume that the identity of the HCP associated with an ID badge or a wearable device until the ID badge or wearable device is off. Details related to identifying an HCP and/or a user role via a wearable device may be found in U.S. Application No. 17/156, 324, entitled ACTIVE RECOGNITION AND PAIRING SENSING SYSTEMS.

The HCPs in the OR may be recognized and tracked via video processing from video captured by one or more cameras in the OR. Various known image or video processing technologies, such as keypoint detection and analysis, bounding box annotation, polygon mesh processing, image segmentation, facial recognition, gesture recognition, point cloud, lines and splines, and/or the like may be used to analyze the video feeds.

The location, movement, and/or orientation of various surgical products and instruments in the operating room may be identified and tracked. For example, a gyroscope or 3 axis accelerometers may be used to determine device orientation and position.

For example, a surgical instrument may be identified via one or more spatial registration markers located thereon. For example, visible fiducial markers could be placed on the instrument. The surgical hub may monitor the location, movement, and/or orientation of the surgical instrument via one or more camera in the OR. The fiducial marks may be in a predefined pattern. The surgical hub may associate a surgical instrument with particular fiducial mark(s) and may use the mark(s) to identify and model the instrument with the 3D computer environment it creates and records. The registration allows for the compensation for translation, rotation, scale, skew, and perspective. This may enable the surgical hub to detect and monitor the instruments even once a portion of the instrument is obscured.

For visible monitoring, camera calibration may be conducted when the system starts up. A predefined set of calibration markers within the hub camera view that may be fixed, such that the surgical hub may calibrate the camera for distance and focal length. The surgical hub may determine the exact length from itself to another calibration preset scale in the OR, and may use the measurement and the scale to calibrate the camera and focal distance.

For example, the surgical hub may determine the exact distance via a measurement system. For example, measurement system(s) that employ laser Doppler, ultrasonic pinging, RF and/or other energy digital communication may be perform distance measurement and send the measurements to the surgical hub. The measurement system may be included in the surgical hub.

Distance may be inferred from active and/or passive electronic signal processing. For example, by monitoring the signal strength and compensating for emission power, emitting device antenna path, fight path, receiving device antenna path, and/or receiver sensitivity the communication between two paired systems or devices, the distance between the two systems or devices may be determined. For example, UHF or HF RFID tagged object may be tracked through a combination of predefined tag and distances in combination with unknown tags. The tags may be used to identify a product, device or instrument, and may allow the product, device or instrument tracked within the OR once identified. The tags may provide further information about the identified product, device or instrument. A RFID map may be generated from passive or active references tags with known locations (e.g., landmarks) to locate any unknown tag detected by the RFID reader antennas. The distances between the readers and the common detected tags may be measured using a large-scale path loss propagation model. The distance between the unknown tag and the detected landmarks (e.g., inter-tags distance) may be calculated.

A millimeter-wave radar may be used to track objects. Millimeter-wave radar may achieve an accuracy of around a few micrometers. With the radar operating using frequency-modulated continuous waves (FMCW), frequency and/and phase of radar beat signal may be used to determine the distance between the radar sensor and the object from which the radar signal is reflected.

A mapping or evaluation of the bounds of the operating room may be performed. For example, the surgical hub 20006 may maintain spatial awareness during operation by periodically mapping its operating room, which can be helpful in determining if the surgical hub 20006 has been moved. The reevaluation can be performed periodically or it can be triggered by an event such as observing a change in the devices of the HCP monitoring system 20002 that are deemed within the operating room. The change may be the detection of a new device that was not previously deemed as within the bounds of the operating room. The change may be a disappearance, disconnection, or un-pairing of a paired device that was previously deemed as residing within the operating room. The surgical hub 20006 may continuously monitor the connection with paired devices to detect the disappearance, disconnection, or un-pairing of a paired device.

An operating-room mapping module may contain a compass and integrated Bluetooth transceiver. Other communication mechanisms, which are not significantly affected by the hospital environment or geographical location, can be employed. Bluetooth Low Energy (BLE) beacon technology may achieve indoor distance measurements with accuracy of about 1-2 meters, with improved accuracy in closer proximities (within 0-6 meters). To improve the accuracy of the distance measurements, a compass is used with the BLE. The operating-room mapping module may the BLE and/or the compass to determine where modules are located in relation to the patient. For example, two modules facing each other (detected by compass) with greater than one meter distance between them may clearly indicate that the modules are on opposite sides of the patient. The more "hub"-enabled modules that reside in the operating room, the greater the achievable accuracy becomes due to triangulation techniques. The operating-room mapping module may be included in the surgical hub 20006 as described herein. The operating-room mapping module may be in operative communication with the surgical hub 20006 as described herein.

The operating-room mapping module may map the physical location of device(s) and/or surgical modules that resides within the operating room. This information could be used by the user interface to display a virtual map of the room, enabling the user to more easily identify which modules are present and enabled, as well as their current status. The mapping data collected by surgical hub 20006 may be analyzed for identifying how an operating room is physically setup, for example.

For example, the surgical hub 20006 may determine a device's location by assessing transmission radio signal strength and direction. For Bluetooth protocols, the Received Signal Strength Indication (RSSI) is a measurement of the received radio signal strength. In one aspect, the devices of the HCP monitoring system 20002 can be equipped with USB Bluetooth dongles. The surgical hub 20006 may scan the USB Bluetooth beacons to get distance information. For example, multiple high-gain antennas on a Bluetooth access point with variable attenuators can produce more accurate results than RSSI measurements. The hub surgical hub 20006 may determine the location of a device by measuring the signal strength from multiple antennas.

The surgical hub 20006 can identify components of the HCP monitoring system 20002 as they are brought into an operating room. For example, the devices of the HCP monitoring system 20002 can be equipped with an identifier recognizable by the surgical hub 20006, such as, for example, a bar code or an RFID tag. NFC can also be employed. The surgical hub 20006 can be equipped with a suitable reader or scanner for detecting the devices brought into the operating room. [Details related to Spatial awareness of surgical hubs in operating rooms, can be found in U.S. Patent Application Serial No. 15/940,666, titled SPATIAL AWARENESS OF SURGICAL HUBS IN OPERATING ROOMS.

The computing system may be or may include an HCP monitoring system such as the HCP monitoring system 20000, 20002, 20003, or 20004 as described herein with respect to FIGs. 1-3. The computing system may be a computing system operatively connected to the HCP monitoring system(s) 20000, 20002, 20003, and/or 20004. The computing system may be or may include the computing system 20271 described herein with respect to FIG. 9. The computing system may be or may include the computer system 20063 described herein, for example, with respect to FIG. 4. The computing system may be or may include the computer system 20064 described herein, for example, with respect to FIG. 4. The computing system may be or may include the surgical hub 20006 as described herein with respect to FIGs. 1-3, surgical hub system 20060 in FIG. 4, the computer-implemented interactive surgical system 20070, in FIG. 5, the surgical hub or computing device 20243 in FIG. 7, the surgical hub 20270 in FIG. 9, the console 20294 in FIG. 10, and/or the surgical hub 5104 in FIG. 11. For example, the computing system may obtain surgical monitoring data associated with one or more surgical procedures. The surgical procedures may take place in an OR or multiple ORs.

The surgical monitoring data may be obtained via the surgical hubs. For example, a surgical hub may obtain surgical monitoring data from various sensing systems such as the wearable sensing system(s) 20011, and/or environmental sensing system(s) 20015 described herein with respect to FIG. 1. The surgical hub may obtain surgical monitoring data from HCP monitoring devices 35510, environmental monitoring devices 35512, patient monitoring devices 5124, and/or modular devices 5102 as described herein with respect to FIG. 11. The surgical monitoring data may include situational awareness data described herein with reference to FIG. 11.

The computing system may obtain the surgical monitoring data from various sensing systems such as the wearable sensing system(s) 20011, and/or environmental sensing system(s) 20015 described herein with respect to FIG. 1. The surgical monitoring data may be obtained from HCP monitoring devices 35510, environmental monitoring devices 35512, patient monitoring devices 5124, and/or modular devices 5102 as described herein with respect to FIG. 11.

A computing system may determine a virtual boundary associated with a restricted access in an OR. The virtual boundary described herein may be or may include an area associated with a restricted access in the OR. For example, the area associated with a restricted access in the OR may be an enclosed area, such as a circle, an oval, a rectangle, a square, and/or the like.

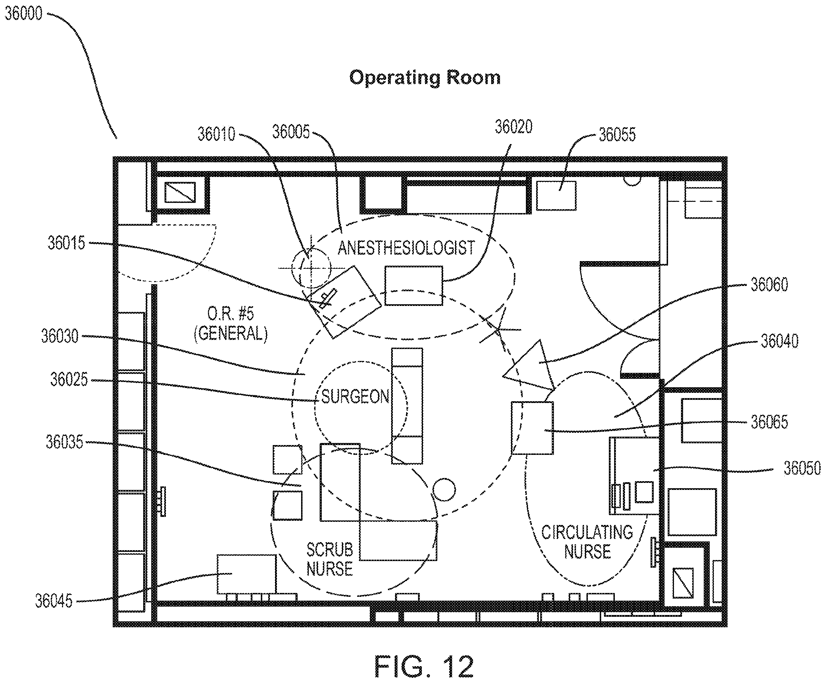

FIG. 12 illustrates examples of one or more virtual boundaries associated with restricted accesses in an OR. As illustrated in FIG. 12, an OR 36000 may include one or more virtual boundaries, and each of the virtual boundaries may be associated with corresponding restricted accesses.

A virtual boundary may be associated with a restricted access area for an anesthesiologist in an OR. The restricted access area may be referred to as virtual boundary area herein. For example, a virtual boundary area for an anesthesiologist 36005 may be or may include a restricted access area for an anesthesiologist, e.g., as illustrated in FIG. 12. The virtual boundary area for an anesthesiologist 36005 may limit access to health care professionals (HCPs) in an OR and may allow access to an anesthesiologist, an anesthesiologist staff, and/or an anesthesiologist equipment. For example, an anesthesiologist may be allowed to enter, exit, and/or be stationed in the virtual boundary area for an anesthesiologist 36005, e.g., during a surgical procedure. As described herein, a computing system may determine a virtual boundary associated with a restricted access area for an anesthesiologist 36005. The computing system may monitor movement of HCPs in an OR. If the computing system determines that an HCP, other than an anesthesiologist and/or an anesthesiologist staff, is in a proximity to the virtual boundary area for an anesthesiologist 36005 and/or attempts to enter or interact with the virtual boundary area for an anesthesiologist 36005, the computing system may send a notification to the one or more HCPs. The notification may indicate that the HCP does not have an access authorization to enter or interact with the virtual boundary area for an anesthesiologist 36005.

A virtual boundary area for an anesthesiologist 36005 may be or may include one or more subsets of virtual boundary areas relating to an anesthesiologist. For example, a virtual boundary area 36010 may be associated with an area where an anesthesiologist is stationed during a surgical procedure. A virtual boundary area 36015 may be a virtual boundary area for an anesthesiologist staff, such as an anesthesiologist nurse, and/or an anesthesiologist equipment, such as a display for an anesthesiologist. A virtual boundary area 36020 may be a virtual boundary area where an anesthesiologist instrument for an anesthesiologist may be stationed.

A virtual boundary area may be associated with a restricted access area for a surgeon in an OR. For example, a virtual boundary area associated with a restricted access area for a surgeon 36025 may be illustrated in FIG. 12. The virtual boundary for a surgeon 36025 may be a restricted access area for a surgeon in an OR to enter, exit, and/or be within during a surgical operation. The virtual boundary for a surgeon 36025 may be or may include a restricted access area where a surgeon operates during a surgical step of an operation. The virtual boundary area for a surgeon 36025 may limit access to a surgeon who is/will be operating in a current surgical step. For example, a computing system may determine a virtual boundary area for a surgeon 36025 in an OR 36000. The computing system may monitor movements for HCPs in the OR. For a current surgical step, the computing system may identify a surgeon, such as a lead surgeon, who is going to perform the current surgical step. The computing system may allow the lead surgeon to access the virtual boundary area for a surgeon 36025 (e.g., to enter, exit, or be stationed). If the computing system determines that an HCP other than the identified surgeon is in a proximity to the virtual boundary area for a surgeon 36025 or about to enter/interact with the virtual boundary area, the computing system may send a notification to the HCP alerting that the HCP does not have an access authorization to enter or interact with the virtual boundary area for a surgeon 36025 and limit the access.

As described herein, the virtual boundary area for a surgeon 36025 may be or may include one or more subset of restricted access area (not illustrated). For example, a subset of the virtual boundary area for a surgeon 36025 may include a virtual boundary area for a surgeon who is performing a current surgical step of an operation. A subset of the virtual boundary area for a surgeon 36025 may include a virtual area for an assistant surgeon for a surgical procedure. A subset of the virtual boundary area for a surgeon 36025 may include a virtual boundary area for an observer, such as an intern. The computing system may allow the identified HCP, such as a surgeon, an assistant surgeon, and/or an observer, to access the corresponding virtual boundary area and limit the access to other HCPs.

A virtual boundary may be associated with a restricted access area for a surgical table in an OR. For example, a virtual boundary associated with a restricted access area for a surgical table 36030 may be illustrated in FIG. 12. The virtual boundary area for a surgical table 36030 may be a restricted access area that allows a surgeon, such as the surgeon who will be performing a current step of a surgical procedure and/or a patient. For example, the virtual boundary area for a surgical table 36030 may be a sterilized area where a patient is positioned for a surgical operation. The virtual boundary area for the surgical table 36030 may be sterilized. The computing system may limit access to the virtual boundary area for a surgical table 36030. For example, the computing system may allow a patient to enter and be positioned in the virtual boundary area for a surgical table 36030. The computing system may control an access to the virtual boundary area for a surgical table 36030 and allow an HCP who has an access authorization to the virtual boundary area for a surgical table 36030 to enter, exit, and/or be stationed. As described herein, the computing system may send a notification to an unauthorized HCP to enter or interact with the virtual boundary area for a surgical table 36030 and block the access.

A virtual boundary may be associated with a restricted access area for a scrub nurse. For example, a virtual boundary associated with a restricted access area for a scrub nurse 36035 may be illustrated in FIG. 12. As described herein, the virtual boundary area for a scrub nurse 36035 may be a restricted access area that allows a scrub nurse to enter, exit, and/or be stationed, e.g., during a surgical procedure. The computing system may allow a scrub nurse, who has an access authorization, to interact with or be in the virtual boundary area for a scrub nurse 36035. The computing system may block an unauthorized HCP to access or interact with the virtual boundary area for a scrub nurse 36035 and send a notification to the unauthorized HCPs.

A virtual boundary may be associated with a restricted access area for a circulating nurse. For example, a virtual boundary associated with a restricted access area for a circulating nurse 36040 may be illustrated in FIG. 12. As described herein, the virtual boundary area for a circulating nurse 36040 may be a restricted access area that allows a circulating nurse to enter, exit, and/or stationed, e.g., during a surgical procedure. The computing system may allow a circulating nurse who has an access authorization to interact with or be in the virtual boundary area for a circulating nurse 36040. The computing system may send a notification to an unauthorized HCP and may block the access of the unauthorized HCP accessing or interacting with the virtual boundary area for a circulating nurse 36040.

A virtual boundary area may be associated with one or more restricted access areas for a surgical instrument, sterile tray table, and/or other surgery related equipment. For example, a virtual boundary associated with a restricted access area for a surgical instrument, a sterile tray table, and/or other surgery related equipment 36045, 36050, 36055, 36060, 36065 may be illustrated in FIG. 12. Examples of surgery related equipment may include one or more of: a filtration system, a particle capture system, and/or an airborne contamination zone. As described herein, the computing system may allow an HCP with an access authorization to allow entering, exiting, interacting with, and/or be stationed in the virtual boundary area for the surgical equipment. For example, a scrub nurse who has an access authorization to enter a virtual boundary area for a sterile tray table 36045 may enter/exit the virtual boundary area for a sterile tray table 36045 during a surgical operation. The computing system may block other HCPs who do not have the access authorization to interact with (e.g., enter) the virtual boundary area for a sterile tray table 36045 from interacting with the virtual boundary area for a sterile tray table 36045. The computing system may send a notification to the unauthorized HCPs indicating that the HCPs do not have access authorizations to enter the virtual boundary area for a sterile tray table 36045.

As illustrated in FIG. 12, one or more virtual boundary areas may overlap. For example, a virtual boundary area for a surgeon 36025 may be inside of a virtual boundary area for a surgical table 36030. In examples, during a surgical operation, a scrub nurse may be assisting a surgeon by hand over a surgical instrument to the surgeon. A virtual boundary area for a scrub nurse 36035 may overlap with a virtual boundary area for a surgeon 36025 and/or a virtual boundary area for a surgical table 36030.

A virtual boundary associated with a surgical instrument and/or a virtual boundary associated with a patient may change. For example, a virtual boundary associated with a restricted access area for a surgical instrument may change based on the surgical instrument being in an off position or being in an on position (e.g., while the surgical instrument is in use during a surgical procedure). A virtual boundary area associated with a patient may change during a surgical procedure as the body of the patient is moved, turned, repositioned, or etc.

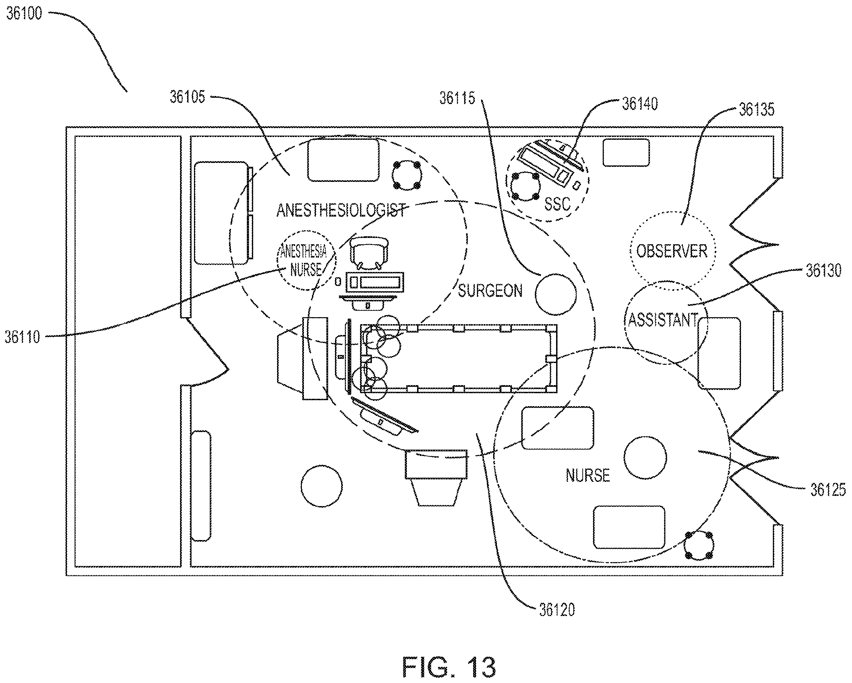

FIG. 13 illustrates other examples of one or more virtual boundaries associated with restricted accesses in an OR 36100. As described herein, a virtual boundary may be associated with a restricted access area for an anesthesiologist 36105. A virtual boundary area for an anesthesiologist 36105 may include a virtual boundary area for an anesthesiologist nurse 36110. As an anesthesiologist nurse assists an anesthesiologist during a surgical procedure by handing over an anesthesiologist equipment, the anesthesiologist nurse may have access to enter, exit, and/or be stationed inside of the virtual boundary area for an anesthesiologist 36105.

As described herein, a virtual boundary may be associated with a restricted access area for a surgical table and/or an operation area 36120. A virtual boundary area for a surgical table and/or an operation area 36120 may include a virtual boundary area for a surgeon 36115. As a surgeon needs to operate a patient who is on a surgical table, the surgeon may be authorized to interact with (e.g., enter, exit, be stationed inside of) the virtual boundary area for a surgical table and/or an operation area 36120.

As described herein, a virtual boundary may be associated with a restricted access area for a nurse 36125. A nurse may assist a surgeon during a surgical procedure. For example, a nurse may hand a surgical instrument to a surgeon. The nurse may be authorized to interact with or enter, exit, and/or be stationed in the virtual boundary area for a surgeon 36115 and/or the virtual boundary area for a surgical table and/or an operation area 36120.

A virtual boundary may be associated with a restricted access area for an assistant 36130. An assistant may answer a device associated with one or more HCPs in an OR. For example, an assistant may answer a cellphone and/or a pager for a surgeon. As a role for an assistant may be non-surgical related, an access authorization associated with an assistant may be at a minimum level (e.g., a default level or a basic level). For example, an assistant may not have an access authorization to interact with, enter, exit, be stationed in other virtual boundaries in an OR. In addition to and/or alternatively, a virtual boundary area for an assistant 36130 may be used as a boundary or a barrier for a computing device to monitor movement of an assistant. For example, in case an assistant exits and/or leaves a virtual boundary area for an assistant 36130, the computing system may send a notification to the assistant. The notification may indicate that the assistant may be outside of the virtual boundary area designated for an assistant 36130 and may potentially contaminate other virtual boundary areas with sterilized fields.

As described herein, a virtual boundary may be associated with a restricted access area for an observer 36135. An observer may be stationed close to (e.g., next to or in proximity to) a surgical table to observe a surgical procedure. An access authorization associated with an observer may be at a minimum level (e.g., a default level or a basic level). For example, a role for an observer is to observe a surgical procedure and not to interfere with the surgical procedure. An observer may not have an access authorization to interact with, enter, exit, be stationed in other virtual boundaries in an OR. In addition to and/or alternatively, a virtual boundary for an observer 36135 may be used as a boundary or a barrier for a computing device to monitor movement of an observer. For example, if an observer exists and/or leaves the virtual boundary area for an observer 36135, the computing system may send a notification to the observer. The notification may indicate that the observer may be outside of the virtual boundary area designated for an observer 36135 and may contaminate other virtual boundary areas with sterilized fields.

As illustrated in FIG. 13, a virtual boundary may be associated with a restricted access area for a surgical safety checklist (SSC) 36140. In examples, a computing system may determine whether an HCP is wearing personal protection equipment (PPE). If the computing system determines that the HCP is not wearing proper PPE for a surgical operation, the computing system may send a notification to the HCP. The notification may indicate that the HCP is in proximity to the virtual boundary area for an SSC 36140 and the HCP and may not be wearing PPE. In examples, the computing system may allow an HCP to enter the virtual boundary area for an SSC 36140. The computing system may determine whether the HCP is wearing PPE suitable for a surgical procedure (e.g., when the HCP is in the virtual boundary area for an SSC 36140). If the computing system determines that the HCP is in proximity to the virtual boundary area for an SSC 36140 and is about to exit and/or leave the virtual boundary area for an SSC 36140, the computing system may send a notification to the HCP. The notification ma indicate to the HCP that the HCP is not wearing proper PPE suitable for the surgical procedure and may be exposed to danger if the HCP exits and/or leaves the virtual boundary area for an SSC 36140.

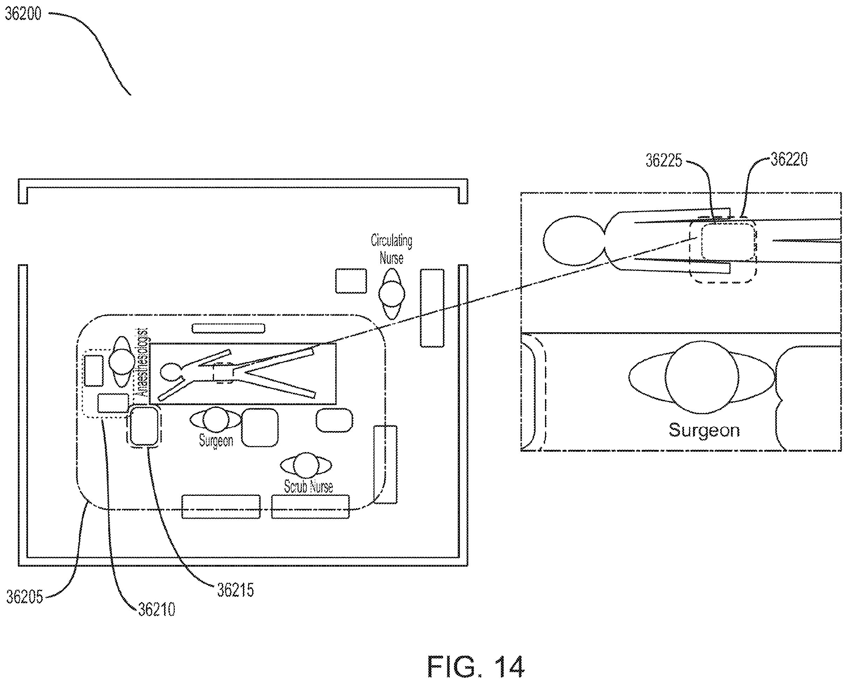

A virtual boundary associated with a restricted access area described herein may be associated with a virtual boundary area (e.g., a constant virtual boundary area), a selective boundary area, and/or an adaptive boundary area. FIG. 14 illustrates examples of a virtual boundary area (e.g., a constant virtual boundary area), a selective virtual boundary area, and/or an adaptive virtual boundary area in an OR 36200. A virtual boundary area (e.g., a constant virtual boundary area) may be associated with a predefined and/or a preconfigured equipment area in an OR. For example, a virtual boundary area may be associated with a sterile field area for operating zone 36205, a virtual boundary area for anesthesiologist 36210, a virtual boundary area for a surgical instrument, such as a high energy equipment area 36215 and/or the like. The virtual boundary area described herein may be unchanged (e.g., unadjusted or constant). A virtual boundary area may be associated with a high-risk area. For example, a high-risk area may be associated with an operating zone for an infectious disease, contagious disease, and/or a surgical operation with a radioactive material.

A virtual boundary area may be a predefined area around an equipment area in an OR, e.g., based on a preference of a surgeon, a guideline of a hospital, a health organization protocol, and/or the like. A virtual boundary area may be a predefined area around a high-risk area (e.g., following a guideline of a health agency, such as the Centers for Disease Control and Prevention, the World Health Organization, and/or the like).

A virtual boundary area associated with an equipment area may be adjusted. An equipment area and/or a high-risk area may be moved and/or shifted, e.g., during a surgical procedure. Based on the moved and/or shifted area of an equipment area and/or a high-risk area, a virtual boundary area for the equipment may be moved and/or shifted accordingly (e.g., with the equipment).

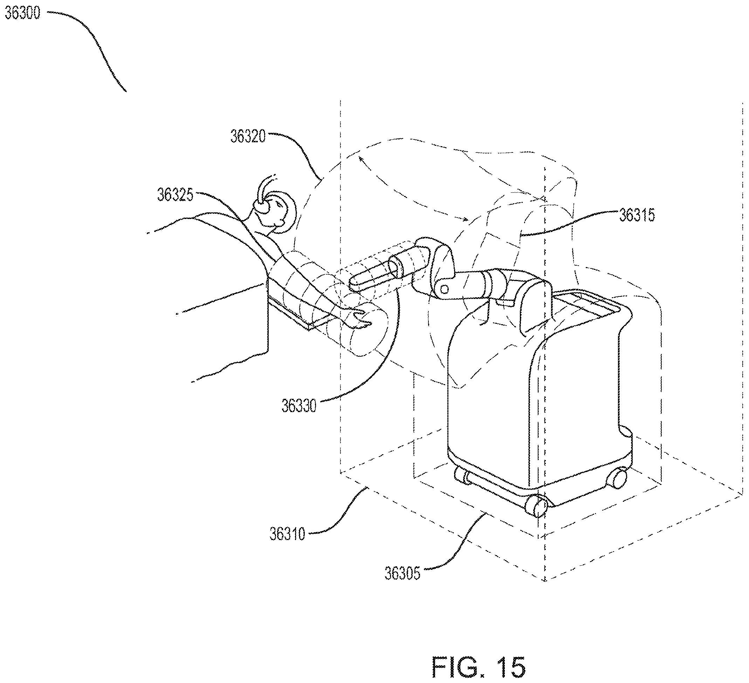

A virtual boundary area for an equipment area and/or a high-risk area may be adjusted, e.g., self-adjusted automatically. In examples, a surgical equipment may be moved and/or shifted when the surgical equipment is in an on position or in an off position (e.g., as will be illustrated and described in FIG. 15). The virtual boundary area associated with the surgical equipment may be self-adjusted accordingly. In examples, during a surgical operation, the body of a patient may be repositioned and/or moved. Based on the repositioned and/or moved body of the patient, the virtual boundary area associated with a high-risk area may be adjusted accordingly.

A virtual boundary area for an equipment area and/or a high-risk area may be adjusted by the computing system. In examples, the computing system may determine whether a surgical equipment is in an on position or in an off position. The computing system may adjust a virtual boundary area for an equipment area when the computing system determines that a surgical equipment is in an on position or in an off position.

In examples, the computing system may monitor and/or track the body position of a patient during a surgical operation. The computing system may determine that a patient is a high-risk area. If the computing system determines that the body position of the patient has been repositioned, moved, adjusted, and/or the like, the computing system may adjust the virtual boundary area for a high-risk area (e.g., associated with the body of the patient).

A virtual boundary area for an equipment area and/or a high-risk area may be adjusted by an HCP. In examples, a surgical instrument may be moved by an HCP during a surgical procedure. The HCP may adjust the virtual boundary area for the surgical instrument as the surgical instrument is being moved. In examples, an HCP may reposition the body of a patient for a current surgical step or procedure. Based on the reposition of the patient, the HCP may adjust the virtual boundary area associated with a high-risk area (e.g., associated with the body of the patient) accordingly.

A virtual boundary associated with a restricted access area described herein may have a selective boundary area. For example, an HCP, such as a surgeon, may define a virtual boundary area of an operating zone around a patient. As illustrated in FIG. 14, such area may be focused on or around a stomach of a patient 36220. A surgical instrument, such as a high energy surgical instrument, may be activated inside of a selective virtual boundary area, e.g., on or around the stomach of the patient 36220. The surgical instrument may be inactive outside of the selective virtual boundary area. For example, the computing system may identify a selective virtual boundary, e.g., generated by an HCP. The computing system may monitor a location and/or a position of a surgical instrument. The computing system may monitor an energy state of the surgical instrument. If the computing system determines that the surgical instrument is located and/or positioned within the boundary of the selective virtual boundary area, the computing system may generate a signal to activate the surgical instrument (e.g., transition from a low energy state to a high energy state). If the computing system determines that the surgical instrument is located and/or positioned outside the selective virtual boundary area and/or if the energy state of the surgical state has been activated while outside of the selective virtual boundary area, the computing system may generate a signal to deactivate the surgical instrument. The computing system may send an alert to an HCP indicating that the surgical instrument has been activated outside of the selective virtual boundary area.

A virtual boundary associated with a restricted access area described herein may have an adaptive boundary. During a surgical procedure, the body of a patient may be repositioned or moved. A selective virtual boundary of a surgical instrument (e.g., generated by an HCP) may be changed or adjusted upon detecting the reposition and/or movement. For example, as illustrated in FIG. 14, the body of a patient may be repositioned (e.g., repositioned to its side) and the selective virtual boundary area may be reduced and/or an arm of the patient may be in the selective virtual boundary area where a surgical instrument may be allowed to be in a high energy state. An adaptive virtual boundary area 36225 may be generated based on the change and/or reposition of the body of the patient, and the surgical instrument may not be activated in a high energy state to the repositioned arm area of the patient.

The computing system may generate an adaptive virtual boundary area and/or identify an adaptive virtual boundary area generated by an HCP. For example, the computing system may monitor a position of a patient and a selective virtual boundary area. If the computing system detects and/or determines that the position the patient has been changed and/or the selective virtual boundary area needs to be adjusted, the computing system may generate an adaptive virtual boundary area to accommodate for the change. The adaptive virtual boundary area may be temporary and may be turned off if the body of the patient is changed (e.g., changed back to the earlier position). An HCP may generate an adaptive virtual boundary area. As an HCP repositions the body of a patient, the HCP may indicate an adaptive virtual boundary area to the computing system, and the computing system may identify the adaptive virtual boundary area defined by the HCP.

A virtual boundary described herein may be preconfigured by an HCP. For example, an HCP preparing for a surgical procedure may preconfigure one or more virtual boundary areas in an OR. The virtual boundary areas may be based on a placement of surgical instruments, a placement of a surgical table, a placement of a surgical sterile tray table, an anesthetist zone, and/or the like. An HCP may manually input and/or adjust the virtual boundary areas.

The computing system may determine one or more virtual boundary areas based on past data associated with HCPs and/or a surgical procedure. For example, the computing system may determine that the same surgical procedure has been performed by the same HCPs. Based on the past data, the computing system may preconfigure the one or more virtual boundary areas. The past data may be or may include data from a different OR, preferences of HCPs, pre-op data, and/or the like.

As described herein, a surgical operation may be associated with an infectious disease, airborne contamination, and/or the like. A virtual boundary may be created for an infectious disease control tracking, airborne contaminate zone monitoring, and/or the like. For example, a virtual boundary may be associated with a filtration system area, a particle capture system area, an airborne contamination zone, and/or the like.

The computing system may identify an HCP in an OR. As described herein, the computing system may identify an HCP based on ID tag, camera feed, facial recognition, and/or the like. Based on the identification of the HCP in an OR, the computing system may determine an access authorization associated with the HCP. The computing system may look up, download, and/or determine the access authorization associated with the HCP. The access authorization may be or may include access authorization information for respective HCP to interact with, enter, exit, and/or be positioned within a virtual boundary area.

The computing system may monitor a movement of the identified HCP in the OR. For example, the computing system may track the movement of the HCP. The computing system may monitor the movement of the HCP, e.g., based on a camera system in the OR, RFID tag associated with the HCP, and/or the like.

Based on the monitored movement of the HCP and the access authorization associated with the HCP, the computing system may determine whether the HCP has an access authorization to interact with a virtual boundary associated with a restricted access area. As described herein, a virtual boundary associated with a restricted access area may be associated with a surgical equipment area, a high-risk area, an infectious disease control area, an air borne contamination area, and/or the like.

If the computing system determines that an HCP does not have an access authorization to interact with or enter (e.g., unauthorized to enter) a virtual boundary associated with a restricted access area and is in a proximity to the virtual boundary area, the computing system may send a notification to the HCP. The notification to the HCP may indicate that the HCP is unauthorized to interact with or enter the virtual boundary area. The computing system may send the notification to a device associated with the HCP, and the notification may be or may include one or more of a visual notification, an audio notification, a haptic notification, or an augmented reality notification. The computing system may send a notification to a display in the OR and/or a device associated with one or more other HCPs in the OR.

After sending a notification to the HCP who is unauthorized to interact with or enter the virtual boundary area, if the computing system determines that the HCP continues to approach the virtual boundary area and/or move closer to the virtual boundary area, the computing system may send a notification to a display in the OR and/or a device associated with one or more other HCPs in the OR and may alert one or more other HCPs in the OR.

If the computing system determines that an HCP has an access authorization to enter a virtual boundary associated with a restricted access area, the computing system may allow the HCP to interact with, enter, exit, and/or be positioned within the virtual boundary area. For example, the computing system may skip sending a notification to the HCP.

As described herein, a virtual boundary may be associated with a static (e.g., constant) boundary around a sterile field area. The computing system may monitor movements of HCPs in an OR and may send a notification to one or more HCPs who are not wearing proper sterile equipment. For example, the computing system may send a notification to an HCP who is not wearing sterile gown and/or is uncertified for entering a virtual boundary of a sterile field area as the HCP is approaching a proximity area of the virtual boundary area for the sterile field. After sending the notification to the HCP and if the computing system determines that the HCP is about to interact with (e.g., enter) the virtual boundary area for a sterile field area, the computing system may send a notification to a display in the OR and/or other HCPs, such as a scrub nurse and/or to broadcast the notification to the OR at large, to block or intervene the unauthorized HCP from interacting with or entering the virtual boundary area for a sterile field area. The computing system may prevent potential contamination in the virtual boundary area for a sterile field area by sending the notification and blocking the unauthorized HCP from interacting with the virtual boundary area.

As described herein, a virtual boundary may be associated with an area for treating an infectious patient. A dynamic constant virtual boundary may be created around the area where aerosolized particles can be loose. The virtual boundary for the area may be associated with a filtration system area and/or a particle capture system area. The computing system may monitor a movement of an HCP in an OR. The computing system may identify HCPs with PPE in the OR and/or HCPs with certificates or access authorizations to handle a contagious and/or infectious surgical operation, such as treating an infectious patient. If the computing system determines that an HCP does not have a PPE, a certificate, and/or an access authorization to enter the virtual boundary area for an infectious patient, the computing system may send a notification to the HCP. The computing system may alert the HCP, using the notification, to stay away the virtual boundary area for the infectious patient, e.g., via the notification.

A virtual boundary associated with a restricted access area may be or may include an equipment area. As described herein, a virtual boundary for an equipment area may be a predefined boundary surrounding the equipment area. As the equipment moves around during a surgical procedure, the virtual boundary around the equipment may stay constant. For example, a constant predefined virtual boundary area may be generated for a monitor, a surgical table, a sterile table, a surgical equipment, and/or the like. The constant predefined virtual boundary may exist during the surgical procedure. The computing system may monitor (e.g., keep track of) a movement of the equipment during the surgical procedure. The computing system may keep unauthorized HCP(s) out of the virtual boundary area for the equipment as the equipment gets moved around during the surgery. The computing system may allow an authorized HCP to interact with, enter, exit, be located within the constant predefined virtual boundary area for the equipment.

A surgical instrument, such as a robotic arm illustrated in FIG. 15, may send a notification to one or more HCPs in the OR. For example, prior and/or during a movement, a surgical instrument may indicate to the HCPs in the OR that a movement is occurring. The surgical instrument and/or the computing system may indicate a virtual boundary area where the movement will end and display the adjusted area to HCPs. For example, the computing system may provide a visual indication (e.g., such as a colored laser) to indicate the adjusted location of the virtual boundary area associated with the surgical instrument. The visual indication may allow the unauthorized HCPs to keep out of the virtual boundary area for the surgical instrument and allow the authorized HCPs to relocate to be in the virtual boundary, if necessary.

An HCP and/or an equipment may block a movement, e.g., an end movement, of a surgical instrument. If the surgical instrument determines that the surgical instrument cannot reach a full end movement, the surgical instrument may send a notification to an HCP. For example, the surgical instrument may provide an audible alert to the HCP to move out of the way, ask the HCP to move the equipment that is blocking the surgical instrument reaching the end movement, and/or ask the HCP to readjust the surgical instrument. The surgical instrument may provide a visual indication to the HCP, e.g., to ensure that the surgical instrument does not make contact with an HCP and/or an equipment, does not disrupt a current surgical procedure, and/or cause an injury.

As described herein, a virtual boundary may be associated with a selective virtual boundary area. For example, a selective virtual boundary area may be based on a usage of an equipment in an OR. A selective virtual boundary area may be adjusted based on an energized state of a surgical instrument. For example, if the computing system determines that a surgical instrument is in an off mode and/or in a low energized state, the computing system may determine or identify a selective virtual boundary area to allow access to one or more HCPs, such as a surgeon, a scrub nurse, an observer, or etc. If the computing system determines that a surgical instrument is in a high energized state (e.g., a surgeon using the surgical instrument during a current surgical step), the computing system may adjust the selective virtual boundary area to exclude other HCPs who are not using the surgical instrument. For example, if the surgical instrument is in a high energized state, the computing system may block other HCPs and allow access to the surgeon who is performing a current surgical step.

A selective virtual boundary area associated with a surgical instrument may be set based on which arms are active and/or where the arms of the surgical instrument are positioned and can reach. For example, if the surgical instrument (e.g., the arms of the surgical instrument) is in an off mode and/or not moving, the selective virtual boundary area may be removed (e.g., temporarily and/or until the arms are operating).

FIG. 15 illustrates an example surgical instrument with an arm in an OR 36300. The surgical instrument may be in an off mode and a virtual boundary area associated with the off mode 36305 may be generated. A virtual boundary area of the arm of the surgical instrument may be in an off position 36315. If the surgical instrument is operating and/or is in an on mode, a selective virtual boundary area around the surgical instrument associated with an on mode 36310 may be generated. The selective virtual boundary area around the surgical instrument for the on mode 36310 may have a larger area than the virtual boundary area associated with the off mode 36305 and allow the surgical instrument to move around. A selective virtual boundary area of the arm of the surgical boundary associated with an on position 36320 may be generated. The selective virtual boundary 36320 may be adjusted based on a space that the arm can occupy while in use (e.g., while operating). For example, the selective virtual boundary area of the arm of the surgical instrument in the on mode 36320 may cover a sweeping motion of the arm moving.

For example, the surgical instrument shown in FIG. 15 may be an imaging system. For example, a hybrid OR may be built around an imaging device. For example, an imaging device may be a non-mobile imaging device, such as a computerized x-ray imaging (CT) device, magnetic resonance (MR) device, and/or the like. The imaging device may include a high energy imaging component that needs to be avoided by an HCP, e.g., via a virtual boundary area. The imaging device may include a boom and/or other movable component that may need repositioning manually or under power. The boom of the image device may have a selective virtual boundary area when the image device is being used. When the boom is being used, the selective virtual boundary area may be generated to prevent an HCP from an inadvertent interaction with the imaging device and/or any hazards associated with the imaging device.

The imaging device may be a mobile imaging system, e.g., as illustrated in FIG. 15. The mobile imaging system may be moved into and/or within an OR. The mobile imaging system may be manipulated to the site where an imaging is needed, e.g., via a stable arm. The envelope of positioning and/or the exposure area of the mobile imaging system may have corresponding selective virtual boundary areas.

Example mobile imaging system may be fluoroscopy, such as a C-Arm X-ray, a 3D imaging machine, and/or an ultrasonic imaging. As illustrated in FIG. 15, a stable arm of a mobile imaging device may have a selective virtual boundary area when the image device is being used. The selective virtual boundary for the stable arm of the mobile imaging device 36325 may be focused on or around an arm of a patient that needs an imaging review. When the stable arm of the imaging device is operating, the selective virtual boundary area 36325 may be generated to prevent HCPs from inadvertent interactions with the imaging device and/or any hazards associated with the imaging device.

A virtual selective boundary associated with an imaging device described herein may be outlined to a floor of an OR. The virtual selective boundary associated with an imaging device may include vertical boundaries, or three-dimensional boundaries. For example, a mobile system, illustrated in FIG. 15, may need a pace, e.g., a 2 ft spherical boundary, 3 ft off the floor, and may be focused on an imaging site of an arm of a patient for review during an orthopedic hand surgery.

A virtual boundary associated with a restricted access area may be adjusted based on one or more conditions. For example, as described herein, a virtual boundary for a radioactive device and/or a high energy imaging equipment may be adjusted, e.g., based on a power level and/or an imaging focus to minimize an inadvertent exposure to an HCP in an OR. The virtual boundary area may be based on a physical boundary of the equipment, a size of an OR, and/or an energized state (e.g., power level) of the instrument.

A virtual boundary associated with a restricted access area may be adjusted based on a surgical step and/or an infection possibility. For example, when performing an intubation or extubation, a patient may have a virtual boundary area generated around an area of a head and/or shoulders of the patient. The virtual boundary area may be small and/or contained by a filtration system during a surgical procedure, such as a Bronchoscope procedure. During intubation or extubation, the virtual boundary area may be adjusted. For example, during intubation or extubation, the virtual boundary area may be adjusted, e.g., adjusted to be larger due to a likelihood of aerosol from breathing contaminating a larger zone. The virtual boundary around the head and/or shoulders area of the patient may include an adaptive virtual boundary area. For example, the adaptive virtual boundary around the patent area may be adjusted dynamically and may be adjusted (e.g., reacted) as the patient coughs or forcibly expels air, and aerosol and the virtual boundary area may be adjusted by expanding the restricted area.

The computing system may notify HCPs in the OR if the adaptive virtual boundary area for the surgical instrument has been changed. For example, the computing system may notify HCPs that a virtual boundary area, e.g., an adaptive virtual boundary area, for the surgical instrument has been changed. The computing system may monitor the authorized HCPs in the virtual boundary area for exposure levels and/or verify against PPE that the HCPs are using are proper.

A virtual boundary area may be illustrated by using a laser light. HCPs in an OR may see the laser light and visually recognize where the virtual boundary areas in the OR are located. The virtual boundary areas may have different colors to represent different types of virtual boundary areas. For example, a constant virtual boundary area may have different laser color than a selective and/or an adaptive virtual boundary areas. A different later light may provide information about a danger level, a power level, and/or the like associated with a virtual boundary area.

A virtual boundary area may be defined by a layout of an OR, a location/position of a surgical instrument, and/or an equipment area in an OR. As described herein, a virtual boundary area may be adjusted based on a movement of a surgical instrument and/or an equipment during a surgical procedure. A virtual boundary area may be adjusted based on reposition of a patient (e.g., moving the patient to the sides) or reposition of an OR table and a sterile surgical tray table.

The computing system may send a notification to an unauthorized HCP when the computing system determines that the unauthorized HCP is approaching, is in a proximity to, and/or near a virtual boundary associated with a restricted access area in an OR. The computing system may send a notification to an authorized HCP regarding a boundary interaction, e.g., to ensure safety of the HCP. For example, the computing system may send a notification to an authorized HCP before and/or after entering and exiting the virtual boundary. The notification to the HCP (e.g., the authorized and/or unauthorized HCPs) may prevent accidental or inadvertent interaction with the virtual boundary area.

As described herein, the computing system may send a notification to an HCP within a virtual boundary of a restricted access area or upon approaching the virtual boundary area. The computing system may use one or more of a haptic notification, a visual notification, an audible notification, a visuo-haptic notification. The notification may be or may include a modality and/or feedback. The notification may provide a warning, provide awareness about a surrounding, such as a location of a virtual boundary of a restricted access area, prevent a collision others or equipment, such as a sterile area, and/or an indication if an individual is near or violate a controlled area or boundary.

The computing system may provide a visual feedback. The computing system may provide a visual indicator and display a virtual boundary of a restricted access area or zone within an OR. The computing system may utilize a laser projector to project (e.g., outline) a virtual boundary of a restricted area or zone on a floor, on a wall, and/or within an OR. For example, the computing system may use a laser projector to provide a visual indication of a virtual area and/or zone to an HCP in an OR. The computing system may use different colors to represent different virtual boundaries associated with the restricted areas/zones. For example, the computing system may use a blue color to indicate a virtual boundary for a sterile field area, an orange color for a virtual boundary for a high energy equipment, a purple color for a virtual boundary for an anesthesiologist equipment, and/or the like. The computing system may change the color of the lights when an unauthorized HCP is approaching the virtual boundary of a restricted access area. For example, the computing system may turn the lights to yellow if an unauthorized HCP is near the virtual boundary of the restricted access area and turn the lights to red if the unauthorized HCP enters /interacts with the virtual boundary of the restricted access area.

The computing system may use one or more laser markings for virtual boundaries associated with restricted access areas/zones based on one or more of roles, permissions, certificates, etc., associated with HCPs in an OR. For example, a virtual boundary for a sterile area/zone may show a green color when an HCP with a correct authentication and/or an approved credential is near or within the virtual boundary. If an unauthorized HCP with an incorrect authentication and/or improper credential is near or within the virtual boundary, the computing system may use red color to provide a warning and/or an alert to the unauthorized HCP.

The computing system may generate a virtual boundary for a restricted access area, and the virtual boundary may be visible using an augmented reality (AR) device, such as AR glasses. For example, the computing system may generate colored zones as described herein and/or create laser lights onto a OR floor and HCPs may see the virtual boundaries using the AR devices.

The computing system may display an OR. For example, the computing system may display one or more of an OR, HCPs in the OR and movements of the HCPs in the OR, surgical equipment, and the like. The computing system may display one or more virtual boundaries of restricted access areas. The computing system may highlight the virtual boundary areas if an unauthorized HCP is approaching and/or interacting with the virtual boundary area. The computing system may provide a bird's eye overview of the OR to the HCPs in the OR. The OR display and the displayed information may enable an HCP, such as a surgeon, to reinforce one or more virtual boundary areas and/or override the virtual boundary areas that are highlighted.

The computing system may provide an audible feedback. The computing system may provide an audible tone or audible information to an HCP near or interacting with a virtual boundary of a restricted access area. For example, the computing system may broadcast audible information about a virtual boundary area as an authorized HCP is approaching the boundary area or if an unauthorized HCP is approaching or interacting with the virtual boundary area. The computing system may provide different tones based on a defined virtual boundary area. For example, the computing system may have different tones for a virtual boundary for a sterile field area, a virtual boundary for a patient area, a virtual boundary for an equipment area, a virtual boundary for a critical area.

The computing system may provide different tone level based on different virtual boundary areas and/or closeness of an HCP to the virtual boundary area. For example, if an unauthorized HCP is approaching a virtual boundary area, a computing system may provide a short quiet audible feedback to the HCP. As the unauthorized HCP is getting closer to the virtual boundary area, the audible feedback may be a long and louder audible feedback (e.g., increasing a level of the audible feedback). The computing system may provide the audible feedback to a corresponding HCP, e.g., via a device associated with the HCP. If the computing system is broadcasting the audible feedback in the OR, the computing system may noise-cancel the audible feedback to other HCPs, e.g., to minimize distraction.

The computing system may provide a haptic feedback. An HCP may be wearing one or more devices, such as a phone, a smart watch, a smart tracker may. For example, an HCP may be wearing a device on a wrist and/or an ankle (e.g., glove, hand, shoes, feet, etc.). The HCP may wear the device that may have wrist and/or ankle bands on each extremities. The computing system may provide a haptic feedback to an extremity that is at risk for contacting a virtual boundary for a restricted access area. For example, if a left foot of an unauthorized HCP is near a virtual boundary for a sterile field area, the computing system may send a haptic feedback to a device that is on a left ankle of the unauthorized HCP. The computing system may send the haptic feedback to the left ankle device and provide a notification that the HCP may not step or walk in that direction (e.g., left direction).

The computing system may provide a different level of a haptic feedback. The computing system may increase or decrease a level of a haptic feedback as an HCP is closing to and/or approaching or moving away from a virtual boundary of a restricted access area. For example, if an unauthorized HCP is approaching a virtual boundary of a restricted access area, the computing system may increase an intensity of a haptic feedback and/or increase a pulse of a haptic feedback. If the unauthorized HCP is moving away from the virtual boundary of the restricted access area, the computing system may decrease the intensity of the haptic feedback and/or decrease the pulse of the haptic feedback.

If the computing system determines that an unauthorized HCP is ignoring a haptic feedback to a particular device (e.g., to a left ankle device), the computing system may send a haptic feedback to one or more devices that the unauthorized HCP is wearing.

The computing system may combine one or more feedbacks described herein and may provide a visuo-haptic mixed reality (VHMR).

A computing system may monitor an HCP and may determine an access authorization for the HCP. For example, the computing system may determine an acceptability of crossing a virtual boundary of a restricted access area. The computing system may detect an environmental parameter and/or a personal parameter, e.g., to determine acceptability to crossing a virtual boundary area. For example, the computing system may detect an environmental parameter and/or a personal parameter based on one or more of a credential, a certificate, and/or other access control level information associated with an HCP.

The computing system may perform aseptic monitoring of an HCP. For example, if the computing system determines that an HCP passes an aseptic test, the computing system may allow the HCP to enter a virtual boundary of a restricted access area. If the computing system determines that an HCP fails an aseptic test, the computing system may revoke an access authorization associated with the HCP and disallow the HCP from entering a virtual boundary of a restricted access area.

As a surgery carries a risk of infection, an HCP in an OR may receive an extensive education in a technique to prevent a surgical infection. For example, an HCP in an OR (e.g., such as a surgeon or a scrub nurse) may be aware of how to properly wear and remove a sterile protective equipment, such as a gown and/or gloves, how to scrub hands to decrease a risk of bacterial contamination, and/or how to manage a surgical instrument/tool to prevent contamination of a sterile instruments or dressings. A circulating nurse, who does not wear a full sterile protective equipment (e.g., less sterile protective equipment than a scrub nurse and/or a surgeon), may be unauthorized to enter a virtual boundary of a restricted access area, such as a sterile area in an OR. The computing system may monitor a movement of a circulating nurse in the OR and prevent the circulating nurse from entering or interacting with the virtual boundary of a sterile field area and prevent from contaminating the sterile area around an operating table.

The computing system may monitor an HCP. For example, the computing system may monitor an HCP for pre-surgery and post-surgery, e.g., to verify that a necessary step has been completed. If the computing system determines that an HCP has failed to complete a step, such as a proper scrubbing before entering an OR or after performing the surgery, the computing system may send a notification to the HCP and/or revoke an access authorization for the HCP. The computing system may revoke an access authorization for the HCP temporarily, e.g., until the step has been completed.

The computing system may monitor an equipment during a surgery. For example, the computing system may monitor a surgical glove from any sign of breaking or monitor a surgical gown for a tear. If the computing system detects a tear or break, the computing system may send a notification to the HCP to replace the equipment.

The computing system may monitor battery transfer to a surgical device. For example, a battery pack (e.g., a lithium and/or lithium-ion battery pack) for a surgical device may not be sterilized. The battery pack may have an embedded RFID chip that tracks an aseptic transfer. For example, the battery pack may enter into an OR for a surgery. The computing system may receive an alert from the battery pack (e.g., via a RFID beacon), and the computing system may identify the batter pack entering the OR. The computing system may track the battery pack and send a notification to an HCP based on a virtual boundary of a restricted access area in the OR.

The computing system may monitor a procedure step associated with the battery back. If a procedure step has been missed or done incorrectly, the computing system may alert an HCP about a potential issue.

The computing system may identify a virtual boundary of a restricted access area for a surgical instrument. A surgical instrument may be able to perform a surgical step using a high energized state. The computing system may detect an energized state associated with a surgical instrument during a surgical procedure. The computing system may identify a surgical step of an operation. For example, the computing system may identify a surgical step based on surgical monitoring data. The surgical step may be or may include a current surgical step or an upcoming surgical step.

Based on the identified surgical step and the detected energized state, the computing system may adjust a virtual boundary area of the surgical instrument. For example, if the computing system detects that the surgical instrument is in a high energized state and matches with the surgical step, the computing system may adjust the virtual boundary of the surgical instrument, e.g., by increasing the virtual boundary area. If the computing system detects that the energized state is above a threshold level, the computing system may increase the virtual boundary area for the surgical instrument. If the computing system detects that the energized state is below the threshold level, the computing system may decrease the virtual boundary area for the surgical instrument.

If the computing system determines that the surgical instrument is in a high energized state but does not match with the surgical step, e.g., if an anomaly exists, the computing system may adjust the virtual boundary area of the surgical instrument. For example, the computing system may increase the virtual boundary area of the surgical instrument if the computing system detects that the surgical instrument has a high energized state when the current step of the surgical procedure does not need the surgical instrument to be turned on and/or be in use. The computing system may send a notification to an HCP, e.g., to prevent inadvertent contact with the surgical instrument.

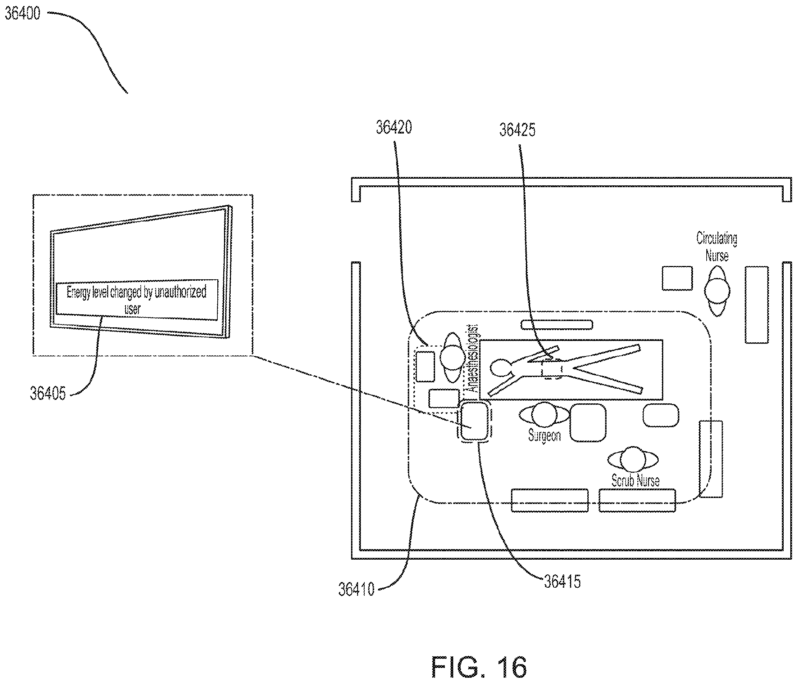

FIG. 16 illustrates an example notification of a computing system for an unauthorized energy level change of a surgical instrument. As illustrated in FIG. 16, the computing system may identify one or more virtual boundaries of restricted access areas in an OR 36400. The one or more virtual boundaries of restricted access areas in the OR may include a virtual boundary area for an anesthesiologist area 36420, a virtual boundary area for an operation table area 36410, a virtual boundary area for a surgical instrument area 36415, and/or a virtual boundary area for an operation area of a patient 36425. As described herein, the computing system may monitor a movement of an HCP in an OR. The computing system may determine whether the HCP has an access authorization to enter, exit, interact with, or be in a virtual boundary of a restricted access area in the OR. The computing system may determine an access authorization of an HCP based on one or more of an HCP role during a surgical procedure, an assignment associated with the HCP during the surgical procedure, a PPE associated with the HCP, a biomarker measurement associated with the HCP, or a restricted area type.

The computing system may monitor an energy level of a surgical instrument in an OR. If the computing system detects a change in the energy level of a surgical instrument, the computing system may determine that the energy level has been changed by an authorized HCP. If the computing system determines that the energy level has been changed by an unauthorized HCP who entered the virtual boundary area for a surgical instrument 36415, the computing system may send a notification. The computing system may send a notification to a display 36405 and notify other HCPs that the unauthorized HCP has inadvertently changed the energy level of the surgical instrument. The computing system may send a notification to the display 36405 and/or to the unauthorized HCP indicating that the HCP does not have an authorization to be in a proximity to or within the virtual boundary for the surgical instrument 36415.

If another HCP, such as a surgeon, sees the notification about the unauthorized HCP and/or the unauthorized energy level change by the unauthorized HCP, the surgeon, e.g., a lead surgeon, may adjust the access authorization associated with the unauthorized HCP. For example, the unauthorized HCP may be another surgeon who is assisting the lead surgeon. The assistant surgeon may have an access authorization to be in the virtual boundary for the operation table area 36410. The assistant surgeon may not have an access authorization to enter or be within the virtual boundary area for the surgical instrument 36415. The lead surgeon may adjust the access authorization of the assistant surgeon. For example, the lead surgeon may adjust the access authorization of the assistant surgeon after seeing the notification on the display 36405 and may allow the assistant surgeon to enter or be in the virtual boundary area for the surgical instrument 36415.

Another HCP may adjust the access authorization of the unauthorized HCP based on an emergency situation. For example, during an emergency where a lead surgeon needs additional hand to operate a surgical instrument, the lead surgeon may adjust an access authorization of an assistant surgeon to enter or be in a virtual boundary area for a surgical instrument 36415 as described herein.

A computing system may identify an equipment, such as a surgical instrument, associated with a surgical procedure in an OR. For example, the computing system may download a surgical plan created by an HCP for a pre-op procedure and may identify one or more surgical instruments to be used for a surgical procedure. The computing system may detect a control input by an HCP to control the surgical instrument. The control input by an HCP may be turning on or turning off the surgical instrument. The control input by an HCP may be adjusting an energy level of the surgical instrument. The control input by an HCP may be adjusting a position of the surgical instrument.

The computing system may determine the HCP's access control level associated with the surgical instrument. For example, an access control level associated with the HCP may include whether the HCP has an authorization to control the surgical instrument. The access control level associated with an HCP may be tiered. For example, a tiered access control level (e.g., a first tiered access control level) may be turning on or turning off the surgical instrument. Another tiered access control level (e.g., a second tiered access control level) may be adjusting an energy level of the surgical instrument. For example, the second tiered access control level associated with adjusting an energy level of a surgical instrument may be a higher tier than the first tired access control level associated with turning on or off the surgical instrument.

The computing system may determine whether to effectuate the detected control input by the HCP. The computing system may determine whether to effectuate the control input by the HCP based on the access control level associated with the HCP. If the computing system determines that the HCP is authorized to control the surgical instrument (e.g., HCP is authorized to effectuate the control input to control the surgical instrument) based on the access control level, the computing system may effectuate the control input by the HCP to control the surgical instrument. If the computing system determines that the HCP is unauthorized to control the surgical instrument (e.g., the HCP is unauthorized to effectuate the control input to control the surgical instrument) based on the access control level associated with the HCP, the computing system may block the control input by the HCP to control the surgical instrument.

The computing system may send an alert to a device associated with the HCP if the computing system blocks the control input by the HCP attempting to control the surgical instrument. The alert may be or may include a notification to the HCP, notifying that the control input by the HCP has been blocked and/or the access control level information associated with the HCP. The computing system may send an alert to a display in the OR. For example, as illustrated in FIG. 16, the computing system may send an alert to a display 36405 in an OR. The alert may indicate that the control input by the HCP has been blocked.

The computing system may be or may include an HCP monitoring system such as the HCP monitoring system 20000, 20002, 20003, or 20004 as described herein with respect to FIGs. 1-3. The computing system may be a computing system operatively connected to the HCP monitoring system(s) 20000, 20002, 20003, and/or 20004. The computing system may be or may include the computing system 20271 described herein with respect to FIG. 9. The computing system may be or may include the computer system 20063 described herein, for example, with respect to FIG. 4. The computing system may be or may include the computing system 20064 described herein, for example, with respect to FIG. 4. The computing system may be or may include the surgical hub 20006 as described herein with respect to FIGs. 1-3, surgical hub system 20060 in FIG. 4, the computer-implemented interactive surgical system 20070, in FIG. 5, the surgical hub or computing device 20243 in FIG. 7, the surgical hub 20270 in FIG. 9, the console 20294 in FIG. 10, and/or the surgical hub 5104 in FIG. 11. For example, the computing system may obtain surgical monitoring data associated with one or more surgical procedures. The surgical procedures may take place in an OR or multiple ORs.

As described herein, the computing system may monitor a movement of an HCP. The computing system may determine that the HCP is in a proximity to an operating table. The computing system may determine that the HCP is unauthorized to control a surgical instrument, e.g., based on an access control level associated with the HCP. The computing system may send an access control level adjustment message to another HCP, such as a surgeon. The access control level adjustment message may inquire whether the access control level associated with the HCP needs an adjustment based on the proximity of the HCP to the operating table. The computing system may receive an access control level adjustment request from another HCP, such as the surgeon. The access control level adjustment request may indicate a change to the access control level associated with the HCP. The computing system may adjust the access control level for the HCP based on receiving the access control level adjustment request from another HCP. As described herein, the access control level adjustment request may allow the HCP to control the surgical instrument (e.g., turn on/off the surgical instrument and/or adjust an energy level associated with the surgical instrument). The computing system may send an access control level adjustment notification to the HCP. The computing system may send the access control level adjustment notification to the display in the OR. The access control level adjustment notification may indicate the HCP who authorized the access control level change (e.g., by a surgeon).

The computing system may adjust a control access level for a surgical instrument associated with an HCP based on a surgical step in a surgical procedure. The computing system may identify a current surgical step. Based on the current surgical step, the computing system may determine whether to adjust the access control level associated with the HCP. For example, at an initial step of a surgical procedure, a control access level for a surgical instrument associated with a surgeon may be turning on or turning off the surgical instrument. If the surgeon attempts to adjust an energy level associated with the surgical instrument at the initial step of the surgical procedure, the computing system may block the control input by the surgeon.

The computing system may adjust the access control level associated with the HCP to allow the HCP to control the surgical instrument associated with the current surgical step in the surgical procedure. For example, the computing system may determine that the current surgical step involves using the surgical instrument to make an incision. The computing system may adjust the control level of the surgeon. The surgeon may adjust the energy level of the surgical instrument and may be able to make the incision, e.g., by adjusting from a low energized state to a high energized state of the surgical instrument.

After the HCP has completed the current surgical step, the computing system may readjust the access control level associated with the HCP that matches with an upcoming surgical step. For example, after the surgeon has made the incision for the current surgical step and the computing system determines that an upcoming surgical step does not involve a high energized stage of the surgical instrument, the computing system may readjust the access control level associated with the HCP to block a control input of the surgeon, e.g., to increase or to maintain the high energized level of the surgical instrument.

The computing system may adjust an HCP's access control level associated with an instrument based on an energized state of the instrument. The computing system may identify an energized state of a surgical instrument. If the computing system identifies that the surgical instrument has a high energized state, the computing system may adjust access control level of the HCP and block the control input by the HCP to control the surgical instrument. For example, to prevent an inadvertent control of the surgical instrument, the computing system may block a control input by a scrub nurse when the computing system determines that the surgical instrument is in a high energized state.

If the computing system identifies that the surgical instrument has a low energized state or turned off, the computing system may adjust the access control level of the HCP and effectuate the control input by the HCP to control the surgical instrument. For example, the computing system may allow a scrub nurse to turn on or turn off the surgical instrument when the computing system determines that the surgical instrument is in a low energized state or is turned off.

The computing system may adjust a control access level of an HCP based on biomarker measurement(s) associated with the HCP. A biomarker associated with an HCP may be or may include one or more of a fatigue level, a stress level, or an amount of time the HCP has been operating. The computing system may monitor a biomarker associated with an HCP, such as a surgeon. The computing system may determine whether to adjust the access control level associated with the HCP based on the monitored biomarker of the HCP. If the biomarker associated with the HCP indicates that the HCP has an increased fatigue level, an increased stress level, or an exceeded threshold operating hour, the computing system may block a control input from the HCP to control the surgical instrument. The computing system may send an alert to the HCP, indicating the increased fatigue level, the increased stress level, or the exceeded threshold operating hour.

If the biomarker associated with the HCP indicates that the HCP has a decreased fatigue level, a decreased stress level, or a below threshold operating hour, the computing system may effectuate a control input from the HCP to control the surgical instrument. The computing system may send an alert to the HCP indicating a current fatigue level, a current stress level, or a current operating hour associated with the HCP.

In examples, the computing system may receive measurement data associated with an HCP from a sensing system. Based on the measurement data, the computing system may determine an elevated fatigue level associated with an HCP. The measurement data may indicate an HCP, such as a surgeon, may take too long (e.g., longer than a threshold time) to a change in input, which may be referred to as over-correction, for a perceived mistake. The computing system may interpret repeated correction, over-correction, or oscillating reaction as an indicator of fatigue and/or elevated fatigue level associated with the HCP. For example, based on biomarker measurements of an HCP, the computing system may determine a value associated with hydration/dehydration of the HCP. Dehydration may impact energy levels and make a person feel tired and fatigued. Less body fluid tends to increase heart rate. The computing system may analyze heartbeat data in the context of hydration levels and differentiate between stress and other heart elevation events from hydration. The computing system may employ a baseline measure to differentiate acute events from ongoing chronic events and to differentiate between fatigue and dehydration.

In examples, the computing system may receive measurement data from a sensing system associated with an HCP in an OR (e.g., a sensing system associated with a surgeon). The measurement data may indicate that the HCP has an increased stress level. For example, an increased stress level may be indicated by change in a heart rate of the HCP, e.g., from a base value. The computing system may derive/infer an increased stress level of the HCP by cross-referencing the receipt of data from the corresponding sensing system.

An HCP's fatigue level may be measured /determined based on instrument usage data, for example. The computing system may calculate a weighted measure of fatigue for the HCP operating the surgical instrument as well as others in the operating room. The weighted measure of fatigue may be based on cumulative cooperative events and contributions. For example, the weighted measure of fatigue may be based on the intensity of stress experienced by an HCP and the force exerted by the HCP over time in controlling an actuator such as closure trigger over time.

Details on energy level and fatigue level measurements are described in U.S. App No. 17/156,287 (attorney docket number END9290USNP1), titled METHOD OF ADJUSTING A SURGICAL PARAMETER BASED ON BIOMARKER MEASUREMENTS, filed January 22, 2021.

As described herein, the computing system may adjust and/or limit a control input by an HCP to control a surgical instrument. For example, the computing system may limit a control input by an HCP to control a surgical instrument based on a permission, such as an access control level and/or a proximity to a virtual boundary of a restricted access area, such as a sterile field, an operating table, or other HCP such as a surgeon.

The computing system may adjust a control input by an HCP attempting to control the surgical instrument based on a certification associated with an HCP. For example, the computing system may use a certification associated with an HCP to determine whether the HCP has a permission to make a control input and/or change a setting for a surgical instrument. Based on the certification and/or the permission, the computing system may effectuate or block the control input by the HCP.

The computing system may limit and/or adjust a control input of an HCP to an equipment based on a certification associated with an HCP. The computing system may identify a predefined set of equipment and/or a control associated with the identified equipment, e.g., based on a certification associated with an HCP. The computing system may monitor a control input by an HCP, e.g., to prevent an inadvertent adjustment and/or control of an equipment that an HCP is certified to interact with and/or to control an equipment.

For example, an anesthesiologist may have a certification to control (e.g., full control) an anesthesiology equipment. Other HCPs may have a partial control of an anesthesiology equipment. For example, an anesthetist nurse and/or an anesthesiologist resident may have a partial control of an anesthesiology equipment. Based on a certification associated with an HCP, the computing system may allow or block the HCP to control an equipment. For example, the computing system may determine, e.g., based on the certification associated with the anesthesiologist, that the anesthesiologist is allowed to control a setting and/or have a control interaction for an anesthesiology equipment, such as a sedation and/or a respiration equipment.

Based on a certification associated with other HCP, such as a scrub nurse, the computing system may determine that a scrub nurse does not have a permission to control a setting and/or have a control interaction for an anesthesiology equipment. If the computing system determines that an HCP does not have a permission to control or adjust a setting for an anesthesiology equipment, the computing system may block a control input by the HCP and prevent an inadvertent adjustment of the equipment by the HCP who is not certified to interact with and/or control the equipment.

The computing system may receive an override request from another HCP, such as a surgeon or an anesthesiologist. For example, a surgeon may override (e.g., override a lockout) a control input and/or an adjustment setting of an equipment, e.g., to distinguish between an inadvertently adjustment and an intention or an emergency adjustment of an equipment.

In examples, throughout a surgical operation, one or more surgical instruments may be used. The surgical instruments may need a form of energy to apply a therapeutic treatment and/or have an electromechanical control to function the surgical instrument. An example of a surgical instrument may be a laparoscopic device. The surgical instrument may be used multiple times throughout the surgical operation. The surgical instrument may be placed on/off a surgical table, handed-off to other HCPs, and/or disposed after the surgical operation.

The computing system may limit and/or adjust a control input by an HCP, e.g., based on a confirmation. For example, the computing system may limit and/or adjust a control input by an HCP based on a visual confirmation. The computing system may detect an inadvertent control input based on a visual confirmation and prevent energy applied to motor power of a surgical instrument. The computing system may effectuate a control input if the computing system determines that the control input is from hands of and/or is activated by an authorized HCP with a proper (e.g., correct) certification having a permission.

As described herein, the computing system may generate a virtual boundary of a restricted access area for a target surgical zone and limit control input by an HCP to control a surgical instrument. For example, the computing system may effectuate a control input by an authorized HCP in a virtual boundary of a target surgical zone and prevent inadvertent control and/or prevent injuring other HCP or a patient.

The computing system may adjust an access control level associated with an HCP based on a proximity to other HCP, such as in a presence of a surgeon. For example, the computing system may determine that a scrub nurse or an associate may be unauthorized to operate a circular stapler. The computing system may determine that the scrub nurse or the associate may be in a proximity to or in a virtual boundary of a restricted access area of a surgeon. If the computing system determines that the scrub nurse or the associate is in the proximity to or in the virtual boundary of a surgeon area, the computing system may adjust the access control level associated with the scrub nurse or the associate. The computing system may effectuate a control input by the scrub nurse or the associate, e.g., turning on the circular stapler.

The computing system may be aware of and/or identify a surgical step of a surgical operation. For example, the computing system may identify that a surgery has been completed and/or a surgical instrument is no longer needed for the surgical operation. If the computing system determines that the surgery is completed, the computing system may monitor for an activation of the surgical instrument and/or be in a powered state. If the computing system determines that the surgical instrument has been activated and/or is in a high energized state or is turned on, the computing system may block a control input by an HCP, e.g., to prevent inadvertently activating the surgical instrument while the HCP is cleaning up and/or disposing the instrument.

If the computing system determines that a surgery has been completed and a surgical instrument is a battery-operated device, the computing system may begin an energy removal step, e.g., using a draining circuit of the battery-operated device. The computing system may limit (e.g., block) a control input by an HCP during the energy removal step.

The computing system may adjust and/or limit a control input by an HCP for gas system. During a surgical operation in an OR, medical air, oxygen, carbon dioxide, nitrogen, nitrous oxide, and/or other gases may be supplied. If the computing system detects a control input to turn on/off or increase/decrease gases supplied during an operation, the computing system may determine an access control level of the HCP making the control input to control the device for supplying the gases. The computing system may determine a surgical step and confirm that the gas needs to be turn on/off or increase/decrease. If the computing system determines that the HCP has the access control level to adjust the gas level and the HCP's control of gas matches with the current surgical step, the computing system may effectuate the control input by the HCP. If the computing system determines that the HCP does not have the access control level and/or use of gas does not match with the current surgical step, the computing system may block the control input by the HCP. The computing system may send an alert to the HCP as described herein.

The computing system may control an energized state of a device. The computing system may control an energized state of a device, such as a device for radiology, based on the location of the HCP, the virtual boundary of a restricted access area, whether the HCP is wearing appropriate PPE, a threshold exposure time, the biomarker measurement(s) of the HCP, the biomarker measurement(s) of the patient, and/or the like. As described herein, the computing system may control the energized state of a device and effectuate control input by the HCP to control the device, upon determining that the HCP is in a proximity to a patient (e.g., within a virtual boundary of a patient, a surgical table, and/or a surgeon) and within the surgical area.

The computing system may monitor/determine whether an HCP, such as a radiologist or a radiologist tech is wearing a proper PPE. If the computing system determines that the HCP is not wearing a proper PPE, the computing system may block a control input by the HCP and send an alert. Examples of a proper PPE may be or may include one or more of a lead apron, lead glasses, a lead shield, and/or the like.

The computing system may monitor/determine whether an HCP is in a proper distance, such as an acceptable clearance, from the radiology device. If the computing system determines that the HCP is not in a proper distance, e.g., in an exposure area, the computing system may block a control input by the HCP and send an alert.

The computing system may monitor/determine an exposure time of an HCP to a radiology device. For example, if the computing system determines that the HCP exceeds a threshold exposure time, the computing system may block a control input by the HCP and send an alert.

The computing system may monitor/determine a biomarker associated with an HCP or a patient. For example, if the computing system determines that an HCP has an increased fatigue level and/or an increased level, the computing system may block a control input by the HCP and send an alert. The computing system may monitor a biomarker associated with a patient. If the computing system determines that a biomarker associated with the patient has suddenly changed, e.g., an emergency or a complication happened, the computing system may block a control input by the HCP and send an alert. The computing system may determine a type of patient based on the biomarker associated with the patient. For example, the computing system may determine that the patient is a pediatric patient or a pregnant patient. To minimize an exposure to a radiology, the computing system may block or adjust the control input by the HCP and send an alert.

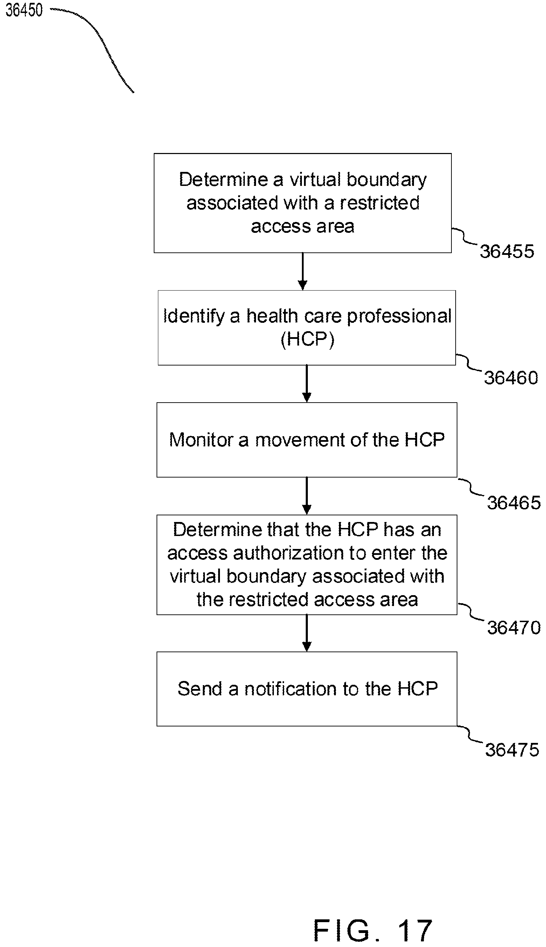

FIG. 17 illustrates an example flow 36450 of monitoring an HCP movement relative to a virtual boundary of restricted access area in an OR as described herein. For example, at 36455, a computing system may determine a virtual boundary associated with a restricted access area. The computing system may be or may include the surgical hub 20006 as described herein with respect to FIGs. 1-3, surgical hub system 20060 in FIG. 4, the computer-implemented interactive surgical system 20070, in FIG. 5, the surgical hub or computing device 20243 in FIG. 7, the surgical hub 20270 in FIG. 9, the console 20294 in FIG. 10, and/or the surgical hub 5104 in FIG. 11. The virtual boundary may be or may include a virtual boundary associated with a restricted access area for an anesthesiologist 36005, 36105, a virtual boundary area associated with a restricted access area for a surgeon 36025, 36115, a virtual boundary associated with a restricted access area for a surgical table 36030, 36120, a virtual boundary associated with a restricted access area for a scrub nurse 36035, 36125, a virtual boundary associated with a restricted access area for a circulating nurse 36040, a virtual boundary associated with a restricted access area for a surgical instrument, a sterile tray table, and/or other surgery related equipment 36045, 36050, 36055, 36060, 36065, a virtual boundary associated with a restricted access area for an observer 36135, a virtual boundary associated with a restricted access area for an SSC 36140, a virtual boundary associated with a restricted access area for an assistant, a virtual boundary associated with a restricted access area for an anesthesia nurse 36110, described herein with respect to FIGs. 12-13.

At 36460, the computing system may identify an HCP in an OR. At 36465, the computing system may monitor a movement of the identified HCP. For example, the computing system may determine whether an HCP is in a proximity to a virtual boundary associated with a restricted access area. The computing system may determine that the HCP is about to interact with (e.g., enter, exit, or leave) the virtual boundary associated with a rescripted access area.

At 36470, the computing system may determine that the HCP has an access authorization to interact with or enter/exit the virtual boundary associated with a restricted access area as described herein. At 36475, the computing system may send a notification to the HCP. For example, if the computing system determines that the HCP is in a proximity to the virtual boundary associated with a restricted access area (e.g., based on the monitored movement of the HCP in 36465) and if the computing system determines that the HCP is unauthorized to interact with or enter/exit the virtual boundary associated with a restricted access area (e.g., based on the determination of the HCP access authorization in 36470), the computing system may send a notification to the HCP indicating that the HCP is unauthorized to enter the virtual boundary. If the computing system determines that the HCP is in a proximity to the virtual boundary associated with a restricted access area (e.g., based on the monitored movement of the HCP in 36465) and if the computing system determines that the HCP is authorized to interact with or enter/exit the virtual boundary associated with a restricted access area (e.g., based on the determination of the HCP access authorization in 36470), the computing system may skip sending a notification to the HCP.

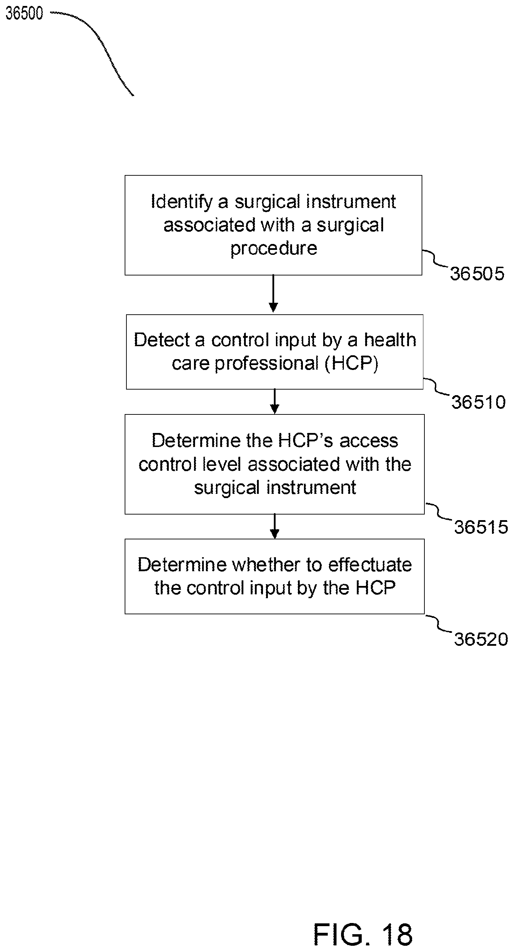

FIG. 18 illustrates an example flow 36500 of control access verification of an HCP in an OR, which may be performed at the computing system described herein. The computing system may be or may include the surgical hub 20006 as described herein with respect to FIGs. 1-3, surgical hub system 20060 in FIG. 4, the computer-implemented interactive surgical system 20070, in FIG. 5, the surgical hub or computing device 20243 in FIG. 7, the surgical hub 20270 in FIG. 9, the console 20294 in FIG. 10, and/or the surgical hub 5104 in FIG. 11.

At 36505, the computing system may identify a surgical instrument associated with a surgical procedure. For example, as described herein, the computing system may download a surgical plan generated by an HCP during a pre-op procedure. The computing system may identify one or more surgical instruments to be used for a surgery and identify one or more instruments to be used for the surgical procedure.

At 36510, the computing system may detect a control input by an HCP. For example, as described herein, the control input may be turning on/off a surgical instrument or change an energized state of the surgical instrument (e.g., from a low energized state to a high energized state or from the high energized state to the low energized state).

At 36515, the computing system may determine that an access control level of the HCP to control the surgical instrument. For example, the computing system may determine whether the HCP has an access control authorization to control the surgical instrument.

At 36520, the computing system may determine whether to effectuate the control input by the HCP. For example, if the computing system determines that the HCP has an access control authorization to control the surgical instrument, the computing system may effectuate the control input by the HCP to control the surgical instrument. If the computing system determines that the HCP is unauthorized to (e.g., does not have the access control authorization) to control the surgical instrument, the computing system may block the control input by the HCP.

A computing system may monitor an HCP or an equipment based on a location within an OR and/or an exposure time in a virtual boundary of a restricted access area. The computing system may determine, based on a local recording, how long an HCP has been in an OR and/or how long a device has been in an OR. For example, the computing system may determine who long a battery-operated devices (ENDO) has been set up in an OR. The battery-operated device may have a shelf life of 12 hours once the device has been set up and battery is installed. The computing system may determine that the battery-operated device may not have an optimum performance if the battery-operated device has been set up more than the shelf life.

The computing system may monitor/determine an exposure time in an OR for a device. As a device may have a specific life after the device has been set up, the computing system may send an alert to an HCP, e.g., to replace the device.

The computing system may track a location of a device within an OR. For example, the computing system may record a movement of the device within an OR and/or between virtual boundaries of restricted access areas, an interaction of the device with an HCP, and/or the like.

## Claims

1. A computer-implemented method comprising:
identifying a surgical instrument associated with a surgical procedure and a healthcare professional (HCP) in an operating room (OR);
identifying the HCP;
detecting a control input by the HCP to control the surgical instrument;
determining the HCP's access control level associated with the surgical instrument; and
based on the detected control input by the HCP, determine whether to effectuate the control input based on the HCP's access control level associated with the surgical instrument,
wherein based on the determination that the HCP's access control level associated with the surgical instrument does not authorize the HCP to effectuate the control input to control the surgical instrument, blocking the control input by the HCP to control the surgical instrument, and
wherein based on the determination that the HCP's access control level associated with the surgical instrument authorizes the HCP to effectuate the control input to control the surgical instrument, effectuating the control input by the HCP to control the surgical instrument.

2. The computer-implemented method of claim 1, wherein the access control level associated with the surgical instrument comprises a first access control level to control the surgical instrument and a second access control level to control the surgical instrument, and the first access control level comprises at least one of turning on the surgical instrument or turning off the surgical instrument and the second access control level comprises at least one of increasing an energy level associated with the surgical instrument or decreasing the energy level associated with the surgical instrument.

3. The computer-implemented method of claim 1 or claim 2, wherein the control input is a first control input, the HCP is a first HCP, and the method comprises:
detecting a second control input by a second HCP to control the surgical instrument, wherein the second control input is configured to turn on or turn off the surgical instrument;
determining the second HCP's access control level associated with the surgical instrument; and
based on the determination that the second HCP's access control level associated with the surgical instrument authorizes the second HCP to turn on or turn off the surgical instrument, effectuating the second control input to turn on or turn off the surgical instrument.

4. The computer-implemented method of any one of claims 1 to 3, wherein the control input is a first control input, the HCP is a first HCP, and the method comprises:
detecting a second control input by a second HCP to control the surgical instrument, wherein the second control input is configured change an energy level associated with the surgical instrument;
determining the second HCP's access control level associated with the surgical instrument; and
based on the determination that the second HCP's access control level associated with the surgical instrument does not authorize the second HCP to change the energy level associated with the surgical instrument, blocking the control input to control the surgical instrument.

5. The computer-implemented method of any one of claims 1 to 4, comprising:
sending an alert to at least one of a device associated with the HCP or a display in the OR, the alert comprising at least one of a notification that the control input by the HCP has been blocked or access control level information assigned to the HCP.

6. The computer-implemented method of any one of claims 1 to 5, wherein the HCP is a first HCP, and the method comprises:
monitoring a movement associated with the first HCP;
determining that the first HCP is in a proximity to an operating table;
based on the determination that the first HCP is in the proximity to the operating table, sending an access control level adjustment inquiry message to a second HCP, the access control level adjustment inquiry message being configured to prompt the HCP to indicate whether the HCP's access control level associated with the surgical instrument needs an adjustment based on the proximity of the first HCP to the operating table;
receiving an access control level adjustment request from the second HCP in response to the access control level adjustment inquiry message; and
based on the access control level adjustment request, adjusting the access control level associated with the first HCP.

7. The computer-implemented method of claim 6, wherein the method comprises:
sending an access control level adjustment notification to the HCP, the access control level adjustment notification notifying that the HCP's access control level associated with the surgical instrument has been adjusted by the second HCP.

8. The computer-implemented method of any one of claims 1 to 7, wherein the method comprises:
identifying a current surgical step in the surgical procedure;
determining whether to adjust the access control level associated with the HCP based on the identified current surgical step; and
based on the determination to adjust the HCP's access control level associated with the surgical instrument, adjusting the HCP's access control level associated with the surgical instrument to allow the HCP to control the surgical instrument during the current surgical step in the surgical procedure.

9. The computer-implemented method of any one of claims 1 to 8, wherein the method comprises:
identifying an energized state of the surgical instrument; and
determining whether to adjust the HCP's access control level associated with the surgical instrument based on the identified energized state of the surgical instrument, wherein based on the identified energized state of the surgical instrument being at a high energized state, the method comprises adjusting the HCP's access control level associated with the surgical instrument to block the control input by the HCP to control the surgical instrument, and wherein based the identified energized state of the surgical instrument being at a low energized state, the method comprises adjusting the HCP's access control level associated with the surgical instrument to effectuate the control input by the HCP to control the surgical instrument.

10. The computer-implemented method of any one of claims 1 to 9, wherein the method comprises:
monitoring a biomarker associated with the HCP, the biomarker may comprise at least one of a fatigue level or a stress level; and
determining whether to adjust the HCP's access control level associated with the surgical instrument based on the monitored biomarker associated with the HCP, wherein based on the monitored biomarker associated with the HCP indicating that the HCP has an increased level of at least one of the fatigue level or the stress level, the method comprises adjusting the HCP's access control level associated with the surgical instrument to block the control input by the HCP to control the surgical instrument, and wherein based the monitored biomarker associated with the HCP indicating that the HCP has an acceptable level of at least one of the fatigue level or the stress level, the method comprises adjusting the HCP's access control level associated with the surgical instrument to effectuate the control input by the HCP to control the surgical instrument.

11. A computing system comprising:
a processor configured to perform the method of any one of claims 1 to 10.

12. A data processing device comprising means for carrying out the method of any one of claims 1 to 10.

13. A system comprising:
the computing system of claims 11; and
a surgical instrument communicatively coupled with the computer system.

14. A system comprising:
the data processing device of claim 12; and
a surgical instrument a communicatively coupled with the data processing device.

15. A computer readable medium having instructions which, when executed by a computer, cause implementation of the method according to any one of claims 1 to 10.

## Patentansprüche

1. Computerimplementiertes Verfahren, umfassend:
Identifizieren eines chirurgischen Instruments im Zusammenhang mit einem chirurgischen Eingriff und einer medizinischen Fachkraft (HCP) in einem Operationssaal (OR);
Identifizieren der HCP;
Erfassen einer Steuereingabe durch die HCP zur Steuerung des chirurgischen Instruments;
Bestimmen der mit dem chirurgischen Instrument verbundenen Zugriffssteuerungsebene der HCP; und
basierend auf der erkannten Steuereingabe durch die HCP, Bestimmen, ob die Steuereingabe basierend auf der mit dem chirurgischen Instrument verbundenen Zugriffssteuerungsebene der HCP ausgeführt werden soll,
wobei basierend auf der Feststellung, dass die mit dem chirurgischen Instrument verbundene Zugriffssteuerungsebene der HCP die HCP nicht autorisiert, die Steuereingabe zur Steuerung des chirurgischen Instruments zu bewirken, die Steuereingabe durch die HCP zur Steuerung des chirurgischen Instruments blockiert wird, und
wobei basierend auf der Bestimmung, dass die mit dem chirurgischen Instrument verbundene Zugriffssteuerungsebene der HCP die HCP autorisiert, die Steuereingabe zur Steuerung des chirurgischen Instruments auszuführen, die Steuereingabe durch die HCP zur Steuerung des chirurgischen Instruments ausgeführt wird.

2. Computerimplementiertes Verfahren nach Anspruch 1, wobei die dem chirurgischen Instrument zugeordnete Zugriffssteuerungsebene eine erste Zugriffssteuerungsebene zur Steuerung des chirurgischen Instruments und eine zweite Zugriffssteuerungsebene zur Steuerung des chirurgischen Instruments umfasst und die erste Zugriffssteuerungsebene mindestens eines von Einschalten des chirurgischen Instruments oder Ausschalten des chirurgischen Instruments umfasst und die zweite Zugriffssteuerungsebene mindestens eines von Erhöhen eines dem chirurgischen Instrument zugeordneten Energieniveaus oder Verringern des dem chirurgischen Instrument zugeordneten Energieniebene umfasst.

3. Computerimplementiertes Verfahren nach Anspruch 1 oder 2, wobei die Steuereingabe eine erste Steuereingabe ist, die HCP eine erste HCP ist und das Verfahren Folgendes umfasst:
Erfassen einer zweiten Steuereingabe durch eine zweite HCP zur Steuerung des chirurgischen Instruments, wobei die zweite Steuereingabe konfiguriert ist, um das chirurgische Instrument einzuschalten oder auszuschalten;
Bestimmen der mit dem chirurgischen Instrument verbundenen Zugriffssteuerungsebene der zweiten HCP; und
basierend auf der Bestimmung, dass die dem chirurgischen Instrument zugeordnete Zugriffssteuerungsebene der zweiten HCP die zweite HCP autorisiert, das chirurgische Instrument ein- oder auszuschalten, die zweite Steuereingabe zum Einschalten oder Ausschalten des chirurgischen Instruments zu bewirken.

4. Computerimplementiertes Verfahren nach einem der Ansprüche 1 bis 3, wobei die Steuereingabe eine erste Steuereingabe ist, die HCP eine erste HCP ist und das Verfahren Folgendes umfasst:
Erfassen einer zweiten Steuereingabe durch eine zweite HCP zur Steuerung des chirurgischen Instruments, wobei die zweite Steuereingabe konfiguriert ist, um eine mit dem chirurgischen Instrument verbundene Energieebene zu ändern;
Bestimmen der mit dem chirurgischen Instrument verbundenen Zugriffssteuerungsebene der zweiten HCP; und
basierend auf der Bestimmung, dass die dem chirurgischen Instrument zugeordnete Zugriffssteuerungsebene der zweiten HCP die zweite HCP nicht autorisiert, die dem chirurgischen Instrument zugeordnete Energieebene zu ändern, Blockieren der Steuereingabe zur Steuerung des chirurgischen Instruments.

5. Computerimplementiertes Verfahren nach einem der Ansprüche 1 bis 4, umfassend:
Senden einer Warnung an mindestens eine Vorrichtung, die mit der HCP assoziiert ist, oder an eine Anzeige im OP, wobei die Warnung mindestens eine Benachrichtigung darüber enthält, dass die Steuereingabe durch die HCP blockiert wurde, oder Informationen über die Zugriffssteuerungsebene, die der HCP zugewiesen wurde.

6. Computerimplementiertes Verfahren nach einem der Ansprüche 1 bis 5, wobei die HCP eine erste HCP ist und das Verfahren Folgendes umfasst:
Überwachen einer Bewegung in Verbindung mit der ersten HCP;
Bestimmen, dass sich die erste HCP in der Nähe eines Operationstisches befindet;
basierend auf der Bestimmung, dass sich die erste HCP in der Nähe des Operationstisches befindet, Senden einer Abfragenachricht zur Einstellung der Zugriffssteuerungsebene an eine zweite HCP, wobei die Abfragenachricht zur Einstellung der Zugriffssteuerungsebene konfiguriert ist, um die HCP aufzufordern, anzuzeigen, ob die Zugriffssteuerungsebene der HCP, die mit dem chirurgischen Instrument verbunden ist, eine Einstellung basierend auf der Nähe der ersten HCP zum Operationstisch benötigt;
Empfangen einer Anfrage zur Anpassung der Zugriffssteuerungsebene von der zweiten HCP als Antwort auf die Anfragenachricht zur Anpassung der Zugriffssteuerungsebene; und
basierend auf der Anfrage zur Anpassung der Zugriffssteuerungsebene, Anpassen der Zugriffssteuerungsebene, die mit der ersten HCP verbunden ist.

7. Computerimplementiertes Verfahren nach Anspruch 6, das Verfahren umfassend:
Senden einer Benachrichtigung über die Anpassung der Zugriffssteuerungsebene an die HCP, wobei die Benachrichtigung über die Anpassung der Zugriffssteuerungsebene meldet, dass die mit dem chirurgischen Instrument verbundene Zugriffssteuerungsebene der HCP von der zweiten HCP angepasst wurde.

8. Computerimplementiertes Verfahren nach einem der Ansprüche 1 bis 7, wobei das Verfahren Folgendes umfasst:
Identifizieren eines aktuellen chirurgischen Schritts im chirurgischen Verfahren;
Bestimmen, ob die mit der HCP verbundene Zugriffssteuerungsebene basierend auf dem identifizierten aktuellen chirurgischen Schritt angepasst werden soll; und
basierend auf der Bestimmung, die Zugriffssteuerungsebene der HCP, die mit dem chirurgischen Instrument verbunden ist, Anpassen der Zugriffssteuerungsebene der HCP, die mit dem chirurgischen Instrument verbunden ist, um der HCP zu erlauben, das chirurgische Instrument während des aktuellen chirurgischen Schrittes in dem chirurgischen Verfahren zu steuern.

9. Computerimplementiertes Verfahren nach einem der Ansprüche 1 bis 8, wobei das Verfahren Folgendes umfasst:
Identifizieren eines erregten Zustands des chirurgischen Instruments; und
Bestimmen, ob die dem chirurgischen Instrument zugeordnete Zugriffssteuerungsebene der HCP basierend auf dem identifizierten erregten Zustand des chirurgischen Instruments angepasst werden soll, wobei basierend auf dem identifizierten erregten Zustand des chirurgischen Instruments ein hoher erregter Zustand vorliegt, das Verfahren das Einstellen der dem chirurgischen Instrument zugeordneten Zugriffssteuerungsebene der HCP umfasst, um die Steuereingabe durch die HCP zur Steuerung des chirurgischen Instruments zu blockieren, und wobei das Verfahren basierend auf dem identifizierten erregten Zustand des chirurgischen Instruments, das sich in einem niedrigen erregten Zustand befindet, das Einstellen der dem chirurgischen Instrument zugeordneten Zugriffssteuerungsebene der HCP umfasst, um die Steuereingabe durch die HCP zur Steuerung des chirurgischen Instruments zu bewirken.

10. Computerimplementiertes Verfahren nach einem der Ansprüche 1 bis 9, wobei das Verfahren Folgendes umfasst:
Überwachen eines Biomarkers, der mit der HCP assoziiert ist, wobei der Biomarker mindestens einen Ermüdungsgrad oder einen Stressgrad umfassen kann; und
Bestimmen, ob die Zugriffssteuerungsebene der HCP, die mit dem chirurgischen Instrument assoziiert ist, basierend auf dem überwachten Biomarker, der mit der HCP assoziiert ist, angepasst werden soll, wobei basierend auf dem überwachten Biomarker, der mit der HCP assoziiert ist, der anzeigt, dass die HCP eine erhöhte Ebene des Ermüdungsniveaus und/oder des Stressniveaus aufweist, das Verfahren das Anpassen der Zugriffssteuerungsebene der HCP, die mit dem chirurgischen Instrument assoziiert ist, umfasst, um die Steuereingabe durch die HCP zur Steuerung des chirurgischen Instruments zu blockieren, und wobei das Verfahren basierend auf dem überwachten Biomarker, der mit der HCP assoziiert ist, der anzeigt, dass die HCP eine akzeptable Ebene des Ermüdungsniveaus und/oder des Stressniveaus aufweist, das Einstellen der Zugriffssteuerungsebene der HCP, die mit dem chirurgischen Instrument assoziiert ist, umfasst, um die Steuerungseingabe durch die HCP zur Steuerung des chirurgischen Instruments zu bewirken.

11. Rechensystem, umfassend:
einen Prozessor, der konfiguriert ist, um das Verfahren nach einem der Ansprüche 1 bis 10 durchzuführen.

12. Datenverarbeitungsvorrichtung, umfassend Mittel zum Ausführen des Verfahrens nach einem der Ansprüche 1 bis 10.

13. System, umfassend:
das Rechensystem nach Anspruch 11; und
ein chirurgisches Instrument, das kommunikativ mit dem Computersystem verbunden ist.

14. System, umfassend:
die Datenverarbeitungsvorrichtung nach Anspruch 12; und
ein chirurgisches Instrument, das kommunikativ mit der Datenverarbeitungsvorrichtung gekoppelt ist.

15. Computerlesbares Medium mit Anweisungen, die, wenn sie von einem Computer ausgeführt werden, die Durchführung des Verfahrens nach einem der Ansprüche 1 bis 10 bewirken.

## Revendications

1. Procédé mis en œuvre par ordinateur comprenant :
l'identification d'un instrument chirurgical associé à une intervention chirurgicale et à un professionnel de santé (HCP) dans une salle d'opération (OR) ;
l'identification du HCP ;
la détection d'une entrée de commande par le HCP pour commander l'instrument chirurgical ;
la détermination du niveau de commande d'accès du HCP associé à l'instrument chirurgical ; et
sur la base de l'entrée de commande détectée par le HCP, le fait de déterminer si l'entrée de commande doit être effectuée sur la base du niveau de commande d'accès du HCP associé à l'instrument chirurgical,
dans lequel, sur la base de la détermination que le niveau de commande d'accès du HCP associé à l'instrument chirurgical n'autorise pas le HCP à effectuer l'entrée de commande pour commander l'instrument chirurgical, le blocage de l'entrée de commande par le HCP pour commander l'instrument chirurgical, et
dans lequel, sur la base de la détermination que le niveau de commande d'accès du HCP associé à l'instrument chirurgical autorise le HCP à effectuer l'entrée de commande pour commander l'instrument chirurgical, la réalisation de l'entrée de commande par le HCP pour commander l'instrument chirurgical.

2. Procédé mis en œuvre par ordinateur selon la revendication 1, dans lequel le niveau de commande d'accès associé à l'instrument chirurgical comprend un premier niveau de commande d'accès pour commander l'instrument chirurgical et un second niveau de commande d'accès pour commander l'instrument chirurgical, et le premier niveau de commande d'accès comprend au moins l'un parmi la mise en marche de l'instrument chirurgical ou l'arrêt de l'instrument chirurgical, et le second niveau de commande d'accès comprend au moins l'un parmi l'augmentation d'un niveau d'énergie associé à l'instrument chirurgical ou la diminution du niveau d'énergie associé à l'instrument chirurgical.

3. Procédé mis en œuvre par ordinateur selon la revendication 1 ou la revendication 2, dans lequel l'entrée de commande est une première entrée de commande, le HCP est un premier HCP, et le procédé comprend :
la détection d'une seconde entrée de commande par un second HCP pour commander l'instrument chirurgical, dans lequel la seconde entrée de commande est configurée pour mettre en marche ou arrêter l'instrument chirurgical ;
la détermination du niveau de commande d'accès du second HCP associé à l'instrument chirurgical ; et
sur la base de la détermination que le niveau de commande d'accès du second HCP associé à l'instrument chirurgical autorise le second HCP à mettre en marche ou arrêter l'instrument chirurgical, la réalisation de la seconde entrée de commande pour mettre en marche ou arrêter l'instrument chirurgical.

4. Procédé mis en œuvre par ordinateur selon l'une quelconque des revendications 1 à 3, dans lequel l'entrée de commande est une première entrée de commande, le HCP est un premier HCP, et le procédé comprend :
la détection d'une seconde entrée de commande par un second HCP pour commander l'instrument chirurgical, dans lequel la seconde entrée de commande est configurée pour modifier un niveau d'énergie associé à l'instrument chirurgical ;
la détermination du niveau de commande d'accès du second HCP associé à l'instrument chirurgical ; et
sur la base de la détermination que le niveau de commande d'accès du second HCP associé à l'instrument chirurgical n'autorise pas le second HCP à modifier le niveau d'énergie associé à l'instrument chirurgical, le blocage de l'entrée de commande pour commander l'instrument chirurgical.

5. Procédé mis en œuvre par ordinateur selon l'une quelconque des revendications 1 à 4, comprenant :
l'envoi d'une alerte à au moins l'un parmi un dispositif associé au HCP ou un écran dans la salle d'opération, l'alerte comprenant au moins l'un parmi une notification indiquant que l'entrée de commande par le HCP a été bloquée ou des informations sur le niveau de commande d'accès attribué au HCP.

6. Procédé mis en œuvre par ordinateur selon l'une quelconque des revendications 1 à 5, dans lequel le HCP est un premier HCP et le procédé comprend :
la surveillance d'un mouvement associé au premier HCP ;
la détermination que le premier HCP est à proximité d'une table d'opération ;
sur la base de la détermination que le premier HCP est à proximité de la table d'opération, l'envoi d'un message de demande d'ajustement de niveau de commande d'accès à un second HCP, le message de demande d'ajustement de niveau de commande d'accès étant configuré pour inviter le HCP à indiquer si le niveau de commande d'accès du HCP associé à l'instrument chirurgical doit être ajusté sur la base de la proximité du premier HCP par rapport à la table d'opération ;
la réception d'une demande d'ajustement de niveau de commande d'accès de la part du second HCP en réponse au message de demande d'ajustement de niveau de commande d'accès ; et
sur la base de la demande d'ajustement de niveau de commande d'accès, l'ajustement du niveau de commande d'accès associé au premier HCP.

7. Procédé mis en œuvre par ordinateur selon la revendication 6, dans lequel le procédé comprend :
l'envoi d'une notification d'ajustement de niveau de commande d'accès au HCP, la notification d'ajustement de niveau de commande d'accès indiquant que le niveau de commande d'accès du HCP associé à l'instrument chirurgical a été ajusté par le second HCP.

8. Procédé mis en œuvre par ordinateur selon l'une quelconque des revendications 1 à 7, dans lequel le procédé comprend :
l'identification d'une étape chirurgicale en cours dans l'intervention chirurgicale ;
le fait de déterminer s'il convient d'ajuster le niveau de commande d'accès associé au HCP sur la base de l'étape chirurgicale en cours identifiée ; et
sur la base de la détermination d'ajuster le niveau de commande d'accès du HCP associé à l'instrument chirurgical, l'ajustement du niveau de commande d'accès du HCP associé à l'instrument chirurgical pour permettre au HCP de commander l'instrument chirurgical pendant l'étape chirurgicale en cours de l'intervention chirurgicale.

9. Procédé mis en œuvre par ordinateur selon l'une quelconque des revendications 1 à 8, dans lequel le procédé comprend :
l'indentification d'un état d'alimentation de l'instrument chirurgical ; et
le fait de déterminer s'il faut ajuster le niveau de commande d'accès du HCP associé à l'instrument chirurgical sur la base de l'état d'alimentation identifié de l'instrument chirurgical, dans lequel, sur la base de l'état d'alimentation identifié de l'instrument chirurgical étant à un état d'alimentation élevé, le procédé comprend l'ajustement du niveau de commande d'accès du HCP associé à l'instrument chirurgical pour bloquer l'entrée de commande par le HCP pour commander l'instrument chirurgical, et dans lequel, sur la base de l'état d'alimentation identifié de l'instrument chirurgical étant à un état d'alimentation faible, le procédé comprend l'ajustement du niveau de commande d'accès du HCP associé à l'instrument chirurgical pour effectuer l'entrée de commande par le HCP pour commander l'instrument chirurgical.

10. Procédé mis en œuvre par ordinateur selon l'une quelconque des revendications 1 à 9, dans lequel le procédé comprend :
la surveillance d'un biomarqueur associé au HCP, le biomarqueur peut comprendre au moins l'un parmi un niveau de fatigue ou un niveau de stress ; et
le fait de déterminer s'il faut ajuster le niveau de commande d'accès du HCP associé à l'instrument chirurgical sur la base du biomarqueur surveillé associé au HCP, dans lequel, sur la base du fait que le biomarqueur surveillé associé au HCP indique que le HCP a un niveau accru d'au moins l'un parmi le niveau de fatigue ou le niveau de stress, le procédé comprend l'ajustement du niveau de commande d'accès du HCP associé à l'instrument chirurgical pour bloquer l'entrée de commande par le HCP pour commander l'instrument chirurgical, et dans lequel, sur la base du fait que le biomarqueur surveillé associé au HCP indique que le HCP a un niveau acceptable d'au moins l'un parmi le niveau de fatigue ou le niveau de stress, le procédé comprend l'ajustement du niveau de commande d'accès du HCP associé à l'instrument chirurgical pour effectuer l'entrée de commande par le HCP pour commander l'instrument chirurgical.

11. Système informatique comprenant :
un processeur configuré pour effectuer le procédé selon l'une quelconque des revendications 1 à 10.

12. Dispositif de traitement de données comprenant des moyens pour réaliser le procédé selon l'une quelconque des revendications 1 à 10.

13. Système comprenant :
le système informatique selon la revendication 11 ; et
un instrument chirurgical couplé de manière communicative au système informatique.

14. Système comprenant :
le dispositif de traitement de données selon la revendication 12 ; et
un instrument chirurgical a couplé de manière communicative avec le dispositif de traitement de données.

15. Support lisible par ordinateur ayant des instructions qui, lorsqu'elles sont exécutées par un ordinateur, provoquent la mise en œuvre du procédé selon l'une quelconque des revendications 1 à 10.
